# EUROPEAN PATENT APPLICATION

(11) **EP 4 628 070 A1**
(43) Date of publication of application: **08.10.2025**
(21) Application number: 23898222.7
(22) Date of filing: 24.11.2023
(51) Int. Cl.: A61K 9/14, A61K 47/36, A61K 33/242, A61P 29/00, A61P 1/00, A61K 9/00

(54) **GOLD NANOZYME COMPRISING GLYCOL CHITOSAN AND GOLD PARTICLES, AND PHARMACEUTICAL COMPOSITION FOR PREVENTING OR TREATING INFLAMMATORY BOWEL DISEASE COMPRISING SAME**

(30) Priority: 30.11.2022 KR 20220164330; 27.01.2023 KR 20230011238
(71) Applicant: IUCF-HYU (Industry-University Cooperation Foundation Hanyang University), Seoul 04763 (KR)
(72) Inventor: LEE, Dong Yun, Seoul 04763 (KR); KIM, Hyung Shik, Seoul 04763 (KR); LEE, Si Eun, Seoul 04763 (KR)
(74) Representative: dompatent von Kreisler Selting Werner - Partnerschaft von Patent- und Rechtsanwälten mbB
(86) International application number: PCT/KR2023/019142
(87) International publication number: WO 2024/117691

(57) **Abstract**

One aspect according to the present invention relates to: a gold nanozyme including glycol chitosan and gold particles; and a pharmaceutical composition for preventing or treating inflammatory bowel disease, the composition comprising the gold nanozyme. The gold nanozyme and the pharmaceutical composition comprising same according to one aspect of the present invention was found to inhibit the expression of inflammatory factors, inhibit the generation of reactive oxygen species (ROS) and reactive nitrogen species (RNS) inside cells, and reduce the production of nitric oxide (NO) inside cells. In addition, the gold nanozyme was found to inhibit the secretion of Hight Mobility Group Box 1 (HMGB1) when further including glycyrrhizin. In addition, the gold nanozyme and the composition comprising same were found to enable the recovery of the length, weight, etc., of damaged intestines, and thus can be used in the inflammatory bowel disease prevention and/or treatment industry/market.

## Description

### [Technical Field]

The present invention relates to a gold nanozyme including glycol chitosan and a gold particle, and a pharmaceutical composition for preventing or treating inflammatory bowel disease, which includes the gold nanozyme.

### [Background Art]

Inflammatory bowel disease (IBD) is a chronic disease in which environmental factors and genetic factors interact to cause immune responses and inflammation in the intestines. IBD can be broadly divided into Crohn's disease, which affects the entire gastrointestinal tract (GI), and ulcerative colitis, which appears only in the large intestine, and the incidence of ulcerative colitis is higher. The exact cause of the disease has not yet been identified, but abnormalities in the regulation of immune responses are considered to be an important etiology.

The representative drugs currently used as therapeutic agents for inflammatory bowel disease include salicylic acid- and steroid-based drugs. Since the administration of steroid-based drugs and immunosuppressants is highly restricted due to their serious side effects, salicylic acid derivatives or biological agents (anti-TNF α antibodies) are mainly prescribed. Mesalazine, which is a type of synthetic drug, is mainly prescribed, and reduces the production of prostaglandin and leukotriene (substances involved in inflammatory responses and contained in leukocytes and immune cells) by inhibiting cyclooxygenase (COX) and lipoxygenase (LOX), and reduces the expression of genes related to intestinal inflammation through the inhibition of the PPAR-γ pathway. There are drugs in development, including JAK inhibitors, which inhibit the activity of JAK substances in cells, which have received clinical approval (e.g., tofacitinib) and those that are in clinical trials (e.g., TD-1473, upadacitinib, and baricitinib).

However, even with the prescription of mesalazine used as a synthetic drug, there are a significant number of patients who do not respond, as the disease gradually progresses, causing complications such as strictures and fistulas, and an objective response rate (ORR) to the drug is usually less than 60%. Biological agents have an initial non-response rate of approximately 10 to 40% and a high rate of secondary loss of response (LOR), in which symptoms relapse or worsen due to decreased efficacy during treatment, of 20 to 40% after one year of treatment, which is primarily due to anti-drug antibodies (ADAs). In addition, in the clinical trials for JAK inhibitors, the US FDA recently confirmed serious side effects, including heart disease, cancer, and thrombosis, and the trials are therefore at risk of being discontinued.

### [Disclosure]

### [Technical Problem]

The present invention is directed to providing a gold nanozyme including glycol chitosan and a gold particle.

The present invention is also directed to providing a pharmaceutical composition for preventing or treating inflammatory bowel disease, which includes the gold nanozyme.

The present invention is also directed to providing a pharmaceutical preparation for preventing or treating inflammatory bowel disease, which includes the gold nanozyme.

The present invention is also directed to providing a health functional food for preventing or improving inflammatory bowel disease, which includes the gold nanozyme.

The present invention is also directed to providing a method of preparing a gold nanozyme, which includes:
mixing a glycol chitosan solution and a gold particle solution;
oxidizing/reducing glycol chitosan and gold particles in the mixed solution; and
forming a nanozyme with the oxidized/reduced glycol chitosan and the gold particles.

The present invention is also directed to providing a method of preventing or treating inflammatory bowel disease, which includes administering the gold nanozyme to a subject in need thereof.

The present invention is also directed to providing a use of the gold nanozyme for preparing a therapeutic agent for preventing or treating inflammatory bowel disease.

### [Technical Solution]

One aspect of the present invention provides a gold nanozyme including glycol chitosan and a gold particle.

The term "glycol chitosan" refers to a water-soluble derivative of chitosan produced by conjugation of chitosan and ethylene glycol. The glycol chitosan may have non-cytotoxicity and biocompatibility, and may increase intestinal mucosal adsorption or drug persistence.

**In** one embodiment, the gold particle may be a gold nanoparticle (AuNP).

The term "gold nanoparticle (AuNP)" refers to an ultrafine particle made of gold, having a size of 1 to 2,500 nm, and a particle exhibiting unusual and diverse properties due to its small size.

In one embodiment, the glycol chitosan may be embedded in the gold particle.

In one embodiment, the gold nanozyme may serve as one or more enzymes selected from the group consisting of peroxidase, superoxide dismutase (SOD), and catalase.

The term "nanozyme" is a compound word of nano and enzyme, and refers to a nanomaterial serving as an enzyme in the body.

The term "gold nanozyme" refers to a nanomaterial produced using gold particles in the nanozyme.

The term "peroxidase" refers to an enzyme that serves to reduce harmful hydrogen peroxide produced during cellular metabolism.

The term "superoxide dismutase (SOD)" refers to an enzyme that catalyzes the reaction in which a superoxide ion (O₂-) is converted into hydrogen peroxide and an oxygen molecule (dismutation).

The term "catalase" refers to an enzyme that catalyzes the reaction in which hydrogen peroxide decomposes to produce water and oxygen.

In one embodiment, the gold nanozyme may remove a hydroxyl radical.

The term "hydroxyl radical" refers to active oxygen generated in the body.

In one embodiment, the gold nanozyme may further include glycyrrhizin.

The term "glycyrrhizin" is a component extracted from licorice and is known as a factor that acts on the 11β-hydroxysteroid dehydrogenase type 1 (11beta-HSD1) hormone to regulate glucocorticoids.

In one embodiment, the glycyrrhizin may suppress the secretion of high mobility group box 1 (HMGB 1).

The term "high mobility group box 1 (HMGB1)" refers to a protein encoded by the HMGB1 gene in humans.

In one embodiment, the gold nanozyme may be stably maintained at pH 0 to 7.

In one embodiment, the gold nanozyme may be stably maintained at pH 0 to 7, pH 0 to 6.7, pH 0 to 6.3, pH 1 to 7, pH 1 to 6.7, pH 1 to 6.3, pH 1.5 to 7, pH 1.5 to 6.7, or pH 1.5 to 6.3.

In one embodiment, it was confirmed whether Citrate@AuNP, Alginate@AuNP, and Glycol chitosan@AuNP are stably maintained in a biomimetic environment such as a GI tract biomimicking environment. As a result, it was confirmed that the properties of Glycol chitosan@AuNP are maintained at pH 2, pH 5, and pH 6, which are biomimetic environments that mimic the GI tract biomimicking environment (see Example 4).

In one embodiment, it was confirmed whether Citrate@AuNP, Alginate@AuNP, Glycol chitosan@AuNP, and Glycol chitosan-GL@AuNP maintain their charges as is and are stably maintained in a biomimetic environment such as the GI tract biomimicking environment. As a result, the properties of Chitosan@AuNP and Glycol chitosan-GL@AuNP are stably maintained at pH 2 and pH 6, which are biomimetic environments that mimic the GI tract biomimicking environment (see Example 23).

The term "Glycol chitosan@AuNP" refers to a gold nanozyme including glycol chitosan and AuNP, and the term "Glycol chitosan-GL@AuNP" refers to a gold nanozyme including glycol chitosan, glycyrrhizin, and AuNP.

Another aspect of the present invention provides a pharmaceutical composition for preventing or treating inflammatory bowel disease, which includes the gold nanozyme.

The "gold nanozyme" may be within the above-described scope.

The term "prevention" may refer to all actions of inhibiting inflammatory bowel disease in a subject or delaying the onset of inflammatory bowel disease by administration of the pharmaceutical composition according to one aspect.

The term "treatment" may refer to all actions involved in improving or beneficially changing the symptoms of inflammatory bowel disease in a subject by administration of the pharmaceutical composition according to one aspect.

The term "administration" refers to introduction of a certain material to a subject by an appropriate method, and "subject" refers to all organisms, including humans, rats, mice, and livestock, which may have inflammatory bowel disease. Specific examples of the organisms may be mammals, including humans.

In one embodiment, the gold nanozyme may be contained at 100 µg/mL to 150 µg/mL.

In one embodiment, the gold nanozyme may be included at 100 µg/mL to 150 µg/mL, 100 µg/mL to 140 µg/mL, 100 µg/mL to 130 µg/mL, 110 µg/mL to 150 µg/mL, 110 µg/mL to 140 µg/mL, 110 µg/mL to 130 µg/mL, 120 µg/mL to 150 µg/mL, 120 µg/mL to 140 µg/mL, or 120 µg/mL to 130 µg/mL.

In one embodiment, Raw 264.7 cells and Caco-2 cells were co-cultured, the Caco-2 cells were stimulated with hydrogen peroxide, and it was confirmed how HMGB1 secreted thereby affected the Raw 264.7 cells when co-treated with either Glycol chitosan@AuNP (125 µg/mL) or Glycol chitosan-GL@AuNP(125 µg/mL), and hydrogen peroxide (H₂O₂ (100 µM)). As a result, it was confirmed that the group treated with Glycol chitosan@AuNP or Glycol chitosan-GL@AuNP showed almost no activated form of Raw 264.7 cells. In addition, it was confirmed that, when treated with chitosan@AuNP or Glycol chitosan-GL@AuNP, a monolayer was maintained as is (see Example 24).

In one embodiment, the pharmaceutical composition may suppress the expression of one or more selected from tumor necrosis factor-α (TNF-α), interleukin 6 (IL-6), interleukin-1 β (IL-1β), inducible nitric oxide synthase (iNOS), monocyte chemoattractant protein-1 (MCP-1), C-reactive protein (CRP), and calprotectin.

In one embodiment, it was confirmed whether Glycol chitosan-GL@AuNP has an anti-inflammatory effect in a DSS-induced mouse model. As a result, it was confirmed that, when Glycol chitosan-GL@AuNP was treated, the expression of TNF-α, IL-6, IL-1β iNOS, and MCP-1 was reduced (see Examples 29 and 30).

In another embodiment, the level of C-reactive protein (CRP), which is a blood inflammatory factor, was confirmed. As a result, it was confirmed that diseased mice induced with DSS showed increased levels of CRP, which is a blood inflammatory factor, but Glycol chitosan-GL@AuNP restored these levels to normal levels (see Example 34).

In still another embodiment, the level of calprotectin, which is an inflammatory factor in feces, was confirmed. As a result, it was confirmed that Glycol chitosan-GL@AuNP treatment reduces the calprotectin level (see Example 35).

In one embodiment, the pharmaceutical composition may inhibit the production of one or more selected from the group consisting of reactive oxygen species (ROS), reactive nitrogen species (RNS), and nitric oxide (NO) in cells.

In one embodiment, the ROS scavenging capability of Citrate@AuNP, Alginate@AuNP and Glycol chitosan@AuNP in intestinal epithelial cells was confirmed by co-treating the cells with H₂O₂ as well as either Citrate@AuNP, Alginate@AuNP or Glycol chitosan@AuNP, and staining the cells. As a result, the production of ROS was inhibited by Citrate@AuNP, Alginate@AuNP, and Glycol chitosan@AuNP by confirming the disappearance of almost all fluorescence in the Citrate@AuNP-, Alginate@AuNP-, and Glycol chitosan@AuNP- treated groups (see Example 12).

In another embodiment, the concentration of secreted nitric oxide (NO) was determined when Raw 264.7 cells that had been stimulated with lipopolysaccharide (LPS) were co-treated with LPS, and either Citrate@AuNP, Alginate@AuNP or Glycol chitosan@AuNP. As a result, when Citrate@AuNP, Alginate@AuNP, or Glycol chitosan@AuNP was treated at a low concentration of 31.25 µg/mL, it was confirmed that the high concentration, 125 µg/mL of NO₂ was removed by approximately 50%, which confirms that AuNP treatment reduces NO production, making it possible to scavenge RNS (see Example 13).

In one embodiment, the pharmaceutical composition may inhibit cell polarization or spleen hypertrophy.

The term "hypertrophy" refers to an abnormal increase in the volume of an organ or tissue due to enlargement of its constituent cells.

In one embodiment, to determine the ratio of M1 and M2 polarization when Raw 264.7 cells were treated with either Glycol chitosan@AuNP or Glycol chitosan-GL@AuNP, flow cytometry was performed. As a result, both Glycol chitosan@AuNP and Glycol chitosan-GL@AuNP were confirmed to reduce the M1 polarization pattern by approximately 32.93% to 0% even after H₂O₂ damage. In addition, Glycol chitosan@AuNP showed a higher M2 polarization ratio, but Glycol chitosan-GL@AuNP showed little morphological change to M1 and M2, confirming that it maintained monocytic morphology (see Example 26).

In another embodiment, mouse spleen hypertrophy was confirmed. As a result, in a group in which 2.5% DSS was treated with PBS, the length of the enlarged spleen was approximately 1.5 cm, but in the groups treated with Glycol chitosan@AuNP and Glycol chitosan-GL@AuNP, its length was shortened similar to the normal state (see Example 27).

In one embodiment, the pharmaceutical composition may repair a damaged colon or restore the tight junction between cells.

In one embodiment, the damaged colon may be a negative change from the normal colon.

In one embodiment, the pharmaceutical composition may restore the weight or length of the damaged colon.

In one embodiment, the mouse colon weight, length, etc. of the positive control and experimental groups (Glycol chitosan@AuNP and Glycol chitosan-GL@AuNP) were confirmed. As a result, the groups treated with Glycol chitosan@AuNP and Glycol chitosan-GL@AuNP showed that the degree of body weight recovery is excellent, and the length of the colon is also recovered to a similar level as the control (see Example 27).

The term "tight junction" refers to the blocking of the movement of cells or materials between two linked cells.

In one embodiment, when Glycol chitosan@AuNP and Glycol chitosan-GL@AuNP were treated, the degree of tight junction (ZO-1 and occludin) recovery was confirmed. As a result, in mice fed only PBS after induction by DSS, a tight junction protein such as occludin or Zo-1 was destroyed, confirming that the monolayer of intestinal epithelial cells was destroyed, and the recovery of the tight junction was confirmed by yellow fluorescence generated in the co-treatment with AuNP (see Example 28).

In one embodiment, the pharmaceutical composition may be for preventing damage to the mucosa or mucus layer or regenerating the damaged mucosa or mucus layer.

In one embodiment, the degree of mucus layer recovery was confirmed in the treatment with Glycol chitosan@AuNP and Glycol chitosan-GL@AuNP. As a result, it was confirmed that, in the subepithelial goblet cell part, which has a role in mucous secretion, the mucus was stained, and Glycol chitosan@AuNP and Glycol chitosan-GL@AuNP prevented DSS-induced mucosal damage (see Example 36).

In one embodiment, the pharmaceutical composition may increase the microbiome associated with the barrier protective function.

The term "microbiome" refers to microorganisms inhabiting the human body, and particularly, refers to intestinal microorganisms.

In one embodiment, the relative diversity of the intestinal microbiome was confirmed. As a result, it was confirmed that, in the Glycol chitosan-GL@AuNP-treated groups of both healthy mice and the DSS group, Glycol chitosan-GL@AuNP can restore intestinal immune homeostasis without disturbing intestinal microflora. In addition, it was confirmed that Glycol chitosan-GL@AuNP treatment significantly increased *Akkermansia muciniphila,* which is a member of the microbiota associated with the protective intestinal barrier function, and further analysis at the family level revealed that Glycol chitosan-GL@AuNP treatment significantly increased *Lactobacillus intestinalis,* which has a protective intestinal barrier function and a beneficial role in IBD, and *Muribaculum intestinale,* which is a member of the microbiota associated with essential anaerobes that can alleviate DSS-induced IBD (see Example 39).

In one embodiment, the inflammatory bowel disease may be one or more selected from the group consisting of Crohn's disease, ulcerative colitis, intestinal Bechet's disease, and enteritis.

In addition, the pharmaceutical composition may include an active ingredient alone, or one or more pharmaceutically acceptable carriers, excipients, and diluents.

Specifically, the carrier may be, for example, a colloidal suspension, a powder, a saline solution, a lipid, a liposome, a microsphere, or a nanosphere. These carriers may form a complex or may be associated with a delivery means, and may be delivered *in vivo* using a delivery system known in the art, such as a lipid, liposome, a microparticle, a gold nanoparticle, a polymer, a condensation agent, a polysaccharide, a polyamino acid, a dendrimer, a saponin, an adsorption-enhancing material, or a fatty acid.

The pharmaceutical composition may be formulated with conventionally used diluents or excipients, such as a lubricant, a sweetener, a flavoring agent, an emulsifier, a suspending agent, a preservative, a filler, a bulking agent, a binder, a wetting agent, a disintegrant, and a surfactant. Solid preparations for oral administration may include pills, powders, granules, and capsules, and such solid preparations are prepared by mixing at least one excipient, such as starch, calcium carbonate, sucrose, lactose, or gelatin with the composition. Aside from simple excipients, lubricants such as magnesium stearate, talc, etc. are further used. Liquid preparations for oral administration include suspensions, liquids for internal use, emulsions, and syrups, and may further include various types of excipients, for example, a wetting agent, a sweetener, a fragrance and a preservative, in addition to a commonly-used simple diluent such as water, or liquid paraffin. Preparations for parenteral administration include sterilized aqueous solutions, non-aqueous solvents, suspensions, emulsions, lyophilized formulations, and suppositories. As a non-aqueous solvent or suspension, propylene glycol, polyethylene glycol, a vegetable oil such as olive oil, or an injectable ester such as ethyl oleate may be used. As a suppository base, Witepsol, Macrogol, Tween 61, cacao butter, laurin butter, or glycerogelatin may be used. When the pharmaceutical composition is prepared in the form of eye drops, a known diluent or excipient may be used.

The pharmaceutical composition may be provided by being mixed with another pharmaceutical composition for preventing or treating inflammatory bowel disease, and the other pharmaceutical composition for preventing or treating inflammatory bowel disease may be a conventionally-known pharmaceutical composition for preventing or treating inflammatory bowel disease or a newly developed pharmaceutical composition for preventing or treating inflammatory bowel disease.

When the pharmaceutical composition further includes another pharmaceutical composition for preventing or treating inflammatory bowel disease or is provided by being mixed with another pharmaceutical composition for preventing or treating inflammatory bowel disease, it is important to mix the amount that can achieve the maximum effect with the minimum amount without side effects, which may be easily determined by one of ordinary skill in the art.

The pharmaceutical composition may be administered in combination with another pharmaceutical composition for preventing or treating inflammatory bowel disease without being mixed therewith, and administered simultaneously, separately, or sequentially, or administered in a single dose or multiple doses. **In** consideration of all of the above-mentioned parameters, it is important to achieve the maximum effect with the minimum dose without a side effect, and such a dose may be easily determined by one of ordinary skill in the art.

The pharmaceutical composition may be administered orally or parenterally, and for parenteral administration, topical application, intraperitoneal injection, intrarectal injection, subcutaneous injection, intravenous injection, intramuscular injection, intra-arterial injection, intramedullary injection n, intracardiac injection, intrathecal injection, percutaneous injection, intranasal injection, injection into the intestinal tract, local injection, sublingual injection, or intracardiac injection may be selected.

The pharmaceutical composition is administered at a pharmaceutically effective amount. The term "pharmaceutically effective amount" refers to an amount sufficient for treating a disease at a reasonable benefit/risk ratio applicable for medical treatment, and an effective dosage may be determined by parameters including a type of a patient's disease, severity, drug activity, sensitivity to a drug, administration time, an administration route and an excretion rate, the duration of treatment and drugs simultaneously used, and other parameters well known in the medical field.

In one aspect, the pharmaceutical composition may be administered once a day, or administered in multiple doses. For example, the pharmaceutical composition may be administered every other day, or once a week. Specifically, the pharmaceutical composition may be administered at 0.001 to 1000 mg/kg/day, and more specifically, at 0.1 to 100 mg/kg/day.

Still another aspect of the present invention provides a pharmaceutical preparation for preventing or treating inflammatory bowel disease, which includes the gold nanozyme.

The "gold nanozyme," "inflammatory bowel disease," "prevention," and "treatment" may be within the above-described scope.

In one embodiment, the preparation may be in the form of an injection, an infusion, a spray, or a liquid formulation.

The pharmaceutical preparation may further include a pharmaceutically acceptable carrier that can be usually added thereto. The pharmaceutically acceptable carrier may further include additives conventionally used in the pharmaceutical field, such as an excipient, a disintegrant, a binder, a lubricant, an emulsifier, a suspending agent, a stabilizer, and a pH adjuster, and if needed, a sweetener, a flavoring, and/or a coloring agent. The excipient includes microcrystalline cellulose, starch, silicon dioxide (SiO₂), sugar ester, Ludipress, sucrose, maltose, fructose, and sorbitol. Preferably, as the excipient, a mixture of lactose and silicon dioxide may be used. The amount of excipient may be approximately 90 wt% or less based on the total weight of the pharmaceutical preparation, but the present invention is not limited thereto. The disintegrant includes carboxymethylcellulose calcium (CMC-Ca), carboxymethylcellulose sodium (CMC-Na), crospovidone, and alginic acid. The amount of disintegrant used may range from 3 to 16 wt% based on the total weight of the pharmaceutical preparation, but the present invention is not limited thereto. The lubricant may include stearic acid, magnesium stearate, zinc stearate, glyceryl behenate, glyceryl palmitostearate, or talc, and may be used at approximately 3 wt% or less with respect to the total weight of the pharmaceutical preparation, but the present invention is not limited thereto.

In addition, the pharmaceutical preparation may be prepared for oral administration, and may be prepared in various forms, for example, a tablet, a film a suspension, a granule, a gel, a pill, a tincture, a decoction, an infusion, a spirit, a fluid extract, an elixir, an extract, a syrup, a powder, an aromatic water, and a lemonade. The tablet may be prepared in various forms, for example, an orally disintegrating tablet, a mucoadhesive tablet, a dispersible tablet, a sublingual tablet, a buccal tablet, a chewable tablet, a dispensing tablet, a multi-layered tablet, a press-coated tablet, an effervescent tablet, and a solution tablet. **In** addition, the various tablets may be modified by one of ordinary skill in the art into various forms if needed. More preferably, the pharmaceutical preparation is formed in a dosage form that disintegrates (dissolves) in the oral cavity (i.e., oral disintegration and dissolution), for example, a dispersible (soluble) form in the oral cavity, such as an oral dissolving film, an orally disintegrating tablet, a suspension, a suspension tablet, a rapid disintegrating tablet, an orally disintegrating granule, an orally disintegrating troche, a sublingual tablet, a powder, and/or a chewable tablet.

**In** one embodiment, the pharmaceutical preparation may be administered daily at approximately 0.001 mg/kg to approximately 10 g/kg, approximately 0.01 mg/kg to approximately 1 g/kg. However, the dosage may vary according to patient's conditions (age, sex, weight, etc.), the severity of the condition being treated, etc. The total daily dose may be divided and administered several times throughout the day for convenience, as needed.

Yet another aspect of the present invention provides a health functional food for preventing or improving inflammatory bowel disease, which includes the gold nanozyme.

The "gold nanozyme," "inflammatory bowel disease," and "prevention" may be within the above-described scope.

The term "improvement" may refer to all actions that reduce at least parameters associated with the condition being treated, for example, the degree of symptoms. Here, the health functional food may be used simultaneously with or separately from a drug for treatment before or after the onset of the disease to prevent or improve inflammatory bowel disease.

**In** the health functional food, the active ingredient may be directly added to food or used together with other food or food ingredients, and may be suitably used according to a conventional method. The mixing amount of the active ingredient may be suitably determined according to the purpose (for prevention or improvement) of use thereof. Generally, in the production of food or beverages, the composition of the present invention is added at 15 wt% or less, and preferably 10 wt% or less with respect to the raw materials. However, in long-term consumption for health and hygiene or health control, the amount of the composition may be below the above-mentioned range.

The health functional food may further include any one or more of a carrier, a diluent, an excipient, and an additive, and may be formulated in one selected from the group consisting of a tablet, a pill, a powder, a granule, a capsule, and a liquid. Foods that can contain a compound according to one aspect include various foods, powders, granules, tablets, capsules, syrups, beverages, gum, tea, vitamin complexes, and health functional foods.

Specific examples of the carrier, excipient, diluent, and additive may be at least one selected from the group consisting of lactose, dextrose, sucrose, sorbitol, mannitol, erythritol, starch, acacia gum, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methylcellulose, methyl hydroxy benzoate, propyl hydroxy benzoate, talc, magnesium stearate, and mineral oil.

The health functional food may contain other components as essential ingredients without any specific limitation, other than containing the active ingredient. For example, like regular beverages, it may contain additional components such as various flavorings or natural carbohydrates. The above-mentioned natural carbohydrates are monosaccharides such as glucose and fructose, disaccharides such as maltose and sucrose, polysaccharides such as common sugars including dextrin and cyclodextrin, and sugar alcohols such as xylitol, sorbitol, and erythritol. As flavorings other than the above examples, natural flavorings (thaumatin and stevia extract (e.g., rebaudioside A and glycyrrhizin)) and synthetic flavorings (e.g., saccharin and aspartame) may be favorably used. The proportion of the natural carbohydrate may be suitably determined by selection by one of ordinary skill in the art.

The health functional food composition according to one aspect may contain various nutrients, vitamins, minerals (electrolytes), flavoring agents including synthetic and natural flavoring agents, coloring agents, fillers (cheese, chocolate, etc.), pectic acid and a salt thereof, alginic acid and a salt thereof, organic acids, protective colloidal thickening agents, pH adjusters, stabilizers, preservatives, glycerin, alcohols, or carbonizing agents used in carbonated beverages, and such components may be used independently or in combination. A proportion of such an additive may also be suitably selected by those of ordinary skill in the art.

The health functional food may be provided by being mixed with a conventionally known health functional food for preventing or improving inflammatory bowel disease, or a newly developed health functional food for preventing or improving inflammatory bowel disease.

When the health functional food further includes a health functional food for preventing or improving inflammatory bowel disease, it is important to mix the amount that can achieve the maximum effect with the minimum amount without side effects, and may be easily determined by one of ordinary skill in the art.

Yet another aspect of the present invention provides a method of preparing a gold nanozyme, which includes: mixing a glycol chitosan solution and a gold particle solution;
oxidizing/reducing glycol chitosan and gold particles in the mixed solution; and
forming a nanozyme with the oxidized/reduced glycol chitosan and the gold particles.

The "glycol chitosan," "gold particle," "nanozyme," and "gold nanozyme" may be within the above-described scope.

**In** one embodiment, in the mixing step, the glycol chitosan solution may further include glycyrrhizin.

**In** one embodiment, in the mixing step, the ratio of the glycol chitosan solution and the gold particle solution may be 1:2 to 1:10.

**In** one embodiment, in the nanozyme forming step, the oxidized/reduced glycol chitosan may be embedded in the gold particles to form a nanozyme.

Yet another aspect of the present invention provides a method of preventing or treating inflammatory bowel disease, which includes administering the gold nanozyme to a subject in need thereof.

The "gold nanozyme," "subject," "administration," "inflammatory bowel disease," "prevention," and "treatment" may be within the above-described scope.

Yet another aspect of the present invention provides a use of the gold nanozyme for preparing a therapeutic agent for preventing or treating inflammatory bowel disease.

The "inflammatory bowel disease," "prevention," "treatment," and "gold nanozyme" are may be within the above-described scope.

### [Advantageous Effects]

It was confirmed that a gold nanozyme according to one aspect and a composition including the same can suppress the expression of inflammatory factors, inhibit the production of reactive oxygen species (ROS) and reactive nitrogen species (RNS) in cells, and reduce the production of nitric oxide (NO) in cells. In addition, when the gold nanozyme further includes glycyrrhizin, it was confirmed that the secretion of high mobility group box 1 (HMGB1) is inhibited. Further, as it was confirmed that the gold nanozyme and the composition including the same repair a damaged colon, they can be utilized in the industry/market for prevention and/or treatment of inflammatory bowel disease.

### [Description of Drawings]

FIG. 1 shows the results of measuring the light absorbance of a gold nanoparticle (AuNP) using an UV-visible spectrophotometer.
FIG. 2 shows the results of analyzing X-ray diffractometer (XRD) patterns in AuNP (Citrate@AuNP (1:10), Alginate@AuNP (0.2%), Glycol chitosan@AuNP (0.32%), and Glycol chitosan-GL@AuNP.
FIG. 3 shows the transmission electron microscopy (TEM) images and TEM lattice images of AuNP(Citrate@AuNP (1:10), Alginate@AuNP (0.2%), and Glycol chitosan@AuNP (0.32%).
FIG. 4 shows the results of measuring the zeta potential (ZP) and dynamic light scattering (DLS) of AuNPs (Data is expressed as mean ± SEM (n=3)).
FIG. 5 shows the results of confirming the colloidal stability of AuNPs (Citrate@AuNP (1:10) Alginate@AuNP 0.2% and Glycol chitosan@AuNP 0.32%) in the GI tract biomimicking environment (pH 2).
FIG. 6 shows the colloidal stability of AuNPs (Citrate@AuNP (1:10) Alginate@AuNP 0.2%, and Glycol chitosan@AuNP 0.32%) in the GI tract biomimicking environment (pH 5).
FIG. 7 shows the results of measuring the PDI values of AuNPs.
FIG. 8 is a schematic diagram of the ROS scavenging of Glycol chitosan@AuNP.
FIG. 9 shows the results of confirming the peroxidase-like activity of AuNPs (Data is expressed as mean ± SEM (n=3)).
FIG. 10 shows the mechanism of color change when a TMB solution was added after the HRP reaction with H₂O₂.
FIG. 11 shows the results of confirming the catalase-like activity of AuNPs (Data is expressed as mean ± SEM (n=3)).
FIG. 12 shows the hydrogen peroxide scavenging mechanism of Glycol chitosan@AuNP.
FIG. 13 shows the results of observing oxygen bubbles through the reaction with AuNPs.
FIG. 14 shows the results of observing oxygen bubbles through the hydroxyl radical reaction with an AuNP-coated material.
FIG. 15 shows the results of TMB assay for chitosan oligosaccharide (COS) and glycol chitosan (Data is expressed as mean ± SEM (n=3)).
FIG. 16 shows the reaction scheme between H₂O₂ and catalase.
FIG. 17 shows the peak of a product generated by oxidation of the amine groups of glycol chitosan and chitosan oligosaccharide (COS).
FIG. 18 shows the results of evaluating AuNP toxicity to Caco-2 cells through an LDH assay (Data is expressed as mean ± SEM (n=4)).
FIG. 19 shows the results of morphologically evaluating AuNP toxicity to Caco-2 cells.
FIG. 20 shows the results of evaluating AuNP toxicity to Raw 264.7 cells through an LDH assay (Data is expressed as mean ± SEM (n=4)).
FIG. 21 shows the results of morphologically evaluating AuNP toxicity to Raw 264.7 cells.
FIG. 22 shows the results of confirming total ROS scavenging efficiency and antioxidation properties through 2,2'-azino-bis(3-ethylbenzothiazoline 6-sulfonate) (ABTS) analysis.
FIG. 23 shows the results of measuring electron spin resonance to confirm the superoxide dismutase (SOD)-like activity of AuNPs.
FIG. 24 shows the results of confirming intracellular ROS scavenging activity using dichlorofluorescein-diacetate (DCF-DA).
FIG. 25 shows the images of the DCF-DA results of FIG. 24.
FIG. 26 shows the results of confirming the effect of AuNPs on the production of NO, which is a type of RNS (Data is expressed as mean ± SEM (n=4). **P < 0.01, and ***P < 0.001).
FIG. 27 shows the results of confirming tight junctions through immunocytochemistry (ICC).
FIG. 28 shows the morphology of Caco-2 cells to confirm whether tight junctions are restored after H₂O₂ stimulation (scale bar: 10 µm).
FIG. 29A to 29B shows the results of confirming M1 and M2 polarization ratios of RAW 264.7 cells by FACS (filled histograms represent iso-type-matched control antibody staining). Specifically, FIG. 29A shows the M1 and M2 polarization ratios of RAW 264.7 cells when M1 cell surface markers include CD80 and M2 cell surface markers include CD206. FIG. 29B shows the results of confirming the M1 and M2 polarization ratios in RAW 264.7 cells when M1 cell surface markers include iNOS and M2 cell surface markers include CD163.
FIG. 30A to 30B shows the results of confirming the population of M1 and M2 polarization in RAW 264.7 cells by FACS (Data is expressed as mean ± SEM (n=3)). Specifically, FIG. 30A shows the results of confirming M1 and M2 population in RAW 264.7 cells by FACS when M1 cell surface markers include CD80 and M2 cell surface markers include CD206. FIG. 30B shows the results of confirming M1 and M2 populations in RAW 264.7 cells by FACS when M1 cell surface markers include iNOS and M2 cell surface markers include CD163.
FIG. 31 shows the monocytic morphology in RAW 264.7 cells co-treated with LPS and AuNPs.
FIG. 32 shows the RT-PCR results for pro-inflammatory cytokines, such as TNF-α, IL-6, IL-1α, iNOS, and MCP-1 (Data is expressed as mean ± SEM (n=3)).
FIG. 33 shows the ELISA results for pro-inflammatory cytokines, such as TNF-α, IL-6, IL-1α, iNOS, and MCP-1 (Data is expressed as mean ± SEM (n=3)).
FIG. 34 shows the mechanism when mice with colitis induced by DSS were orally administered a drug.
FIG. 35 shows the results of confirming the residual level over time using inductively coupled plasma-mass spectrometry (ICP-MS) when AuNPs were administered to colon tissue.
FIG. 36 shows the results of confirming the residual level over time using bio-transmission electron microscopy (BIO_TEM) when AuNPs were administered to colon tissue.
FIG. 37 shows the scheme for conjugating glycyrrhizin and glycol chitosan.
FIG. 38 shows the results of H-NMR of glycol chitosan-glycyrrhizin.
FIG. 39 shows the results of FT-IR of glycol chitosan-glycyrrhizin.
FIG. 40 shows the scheme for synthesizing glycol chitosan and glycyrrhizin and the scheme for coating AuNO with the synthesized products.
FIG. 41 shows the results of confirming the DLS values of AuNPs (Citrate@AuNP (1:10), Alginate@AuNP 0.2%, and Glycol chitosan@AuNP 0.32%) in the GI tract biomimicking environments (pH 2 and pH 6) (Data is expressed as mean ± SEM (n=3)).
FIG. 42 shows the results of confirming the ZP (mV) values of AuNPs (Citrate@AuNP (1:10), Alginate@AuNP 0.2%, and Glycol chitosan@AuNP 0.32%) in the GI tract biomimicking environments (pH 2 and pH 6) (Data is expressed as mean ± SEM (n=3)).
FIG. 43 shows a co-culture system of Raw 264.7 cells and Caco-2 cells.
FIG. 44 shows the results of confirming the morphology of Raw 264.7 cells in a co-culture system of Raw 264.7 cells and Caco-2 cells.
FIG. 45 shows the results of confirming the morphology of Caco-2 cells in a co-culture system of Raw 264.7 cells and Caco-2 cells.
FIG. 46 shows the results of ELISA verification of the amount of HMGB1 secretion in a co-culture system of Raw 264.7 cells and Caco-2 cells (Data is expressed as mean ± SEM (n=4)).
FIG. 47 shows the results of ELISA verification of inflammatory factors secreted from Raw 264.7 cells in a co-culture system of Raw 264.7 cells and Caco-2 cells (Data is expressed as mean ± SEM (n=4)).
FIG. 48 shows the results of FACS verification of M1 and M2 polarization of Raw 264.7 cells in a co-culture system of Raw 264.7 cells and Caco-2 cells.
FIG. 49 shows the results of confirming the population for M1 and M2 polarization of Raw 264.7 cells in a co-culture system of Raw 264.7 cells and Caco-2 cells (Data is expressed as mean ± SEM (n=3)).
FIG. 50 shows the drug treatment scheme in a mouse experiment (N=5).
FIG. 51 shows the results of measuring a mouse survival rate.
FIG. 52 shows the results of measuring the body weights of mice.
FIG. 53 shows the results of measuring the disease activity index (DAI) values of mice.
FIG. 54 shows the results of measuring mouse colon lengths.
FIG. 55 shows the results of confirm damage to mouse colons.
FIG. 56 shows the results of measuring the weights and lengths of mouse colons (Data is expressed as mean ± SEM (n=5)).
FIG. 57 shows images of the mouse anus.
FIG. 58 shows the results of confirming the hypertrophy of the mouse spleen.
FIG. 59 is a diagram showing the results of confirming tight junction recovery through immunohistochemistry in mouse colon tissue.
FIG. 60 shows the results of ELISA verification of inflammatory factors in mouse serum (Data is expressed as mean ± SEM (n=4)).
FIG. 61 shows the results of ELISA verification of inflammatory factors in the mouse colon (Data is expressed as mean ± SEM (n=4)).
FIG. 62 shows the results of performing qRT-PCR on inflammatory factors in the mouse colon (Data is expressed as mean ± SEM (n=4)).
FIG. 63 shows the results of confirming the number of M1 cells in the mouse colon (Data is expressed as mean ± SEM (n=3)).
FIG. 64 shows the results of flow cytometry to confirm the number of M1 and M2 cells in the mouse colon.
FIG. 65 shows the results of confirming toxicity in major organs, such as the heart, liver, lung, kidney, and spleen.
FIG. 66 shows the results of ELISA verification of C-reactive protein (CRP), which is an inflammatory factor in mouse serum (Data is expressed as mean ± SEM (n=4)).
FIG. 67 shows the results of ELISA verification of calprotectin, which is an inflammatory factor in mouse feces (Data is expressed as mean ± SEM (n=4)).
FIG. 68 shows the results of confirming the degree of recovery of a mucus layer when Glycol chitosan@AuNP and Glycol chitosan-GL@AuNP were treated by immunohistochemistry (IHC).
FIG. 69 shows the results of confirming the amount of blood influx during 24 hours after oral administration of Glycol chitosan-GL@AuNP (Data is expressed as mean ± SEM (n=3)).
FIGS. 70A to 70C show the results of confirming the major organ functions and blood toxicity in the oral administration of Glycol chitosan@AuNP and Glycol chitosan-GL@AuNP (Data is expressed as mean ± SEM (n=4)). Specifically, FIG. 70A shows the results of analyzing total protein, albumin, globulin, AST, ALT, BUN, total-bilirubin, and creatinine. FIG. 70B shows the results of analyzing RBC, HGB, HCT, RBS indices (MCV, MCH and MCHC), RDW, MPV, and PLT. FIG. 70C shows the results of WBC and WBC differential counting (NEU, LYM, MONO, EOS, and BASO).
FIG. 71 shows the results of measuring operational taxonomic unit (OTU) richness (Data is expressed as mean ± SEM (n=3)).
FIG. 72 shows the results of measuring Shannon's diversity (Data is expressed as mean ± SEM (n=3)).
FIG. 73 shows the results of measuring the Simpson indices for the alpha diversity of the gut microbiota (Data is expressed as mean ± SEM (n=3)).
FIG. 74 shows the results of analyzing the beta diversity of the gut microbiota (Data is expressed as mean ± SEM (n=3)).
FIG. 75 shows the results of analyzing the heatmap of the taxonomy composition of microorganisms at the family level.
FIG. 76 shows the results of confirming the relative diversity of the intestinal microbiome.
FIG. 77 shows the results of analyzing the heatmap of the taxonomy composition of microorganisms at the phylum-species levels.
FIG. 78 shows the results of confirming the relative richness of *Akkermansia muciniphila* in the microbiota (Data is expressed as mean ± SEM (n=3)).
FIG. 79 shows the results of confirming the relative richness of *Lactobacillus intestinalis* in the microbiota (Data is expressed as mean ± SEM (n=3)).
FIG. 80 shows the results of confirming the relative richness of *Muribaculum intestinale* in the microbiota (* P < 0.05, ** P < 0.01 (PBS-treated group in 2.5 % DSS group), and Data is expressed as mean ± SEM (n=3)).

### [Modes of the Invention]

Hereinafter, the present invention will be described in further detail with respect to examples. However, these examples are intended to illustrate the present invention and the scope of the present invention is not limited to these examples.

### Examples

### 1. Method of synthesizing gold nanoparticle (AuNP)

### (1) Use of citrate

An attempt was made to synthesize a gold nanoparticle (AuNP) using citrate. A synthetic alloy solution (1.13 mM) and each citrate solution (5.69 mM, 7.98 mM, and 11.3 mM) were dissolved in distilled water to have a molar ratio of gold and citrate of 1:5, 1:7, and 1:10. Afterward, the gold solution was poured into the citrate solution, and the mixture was heated on a hot plate at 90 °C while stirring at 1,000 rpm with a magnetic bar. The resulting mixture was centrifuged using an ultracentrifuge at 16,000 rpm for 30 minutes, thereby obtaining a precipitate.

### (2) Use of glycol chitosan

An attempt was made to synthesize AuNP using glycol chitosan. Glycol chitosan (0.32 w/v %) was dissolved in distilled water, and heated on a hot plate at 90 °C. Afterward, the gold solution (1 mM) was slowly added dropwise to a glycol chitosan solution (dropwise), and the resulting mixture was heated on a hot plate at 90 °C while stirring at 1,000 rpm with a magnetic bar. As a result, the yellow solution gradually became colorless and then wine-colored within 20 minutes, confirming the formation of Glycol chitosan@AuNP. The mixture was centrifuged using an ultracentrifuge at 16,000 rpm for 30 minutes, thereby obtaining a precipitate.

### (3) Use of alginate

An attempt was made to synthesize AuNP using alginate. Alginate (0.2 w/v % and 0.4 w/v %) was dissolved in distilled water, and heated on a hot plate at 90 °C. Afterward, the gold solution (1 mM) was slowly added dropwise to the alginate solution. The mixture was heated on a hot plate at 90 °C while stirring it at 1,000 rpm with a magnetic bar. As a result, the yellow solution gradually became colorless and then wine-colored within 20 minutes, confirming the formation of Alginate chitosan@AuNP. The mixture was centrifuged using an ultracentrifuge at 16,000 rpm for 30 minutes, thereby obtaining a precipitate.

### (4) Characteristics of AuNP

An attempt was made to confirm the characteristics of various proportions of AuNPs prepared according to Examples 1.(1) to (3). The size of nanoparticles, lattice size, and XRD were analyzed using a zeta potential, DLS, PDI, and TEM. The analysis results are shown in Table 1 below.

**[Table 1]**

| | | Yield (%) | Zeta potential (mv) | DLS (d.nm) | PDI | TEM (nm) | Lattice parameter (Å) | XRD-Lattice parameter |
|---|---|---|---|---|---|---|---|---|
| Citrate@AuNP | 1:5 | 30 | -37.6 ± 1.3 | 29.4 ± 0.5 | 0.589±0.001 | 27.1 ± 13.6 | 2.3 | 4.07 |
| | 1:7 | 22 | -38.3 ± 2.9 | 23.5 ± 0.9 | 0.57±0.02 | 19.0 ± 4.1 | 2.0 | 4.07 |
| | 1:10 | 10 | -36.1 ± 1.0 | 29.4 ± 1.7 | 0.59±0.09 | 13.5 ± 3.1 | 2.3 | 4.07 |
| Alginate@AuNP | 0.2% | 20 | -34.0 ± 10.0 | 56.5 ± 2.4 | 0.59±0.04 | 14.9 ± 3.1 | 2.4 | 4.04 |
| | 0.4 % | 25 | -45.4 ± 1.1 | 47.0 ± 2.6 | 0.56±0.017 | 24.4 ± 6.5 | 2.2 | 4.06 |
| Glycol chitosan@AuNP | 0.32% | 14 | 29.99 ± 1.36 | 81.45 ± 1.16 | 0.41±0.01 | 8.6 ± 2.11 | 6.6 | 4.05 |

The zeta potentials showed that Citrate@AuNP and Alginate@AuNP have negative charges, while Glycol chitosan@AuNP has a positive charge. DLS and PDI showed the size and safety of AuNPs in an aqueous solution, and TEM and a lattice parameter showed the size of the gold particles themselves, and the presence and size of the lattice structure. In addition, the presence or absence of gold particles was confirmed by XRD analysis.

### 2. Confirmation of AuNP synthesis

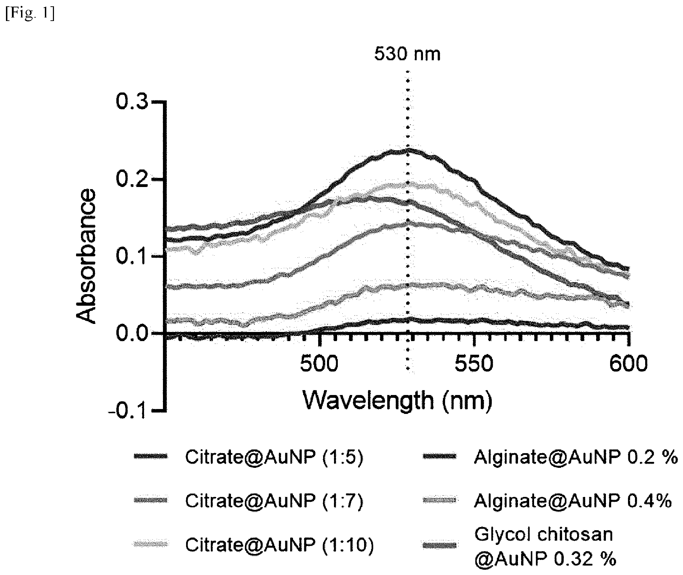

To confirm the synthesis of AuNPs of Examples 1.(1) to (3) from surface plasmon resonance (SPR) peaks, a UV-visible spectrophotometer (NanoDrop 2000; Thermo Scientific, Wilmington, USA) was used. Specifically, the AuNPs synthesized in various proportions (Citrate@AuNP (1:5), Citrate@AuNP (1:7), Citrate@AuNP (1:10), Alginate@AuNP 0.2%, Alginate@AuNP 0.4%, and Glycol chitosan@AuNP 0.32%) were measured using an UV-visible spectrophotometer. It was confirmed that the SPR peaks of all of the synthesized AuNPs were detected at approximately 530 nm (FIG. 1).

### 3. Confirmation of characteristics of AuNPs

### (1) Analysis of X-ray diffractometer (XRD) patterns of AuNPs

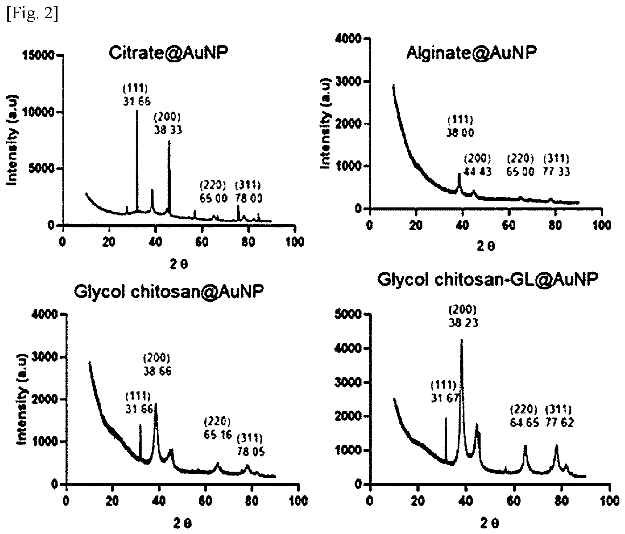

To confirm the presence or absence of the gold element in AuNPs, the synthesized AuNPs was put into a freeze dryer to form a powder. The characteristics of AuNPs were identified using XRD, D8 ADVANCE, and BRUKER. As a result, the peaks at 2θ = 31.6°, 38.2°, 64.6°, and 77.6° were assigned to (111), (200), (220) and (311) lattice planes of Au(0), respectively, confirming that gold was present in the nanoparticles coated with various materials (FIG. 2).

### (2) TEM and TEM lattice analysis of AuNPs

To confirm the size and lattice size of AuNPs, solution-type AuNPs were diluted approximately 1/20 in deionized water (DW). Here, the solution was added dropwise to cover a Cu TEM grid. A transmission electron microscope (JEM 2100F, JEOL) was used to check if the nanoparticles had a particle form, and to measure their sizes.

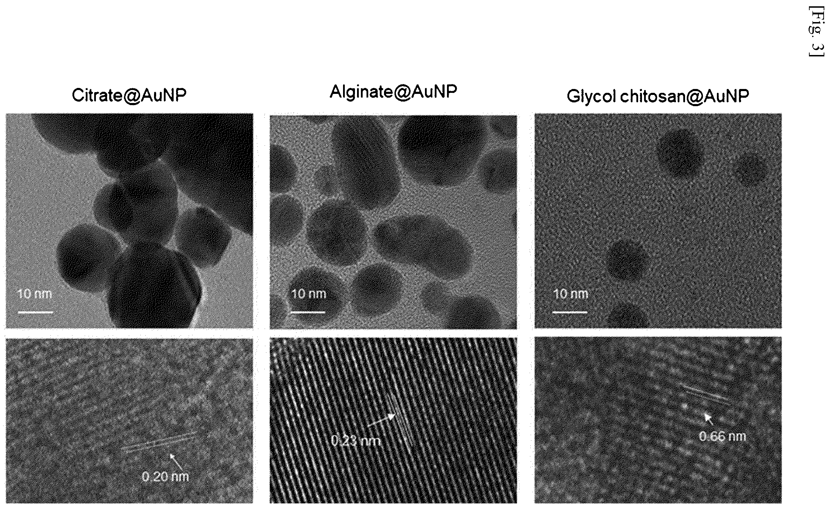

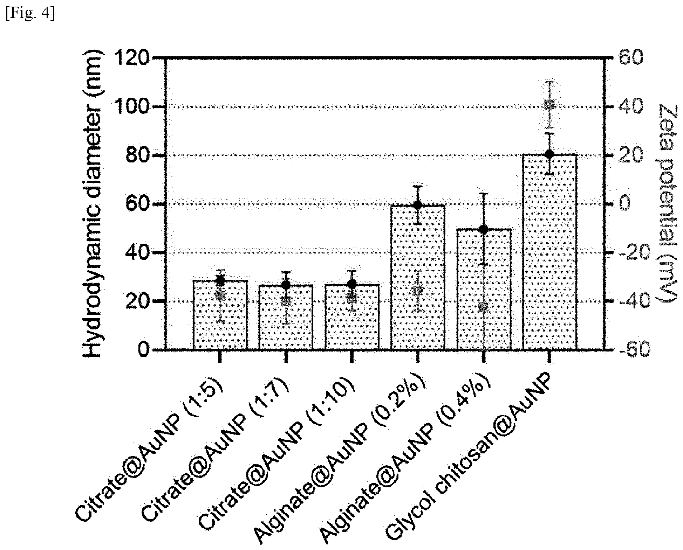

The above results are shown in FIG. 3. Specifically, the TEM image of Citrate@AuNP (1:10) showed that the size of the nanoparticles was approximately 27 nm, the TEM image of Alginate@AuNP 0.2% showed that the size of the nanoparticles was approximately 14.9 nm, and the TEM image of Glycol chitosan@AuNP 0.32% showed that the size of the nanoparticles was approximately 8 nm (TEM (nm) shown in FIG. 4). Accordingly, all three types of nanoparticles are formed in a round shape. In addition, by measuring the lattice size inside the AuNPs, it was confirmed that the lattice sizes of Citrate@AuNP (1:10), Alginate@AuNP 0.2%, and Glycol chitosan@AuNP 0.32% were 2.3 (Å), 2.4 (Å), and 6.6 (Å), respectively.

### (3) Measurement of zeta potential (ZP), dynamic light scattering (DLS), and poly dispersity index (PDI) values of AuNPs

To measure the ZP, DLS, and PDI values of AuNPs, 800 µL of solution-type AuNPs were put into a cuvette and the values were measured. The physical properties of the AuNPs were measured using a Zetasizer Nano ZS (Malvern Panalytical, Worcestershire, UK).

The results are shown in FIG. 4. Specifically, it was confirmed that the surface charge of the AuNPs varies depending on the coated material. It was confirmed that Citrate@AuNP (1:10) showed a negative charge of -36.2 ± 2.9 mV, Alginate@AuNP 0.2% showed a negative charge of -34.5 ± 11.1 mV, and Glycol chitosan@AuNP 0.32% showed a positive charge of 30.1 ± 1.36 mV. The DLS value was approximately 30 nm for Citrate@AuNP (1:10), approximately 60 nm for Alginate@AuNP 0.2%, and approximately 80 nm for Glycol chitosan@AuNP 0.32%.

### 4. Confirmation of colloidal stability of AuNPs according to pH

An attempt was made to confirm whether Citrate@AuNP (1:10), Alginate@AuNP (0.2%), and Glycol chitosan@AuNP (0.32%) were stably maintained in a biomimetic environment, that is, the GI tract biomimicking environment. Specifically, the AuNPs were adjusted to pH 2 and pH 6 with HCl and NaOH, respectively. Afterward, after 0, 1, 3, 6, 9, and 12 hours, the AuNPs were measured using an UV-visible spectrophotometer (NanoDrop 2000; Thermo Scientific, Wilmington, USA).

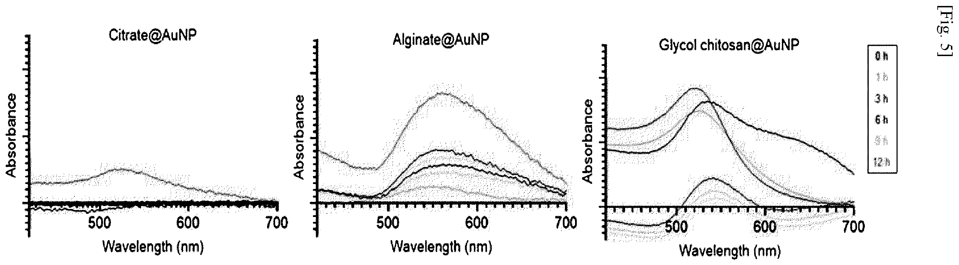

As a result, it was confirmed that Citrate@AuNP (1:10) and Alginate@AuNP show SPR peak collapse starting from 1 hour in a pH 2 environment, while Glycol chitosan@AuNP 0.32% can maintain the peak for up to 3 hours (FIG. 5). This is because, in an acidic environment of pH 2, a carboxylic acid of citrate or alginate is protonated, which neutralizes its surface charge and causes AuNPs to aggregate. However, the AuNPs coated with glycol chitosan having many amine groups can avoid the resulting conjugation.

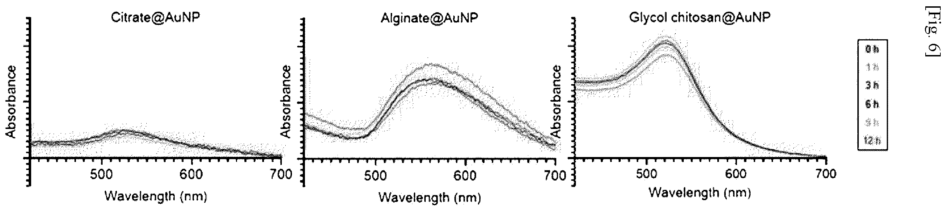

In addition, it was confirmed that, in a pH 5 environment, the colloidal stability of all AuNPs was maintained for 12 hours (FIG. 6). From the results of FIGS. 5 and 6, it was expected that Glycol chitosan@AuNP, which had been orally administered, would be stably maintained in the stomach and intestines.

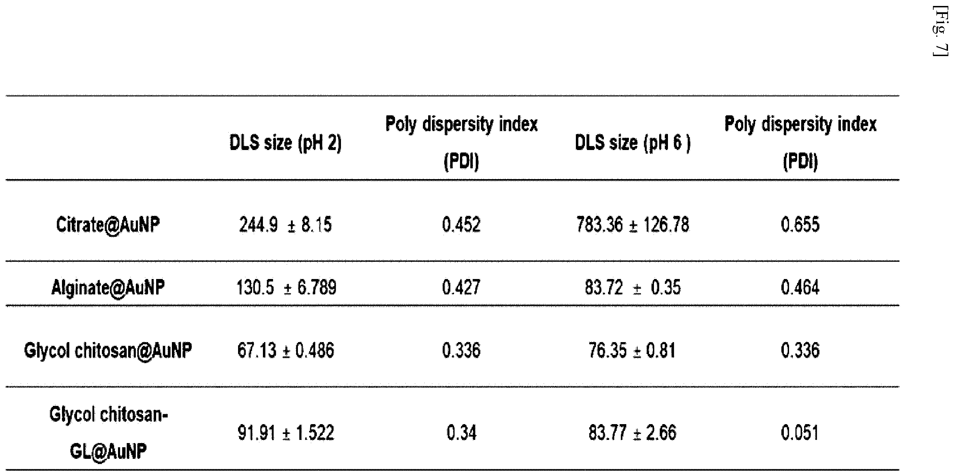

Furthermore, when AuNPs were left at pH 2 and pH 6 for 12 hours, the size of the particles in the aqueous solution was determined, and as a result of measuring PDI values to confirm whether the particles were stably and uniformly dispersed, it was confirmed that all PDI values were 0.5 or less, indicating that the particles were dispersed in a stable and uniform manner (FIG. 7).

### 5. 3,3',5,5'-tetramethylbenzidine (TMB) assay

### (1) Confirmation of peroxidase-like activity of AuNPs

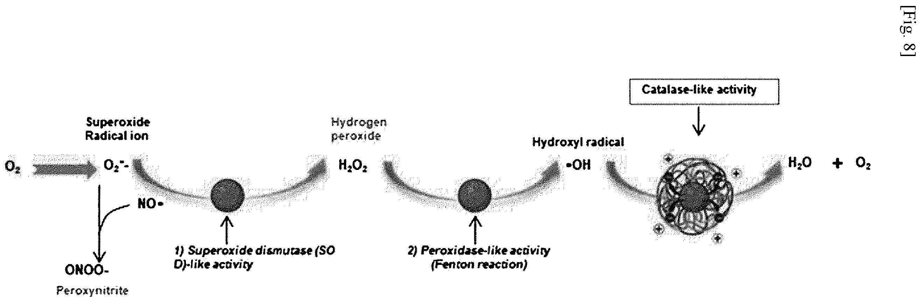

To confirm the peroxidase property of AuNPs, AuNPs were reacted with 80 x 10⁻³ M H₂O₂ and 3,3',5,5'-TMB, adjusted to pH 7.4, pH 5.8, and pH 2.1, for 5 minutes. Afterward, the solution prepared by reacting TMB (pH 7.4, pH 5.8, and pH 2.1 assay buffer with 80 x 10⁻³ M H₂O₂) and horseradish peroxidase (HRP) (1,000 units/mL) for 5 minutes was used as a positive control. The results of peroxidase-like activity (%) were normalized by HRP activity at pH 5.8. Here, the schematic diagram illustrating the ROS scavenging of Glycol chitosan@AuNP is shown in FIG. 8.

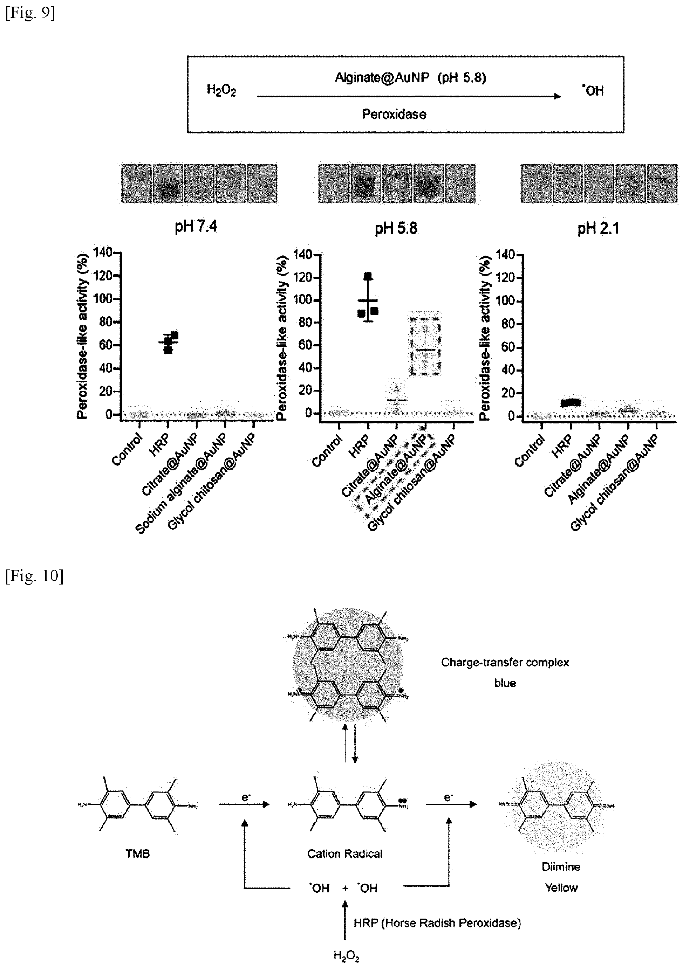

The results of confirming the peroxidase-like activity of AuNPs are shown in FIG. 9. Specifically, TMB is a material used in colorimetric assay, and reacts with •OH produced by H₂O₂ and HRP to produce a blue intermediate or a yellow end product. When •OH is completely removed, it becomes colorless. At pH 7.4, which is similar to the pH in the human body, all three types of AuNPs were confirmed to have little peroxidase activity. In addition, at pH 5.8 similar to the pH of colon tissue, among the AuNPs, Citrate@AuNP and Glycol chitosan@AuNP showed peroxidase activity of approximately 20%, which is very low, but Alginate@AuNP showed higher peroxidase activity of approximately 60%, as compared to other AuNPs. In addition, as can be seen from the colorless glycol chitosan, Glycol chitosan@AuNP no longer had activity to catalyze TMB. That is, it was confirmed that Glycol chitosan@AuNP loses its original peroxidase activity. At pH 2.1 similar to the pH of the stomach, it was confirmed that both the AuNPs and HRP exhibited low peroxidase activity. In addition, the mechanism of color changes when a TMB solution was added after the H₂O₂ and HRP reactions is shown in FIG. 10.

### (2) Confirmation of catalase like activity of AuNPs

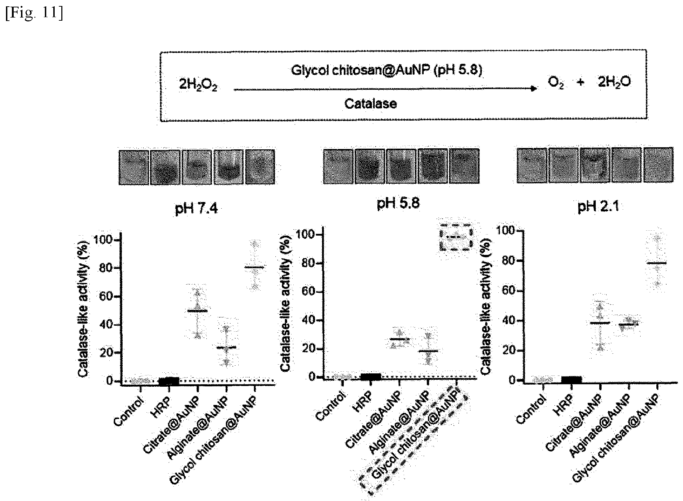

An attempt was made to confirm the catalase property of AuNPs. Specifically, to test H₂O₂ consumption by the catalase-like activity of Citrate@AuNP, Alginate@AuNP and Glycol-Chitosan@AuNP(125 µg/mL), TMB (pH 7.4, pH 5.8 and pH 2.1 assay buffer with 80 x 10⁻³ M H₂O₂) was reacted with the AuNPs for 5 minutes, and a HRP solution (1,000 units/mL) was added to detect remaining H₂O₂. As a result, catalase-like activity (%) was normalized at pH 5.8 based on the activity of Glycol chitosan@AuNP. In addition, it was confirmed that Citrate@AuNP and Alginate@AuNP turned yellow before adding HRP, while glycol chitosan turned colorless (FIG. 11). At pH 5.8 similar to the colon tissue pH, among the AuNPs, Citrate@AuNP and Glycol chitosan@AuNP were confirmed to have low catalase activity of approximately 20%, but Alginate@AuNP was confirmed to have higher peroxidase activity of approximately 60%, compared to other AuNPs. In addition, as can be seen from the colorless glycol chitosan, Glycol chitosan@AuNP no longer had activity to catalyze TMB. That is, it was confirmed that Glycol chitosan@AuNP loses its original peroxidase activity. It was confirmed that, at pH 2.1 similar to the pH of the stomach, both AuNPs and HRP showed low peroxidase activity.

### 6. Experiment to confirm dioxygen bubbles

### (1) Confirmation of oxygen bubbles through reaction with AuNPs

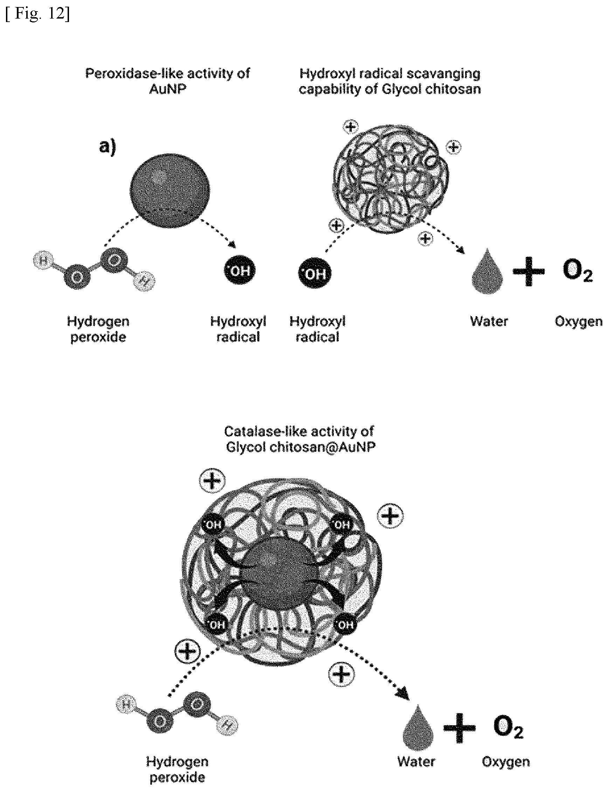

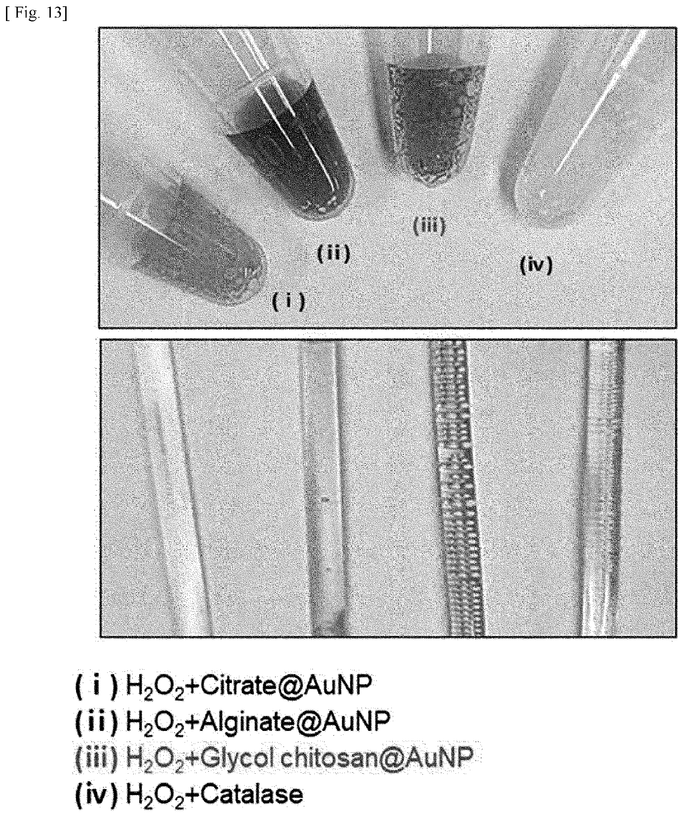

This experiment was conducted to confirm the oxygen produced through the reaction between the AuNPs and hydroxyl radicals, and to confirm the catalase-like activity of Glycol chitosan@AuNP, each of catalase and Glycol chitosan@AuNP was added to a H₂O₂ solution and the formation of oxygen bubbles was observed. When bubbles were observed, it can be expected that they are the result of decomposition into water and oxygen, which are the end products produced by H₂O₂ and catalase-like activity. The hydrogen peroxide scavenging mechanism of Glycol chitosan@AuNP is shown in FIG. 12. Specifically, 80 x 10⁻³ M H₂O₂, Citrate@AuNP (125 µg/mL), Alginate@AuNP (125 µg/mL), Glycol chitosan@AuNP(125 µg/mL), and 1,000 units/mL of catalase were reacted for 5 minutes and the formation of oxygen bubbles was observed. As a result, it was confirmed that Glycol chitosan@AuNP produced bubbles in a very similar amount to Catalase@AuNP (FIG. 13).

### (2) Confirmation of oxygen bubbles through hydroxyl radical reaction with AuNP-coated material

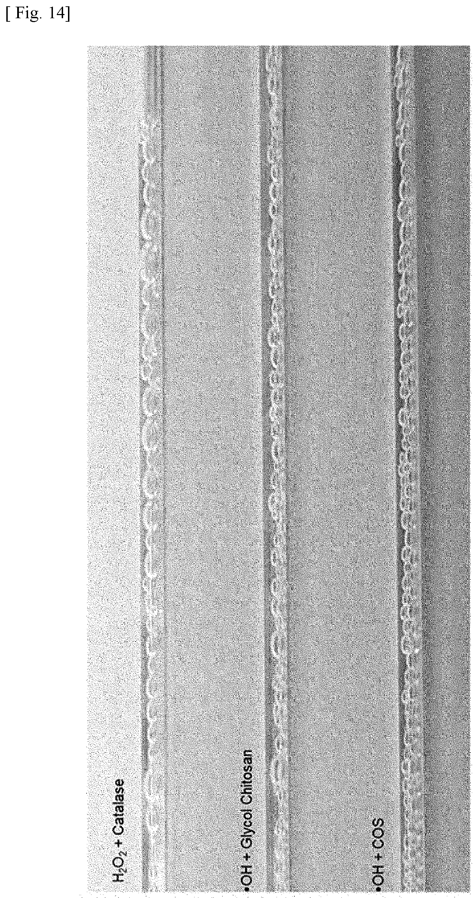

The experiment was conducted to confirm oxygen generation by the hydroxyl radical reaction between glycol chitosan, which is a coating material, and chitosan oligosaccharide (COS) having a similar structure thereto. Five minutes after reaction of hydrogen peroxide with catalase, whether bubbles were generated was checked. Specifically, oxygen bubbles were observed by reacting i) 80 x 10⁻³ M H₂O₂ and glycol chitosan (125 µg/mL) or (ii) chitosan oligosaccharide (COS)(125 µg/mL) and 1,000 units/mL of catalase for 5 minutes. To confirm peroxidase-like activity, HRP (1,000 units/mL) was set as a positive control and normalized based on HRP activity. After reacting HRP with hydrogen peroxide to produce hydroxyl radicals, it was confirmed that bubbles were generated by the hydroxyl radicals reacting with glycol chitosan or COS (FIG. 14). Therefore, it was found that the amine group was oxidized and converted to water and oxygen

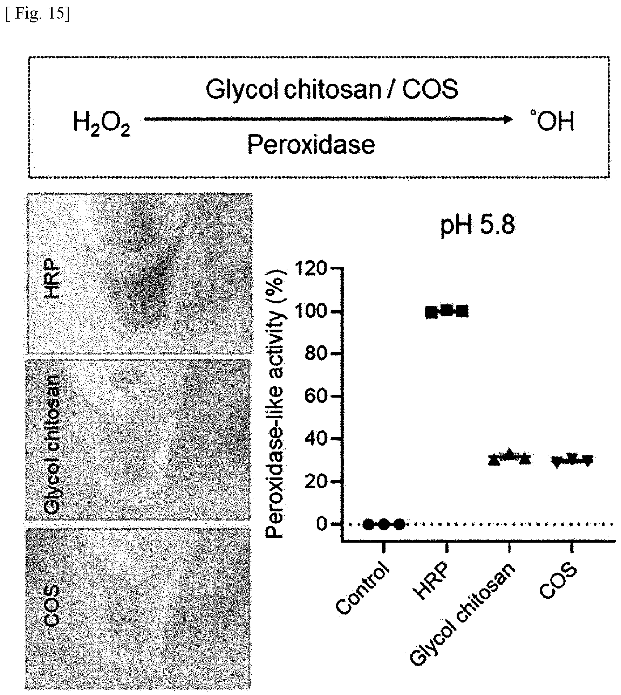

In addition, as a result of performing a TMB assay on glycol chitosan and COS, when glycol chitosan reacted with TMB and H₂O₂ or when COS reacted with TMB and H₂O₂, the color changed to colorless, indicating that they have peroxidase activity (FIG. 15).

### 7. Confirmation of catalase-like activities of amine-rich glycol chitosan and COS

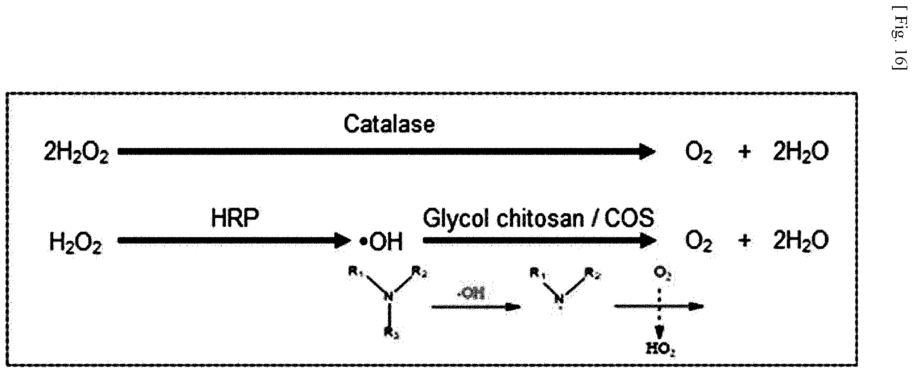

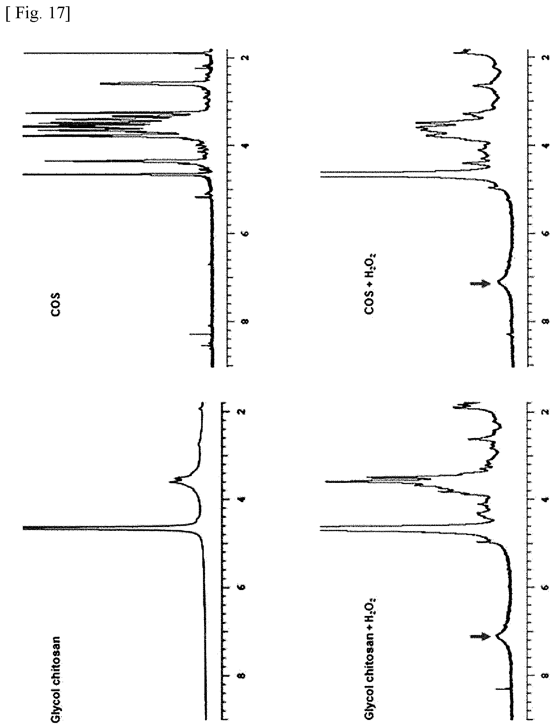

An amine group was oxidized through the reaction of a hydroxyl radical, and the resulting change in functional group was detected by H-NMR. Here, the reaction scheme of H₂O₂ and catalase is shown in FIG. 16. Specifically, glycol chitosan (125 µg/mL) and COS (125 µg/mL) were dissolved in heavy hydrogen (D₂O). Afterward, glycol chitosan (125 µg/mL), H₂O₂ (80 x 10⁻³ M), and HRP (1,000 units/mL) were reacted together for 5 minutes. In addition, COS (125 µg/mL), H₂O₂ (80 x 10⁻³ M), and HRP (1,000 units/mL) were reacted for 5 minutes, and the catalase-like activities of amine-rich glycol chitosan and COS were measured by H-nuclear magnetic resonance (NMR). As a result, it was confirmed that, when glycol chitosan and a hydroxyl radical react with each other, the amine group of glycol chitosan is oxidized, and the peak of the product obtained in oxidation appears between approximately 6.5 and 7.2 ppm (FIG. 17).

### 8. Confirmation of cytotoxicity of gold nanoparticles to intestinal epithelial cells

Caco-2 cells (intestinal epithelial cells), which are immortalized human colorectal adenocarcinoma cells (Korean Cell Line Bank, Seoul, Korea), were treated with various proportions of AuNPs to evaluate cytotoxicity. Cell viability was assessed using a lactate dehydrogenase (LDH) cytotoxicity assay kit. Specifically, Caco-2 cells (1 x 10⁴ cells/well) were seeded in a 96-well plate and cultured in a CO₂ incubator at 37 °C for 48 hours. After washing the cells with PBS, the cells were treated with Citrate@AuNP (1:10), Alginate@AuNP 0.2%, and Glycol chitosan@AuNP 0.32% (7.8 µg/mL, 15.6 µg/mL, 31.3 µg/mL, 62.5 µg/mL and 125 µg/mL) and reacted for 36 hours. For Blank, only 200 µL of a medium was added to three completely empty wells (wells without cells), and for a positive control, 200 µL of 1% Triton X-100 was added to the cells. 100 µL of the cell supernatant was transferred to a new Eppendorf tube. In the dark, 100 µL of an LDH reaction solution (MTT solution, NAD⁺ solution, PES solution, and lactate solution) was added to the tube, and incubated in a 37 °C incubator for up to 30 minutes. Afterward, the tube was centrifuged at 20,000g for 15 minutes, and the supernatant of the mixture was transferred to a microplate, and the absorbance was measured at 565 nm using a microplate reader. The absorbance of Blank was eliminated from the total absorbance, and the cytotoxicity (%) was calculated as (Aₛₐₘₚₗₑ-A_{background})/(Aₘₐₓᵢₘᵤₘ-Aₛₚₒₙₜₐₙₑₒᵤₛ) x 100.

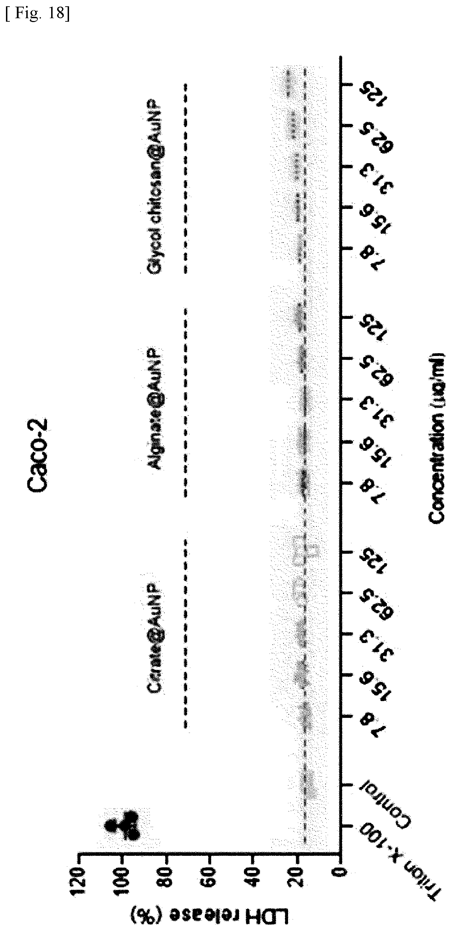

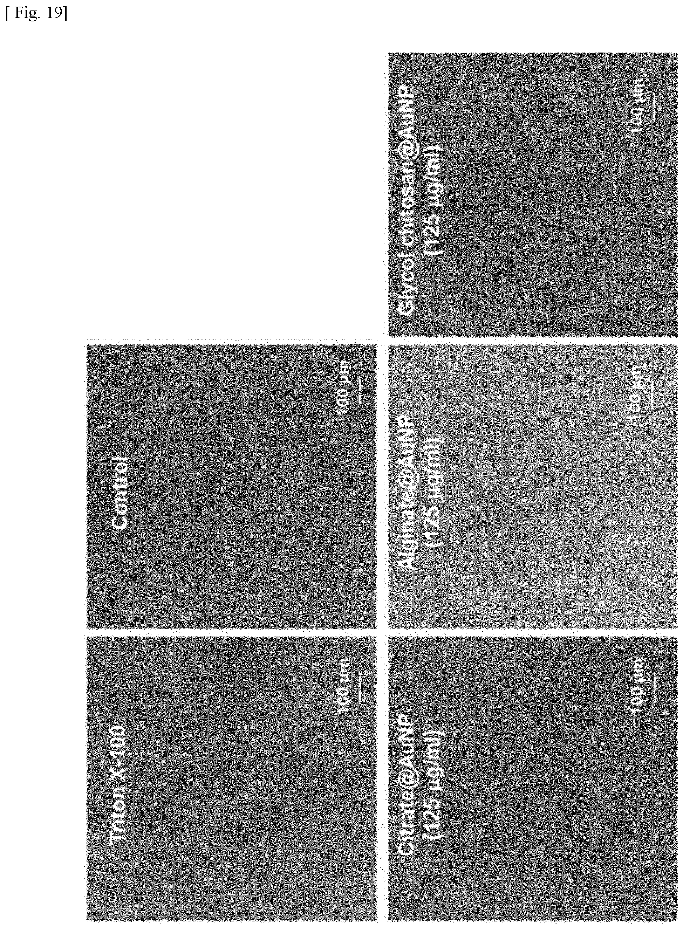

As a result, it was confirmed that there was almost no toxicity at all concentrations (FIG. 18). In addition, as a result of confirming the morphology of Caco-2 cells 36 hours after the treatment of Citrate@AuNP (1:10), Alginate@AuNP 0.2%, and Glycol chitosan@AuNP 0.32% at various concentrations (7.8 µg/mL, 15.6 µg/mL, 31.3 µg/mL, 62.5 µg/mL and 125 µg/mL), it was confirmed that, similar to the control, Caco-2 cells formed a monolayer well (FIG. 19).

### 9. Confirmation of cytotoxicity of AuNPs to immune cells

Murine macrophages Raw 264.7 cells (Korean Cell line Bank, Seoul, Korea), which were established from tumors of male mice induced by Abelson mouse leukemia virus, were treated with various proportions of AuNPs to evaluate cytotoxicity. Cell viability was assessed using a LDH cytotoxicity assay kit. Specifically, Raw 264.7 cells were seeded in a 96-well plate (1 X 10⁴ cells/well) and cultured in a CO₂ incubator at 37 °C for 24 hours. After washing the cells with PBS, the cells were treated with Citrate@AuNP (1:10), Alginate@AuNP 0.2%, and Glycol chitosan@AuNP 0.32% (7.8 µg/mL, 15.6 µg/mL, 31.3 µg/mL, 62.5 µg/mL, and 125 µg/mL) and reacted for 18 hours. For Blank, only 200 µL of a medium was added to three completely empty wells (wells without cells), and for a positive control, 200 µL of 1% Triton X-100 was added to the cells. 100 µL of the cell supernatant was transferred to a new Eppendorf tube. In the dark, 100 µL of a LDH reaction solution (MTT solution, NAD⁺ solution, PES solution, and lactate solution) was added to the tube, and incubated in an incubator at 37 °C for up to 30 minutes. Afterward, the tube was centrifuged at 20,000g for 15 minutes, and the supernatant of the mixture was transferred to a microplate, and the absorbance was measured at 565 nm using a microplate reader. The absorbance of Blank was eliminated from the total absorbance, and the cytotoxicity (%) was calculated as (Aₛₐₘₚₗₑ-A_{background})/(Aₘₐₓᵢₘᵤₘ-Aₛₚₒₙₜₐₙₑₒᵤₛ) x 100.

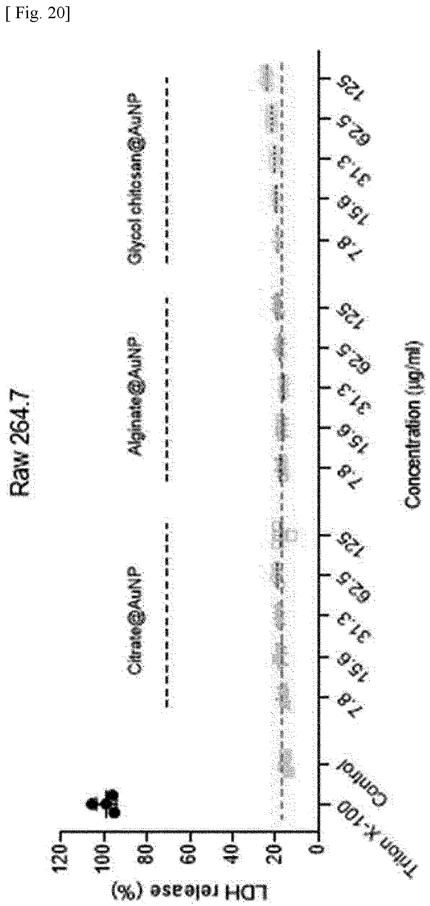

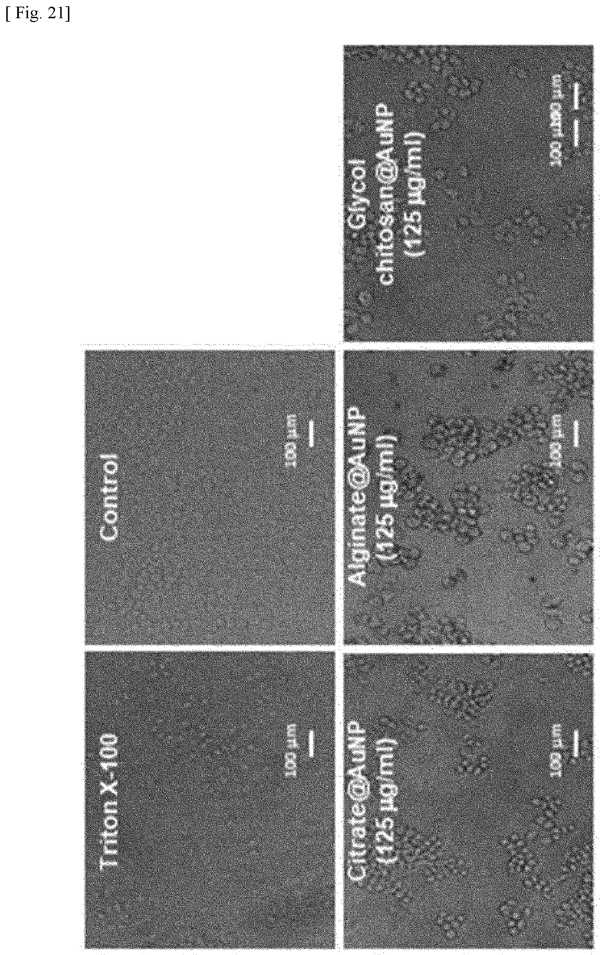

As a result, it was confirmed that there was almost no toxicity at all concentrations (FIG. 20). In addition, as a result of confirming the cell morphology of Raw 264.7 cells 36 hours after the treatment of Citrate@AuNP (1:10), Alginate@AuNP 0.2%, and Glycol chitosan@AuNP 0.32% at various concentrations (7.8 µg/mL, 15.6 µg/mL, 31.3 µg/mL, 62.5 µg/mL and 125 µg/mL), when the Raw 264.7 cells were activated by toxicity, the cells changed into a pointed shape. However, it was confirmed that such a shape did not appear when the cells were treated with AuNPs (FIG. 21).

### 10. Confirmation of ROS scavenging activity

This experiment was conducted to confirm the ROS scavenging capability of AuNPs. To prepare ABTS radicals (ABTS•), a 14 mM ABTS solution and 4.9 mM potassium persulfate were dissolved in DW. Afterward, potassium persulfate was added to the ABTS solution to adjust the final concentration to 2.45 mM. The mixture was incubated overnight (approximately 12 to 16 hours). The ABTS• solution was detected at 734 nm, and diluted in DW at 734 nm until a value of 0.8 was obtained. 500 µL of the ABTS• solution was added to a tube containing 500 µL of AuNPs, and incubated at 50 rpm for 15 minutes using an orbital shaker. Afterward, the resultant was centrifuged at 20,000 g for 10 minutes using a minicentrifuge, 100 µL of the supernatant was taken and added to a 96-well plate carefully so that the pellet did not come together. The plate was detected at 734 nm, and radical scavenging activity was calculated as I%=[(Abs0-Abs1)/Abs0]×100 (Abs 0: ABTS., ABS 1: sample).

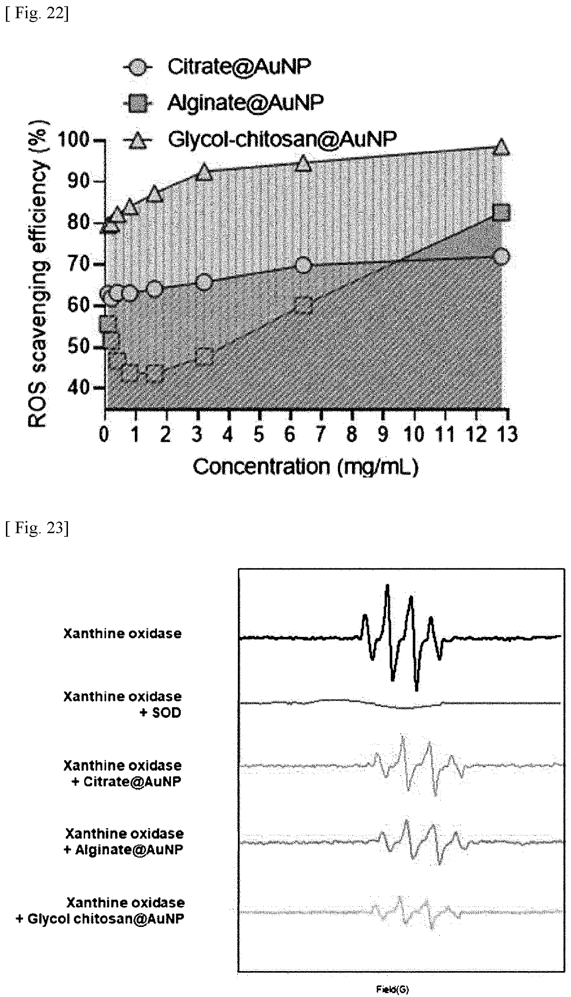

As a result, it was confirmed that Glycol chitosan@AuNP showed the highest performance and that it was concentration-dependent. In addition, it was confirmed that scavenging capability was superior in the order of Citrate@AuNP, Alginate@AuNP, and Glycol chitosan@AuNP (FIG. 22).

### 11. Measurement of electron spin resonance to confirm superoxide dismutase (SOD)-like activity of AuNPs

This experiment was conducted to identify the superoxide dismutase (SOD) properties of AuNPs. 25 µM diethylenetriaminepentaacetic acid (DTPA), 1 mM hypoxanthine, 1 unit/mL of hypoxanthine oxidase, 1 M DMPO, 1,000 units/mL of SOD solution (dissolved in DW) were prepared in 100 mM phosphate buffer (pH 7.4). 700 µL of DTPA solution, 200 µL of DMPO solution, and 1 mL of hypoxanthine were combined and adjusted to have a final concentration of each of 17.5 µM, 10 mM and 0.5 mM. The mixture is a concentrate before making superoxide radicals, 47.5 µL of the reaction solution was transferred, 2.5 µL of xanthine oxidase was added so that the final concentration of the xanthine oxidase was adjusted to 0.05 unit/mL. Afterward, the resulting mixture was reacted for 30 seconds, and the group was used as a positive control. 50 µL of SOD was added to the solution of the positive control and reacted for 30 seconds so that the final concentration was adjusted to 500 unit/mL. 50 µL each of citrate@AuNPs (1:5), (1:7) and (1:10), Glycol chitosan@AuNP 0.32%, and Alginate@AuNP 0.2% was added to the control solution to adjust the final concentration to 125 µg/mL, and then the resulting mixture was reacted for 2 minutes. Afterward, the SOD-like activity of AuNPs was measured using an electron spin resonance spectrometer(EMXplus-9.5/12/P/L System (Bruker, Germany)).

SOD plays a major role in an antioxidant effect by converting superoxide into H₂O₂ and oxygen. To confirm whether the synthesized AuNPs have SOD-like activity, an ESR experiment was conducted, and the stronger the intensity of the y-axis, the more superoxide was produced. When superoxide was produced with xanthine oxidase and hypoxanthine, the strongest intensity was observed and it was judged as a positive control. Afterward, in the case of treatment with SOD, it was confirmed that the degree of intensity was reduced. When Citrate@AuNP (1:10), Alginate@AuNP 0.2%, and Glycol chitosan@AuNP 0.32% were treated in a similar manner, all intensities decreased, showing that all three synthesized AuNPs have SOD-like activity. Among them, the intensity of glycol chitosan was reduced the most, showing the best superoxide inhibitory effect (FIG. 23).

### 12. Confirmation of intracellular ROS scavenging activity

A dichlorofluorescein-diacetate (DCF-DA) assay was performed to confirm the ROS scavenging capability of AuNPs in intestinal epithelial cells when H₂O₂ was treated along with AuNPs. Caco-2 cells were cultured in a 96-well plate at 1.0 x 10⁴ cells/well. Each of Citrate@AuNP (1:10), Alginate@AuNP 0.2%, and Glycol chitosan@AuNP 0.32% was reacted with H₂O₂ (100 µM), added to each well, and reacted for 4 hours. After 4 hours, the cells were stained with 2', 7'-dichlorofluorescin diacetate (DCF-DA) for 15 minutes (final concentration of 2 µM), and washed with PBS 3 times. Intracellular ROS production was detected using a microreader after irradiation at 490 nm (excitation) and 520 nm (emission). Here, the group treated with H₂O₂ alone was used as a positive control.

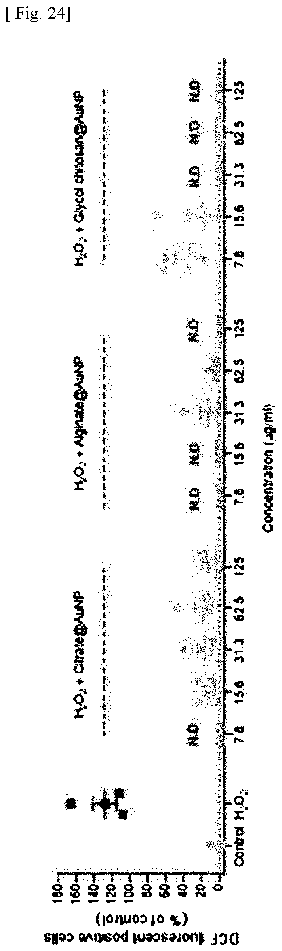

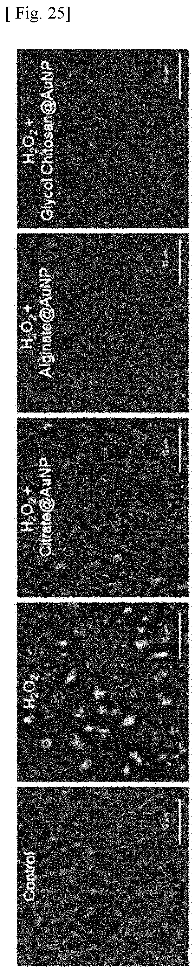

As a result, it was confirmed that DCF fluorescence values decreased in almost all AuNP-treated groups, compared to the positive control (FIG. 24). In addition, the positive control shows strong fluorescence, indicating that a large quantity of ROS is produced. In the AuNP-treated groups, almost all fluorescence disappeared, indicating that AuNPs inhibited intracellular ROS production (FIG. 25).

### 13. Confirmation of RNS scavenging activity and nitric oxide (NO) production

When Raw 264.7 cells stimulated with lipopolysaccharide (LPS) were co-treated with LPS and AuNPs, the concentration of NO secretion was determined using the Griess reagent. Raw 264.7 cells seeded in a 48-well plate (5 x 10⁵ cells/well) were cultured in a complete medium (DMEM, FBS 10%, PS 1%) for 24 hours. Afterward, the cells were cultured in a medium with/without AuNPs for 5 hours, washed with PBS, and incubated in a medium with/without LPS (1 µg/mL) for 20 hours. The cell supernatant (100 µL) was transferred to a 96-well plate and mixed with an equal volume of Griess reagent for 10 minutes, and then the absorbance on the plate was measured at 546 nm. An NO concentration was calculated using an NO standard curve (0 to 10 µM). Here, the positive control is a group treated with LPS (1 µg/mL) alone.

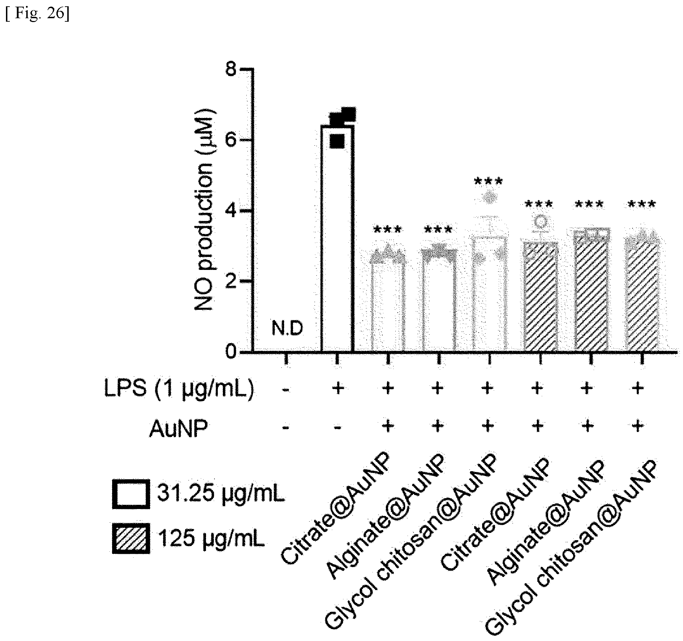

It was confirmed that, when all AuNPs were treated at a low concentration of 31.25 µg/mL, approximately 50% of a high concentration (125 µg/mL) of NO₂ was removed, confirming that AuNP treatment reduces NO production and enables RNS scavenging (FIG. 26).

### 14. Confirmation of tight junctions by immunocytochemistry (ICC)

It was confirmed whether tight junctions (ZO-1, Occludin) recovered when Caco-2 cells stimulated by H₂O₂ were treated with AuNPs. The Caco-2 cells (1 X 10⁶ cells/well) were seeded in a 6-well plate and then cultured for 48 hours. The Caco-2 cells were simply washed with PBS, and fixed with 4%paraformaldehyde. Afterward, the cells were washed twice with PBS-Tween 20 for approximately 2 minutes, and for blocking, the cells were incubated in 20% goat serum-containing PBS-Tween 20 as a solvent at room temperature for 30 minutes. The goat serum (20% in PBST-20) and primary antibodies (anti-mouse occludin, anti-rabbit ZO-1) that were diluted 100:1 were incubated at room temperature for 1 hour, and washed with PBS. In addition, secondary antibodies (goat anti mouse-488, goat anti rabbit-594) were diluted in PBS in a ratio of 1:200 and incubated with the cells. The cells were blocked from light and washed twice with PBS for 3 minutes. After adding one or two drops of DAPI mounting solution (blue), the slide was covered with a cover slide and observed under a fluorescence microscope.

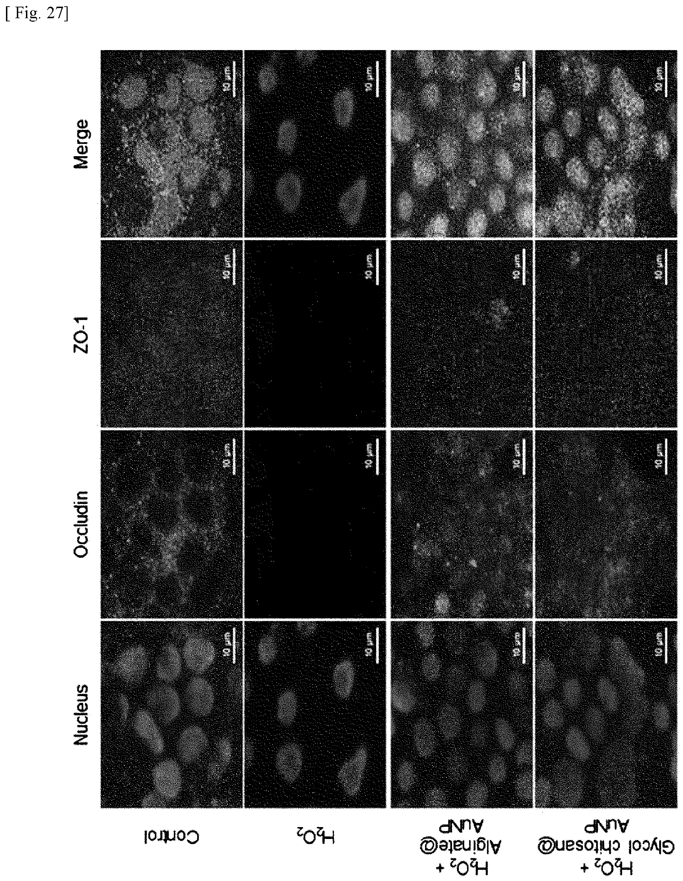

As a result, it was confirmed that the oxidative stress caused by H₂O₂ destroyed a tight junction protein such as occludin (green) or zonula occludens-1 (Zo-1, red), resulting in the destruction of the monolayer of intestinal epithelial cells (FIG. 27). In addition, it was confirmed that the degree of monolayer destruction was very low in case of the co-treatment with AuNP and H₂O₂. Therefore, it was confirmed that AuNPs helped prevent the destruction of tight junctions.

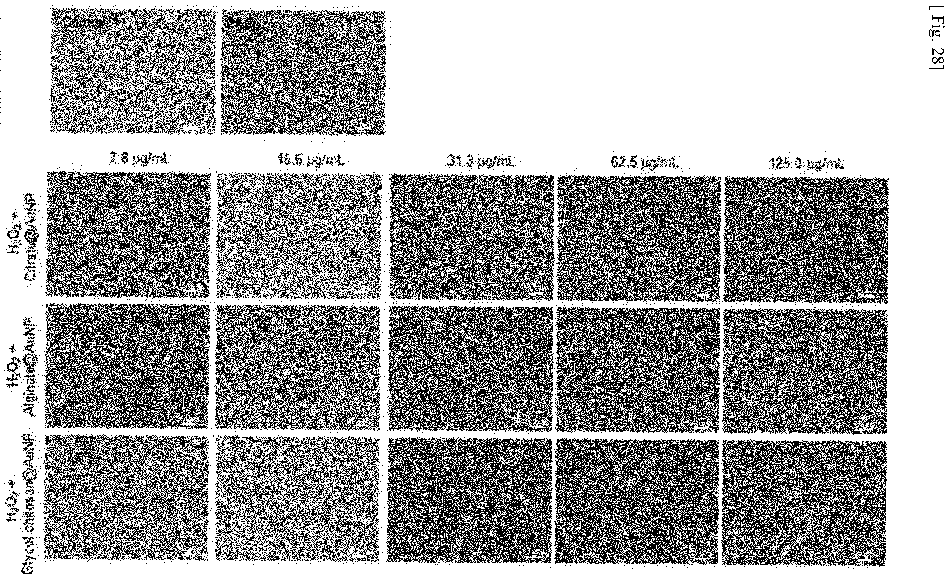

In addition, FIG. 28 shows the images that show the morphology of the Caco-2 cell tight junctions 36 hours after treatment with Citrate@AuNP, Alginate@AuNP, and Glycol chitosan@AuNP at various concentrations (7.8 µg/mL, 15.6 µg/mL, 31.3 µg/mL, 62.5 µg/mL, and 125 µg/mL).

### 15. Confirmation of M1 and M2 polarizations of Raw 264.7 cells

Flow cytometry was performed to determine the M1 and M2 polarization ratios in a group in which Raw 264.7 cells were treated with LPS and groups in which Raw 264.7 cells were co-treated with LPS and AuNPs (Alginate@AuNP and Glycol chitosan@AuNP). Raw 264.7 cells were seeded in a 100-mm dish (2.0 X 10⁶ cells) and cultured for 24 hours (DMEM; 10% FBS, 1% PS). Afterward, the cells were washed with PBS, and incubated with AuNPs (125 µg/mL) or without AuNPs for 5 hours. The cells were separated using a scraper and centrifuged at 12,000 rpm for 3 minutes. After removing the supernatant, the cells were redispersed with PBS and incubated with or without antibodies at room temperature for 20 minutes. The antibodies were CD206 APC-eFluor 780, CD80-FITC, CD 163-eFluor^{™} 450 (eBioscience^{™}), and iNOS-PE (eBioscience^{™}): 1) Negative control (CD206 APC-eFluor 780, CD80-FITC co-staining), positive control (non-staining), Raw cells and CD 206 marker (staining), Raw cells and CD 80 marker (staining), Raw cells, and CD 206 and CD 80 markers (co-staining), Alginate@AuNP-treated Raw cells stained with CD 206 and CD 80 (co-staining), and Glycol chitosan@AuNP-treated Raw cells stained with CD 206 and CD 80 (co-staining). 2) Negative control (CD163-eFluor^{™} 450, iNOS-PE co-staining), positive control (non-staining), Raw cells and CD 163 marker (staining), Raw cells and iNOS marker (staining), Raw cells stained with CD 163-eFluor^{™} 450 and iNOS-PE (co-staining), Alginate@AuNP-treated Raw cells stained with CD 163-eFluor^{™} 450 and iNOS-PE (co-staining), and Glycol chitosan@AuNP-treated Raw cells stained with CD 163-eFluor^{™} 450 and iNOS-PE (co-staining). The samples were analyzed by BD FACS (BD FACSCalibur, BD Bioscience, New Jersey, USA), and data was analyzed using FlowJo V10. The unstained positive control was used for gating.

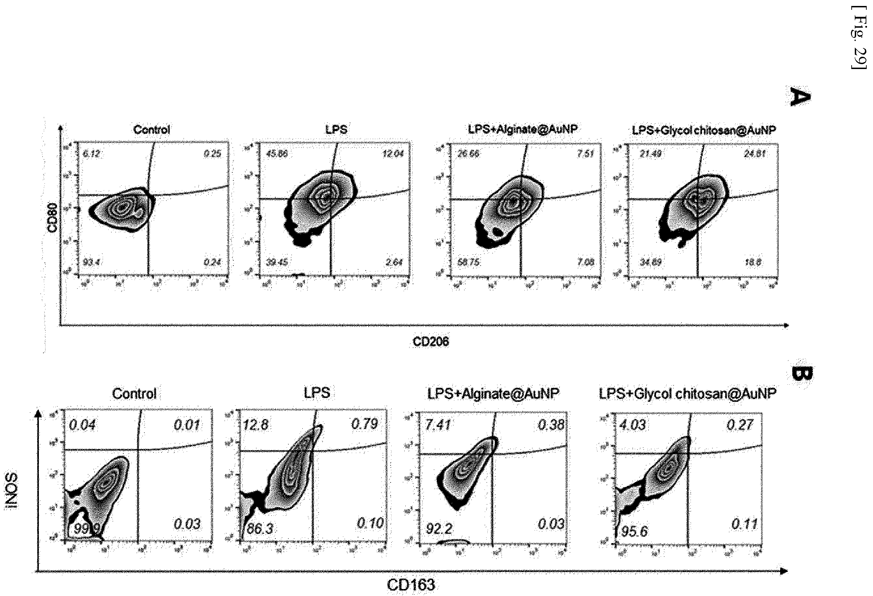

As a result, in the case of the above group 1), the M1 ratio of the cells stimulated with LPS increased by approximately 7 times to 45.86% compared to 6.12% of the negative control, and in the groups treated with AuNPs, the M1 ratio of the cells decreased to approximately 20%, which was approximately 50% lower than the positive control (FIG. 29A). In addition, in the case of the above group 2), the M1 ratio of the cells stimulated with LPS increased by 12.8%, compared to 0.04% of the negative control, but in the AuNP-treated groups, the M1 ratio of the cells decreased to approximately 7% and 4%, which were lower than the positive control (treated with LPS) (FIG. 29B).

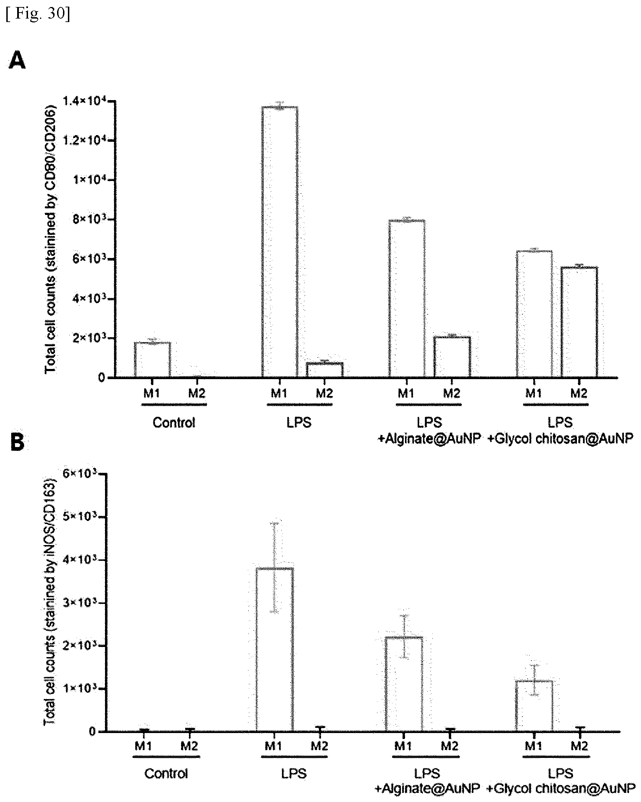

As a result of confirming M1 and M2 polarization populations of RAW 264.7 cells by FACS, in group 1), when the cells were stimulated with LPS, just like the FACS ratio curve, the M1 cell ratio increased greatly, but when the LPS-stimulating groups were treated with AuNPs, the M1 cell ratio decreased by approximately 50%. In addition, the M2 cell ratio increased, confirming that the cells have anti-inflammatory efficacy (FIG. 30A). The graph of the total cell number represents the average fluorescence intensity of stained cells. In the case of group 2), when the cells were stimulated with LPS, just like the FACS ratio curve, the M1 cell ratio increased greatly, but when the LPS-stimulating groups were treated with AuNPs, the M1 cell ratio decreased by approximately 50% (FIG. 30B).

### 16. Observation of morphology of Raw 264.7 cells

It was confirmed how well AuNPs could inhibit LPS-induced M1 polarization in Raw 264.7 cells. The Raw 264.7 cells were seeded in a 24-well plate (5.0 x 10⁴ per well) and cultured in a complete medium (DMEM, 10% FBS, 1% PS) for 24 hours. After complete culture, LPS (1 µg/mL) and AuNPs (Alginate@AuNP 125 µg/mL, and Glycol chitosan@AuNP 125 µg/mL) were co-cultured, and after 0, 1, 4, and 8 hours, the cell morphology of each culture was observed under a microscope.

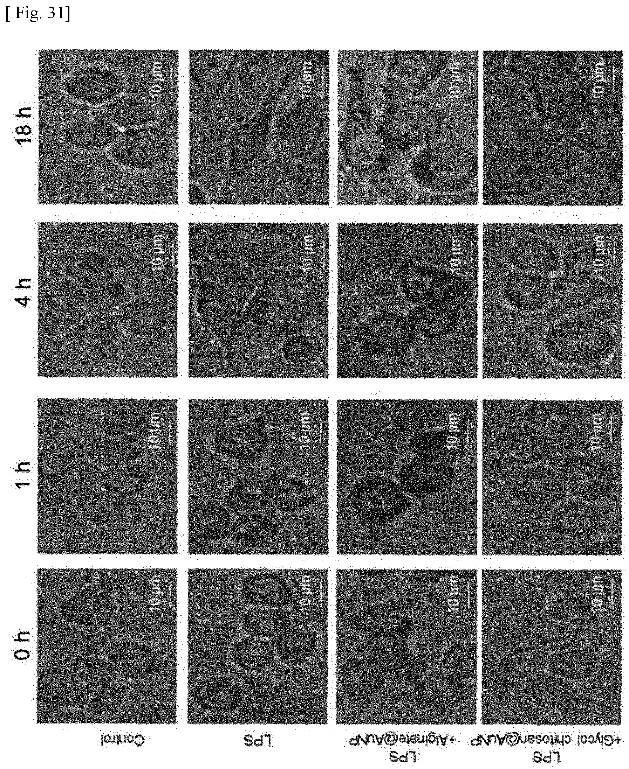

As a result, when stimulated only with LPS, the cells were in the state in which M1 was activated (pseudopodia) from 4 hours, but when treated with LPS and AuNP, the cells maintained a monocytic morphology, and were not activated to M1 (FIG. 31).

### 17. Confirmation of pro-inflammatory cytokine secretion of Raw 264.7 cells through PCR

PCR analysis was performed to confirm how much the secretion of pro-inflammatory cytokines in LPS-stimulated Raw 264.7 cells was suppressed by AuNPs. The Raw 264.7 cells were seeded in a 12-well plate (1.0 x 10⁵ per well) and cultured in a complete medium for 24 hours. Afterward, the cells were washed with PBS and incubated with AuNPs (Alginate@AuNP 125 µg/mL, and Glycol chitosan@AuNP 125 µg/mL) for 5 hours. Before incubating the cells in a medium with LPS (1 µg/mL) or without LPS for 20 hours, the cells were washed with PBS. The analyzed factors were TNF-α, IL-6, IL-1 beta, MCP-1, and iNOS. After extracting the total RNA of the cells using a QIAzol lysis reagent and an RNeasy mini kit (QIAGEN, Germany), 1 µg RNA was reverse-transcribed to cDNA using an iscript cDNA synthesis kit (BIO RAD Laboratories, USA). Afterward, qRT-PCR was performed on an Applied Biosystems instrument (USA) using SYBR Green PCR Master Mix (Applied Biosystems^{™}, USA). The relative mRNA expression of the genes shown in Table 2 below was normalized using GAPDH as an endogenous control.

**[Table 2]**

| | Sequence | SEQ ID NO: |
|---|---|---|
| GAPDH | (Forward) CAGGAGGCATTGCTGATGAT | SEQ ID NO: 1 |
| | (Reverse) GAAGGCTGGGGCTCATTT | SEQ ID NO: 2 |
| TNF α | (Forward) CCCTCACACTCAGATCATCTTCT | SEQ ID NO: 3 |
| | (Reverse) GCTACGACGTGGGCTACAG | SEQ ID NO: 4 |
| IL-6 | (Forward) TAGTCCTTCCTACCCCAATTTCC | SEQ ID NO: 5 |
| | (Reverse) TTGGTCCTTAGCCACTCCTTC | SEQ ID NO: 6 |
| IL-1β | (Forward) GCAACTGTTCCTGAACTCAACT | SEQ ID NO: 7 |
| | (Reverse) ATCTTTTGGGGTCCGTCAACT | SEQ ID NO: 8 |
| MCP-1 | (Forward) TTAAAAACCTGGATCGGAACC | SEQ ID NO: 9 |
| | (Reverse) GCATTAGCTTCAGATTTACGGGT | SEQ ID NO: 10 |
| iNOS | (Forward) CCCCGCTACTACTCCATCAG | SEQ ID NO: 11 |
| | (Reverse) CCACTGACACTTCGCACAAA | SEQ ID NO: 12 |
| | | |

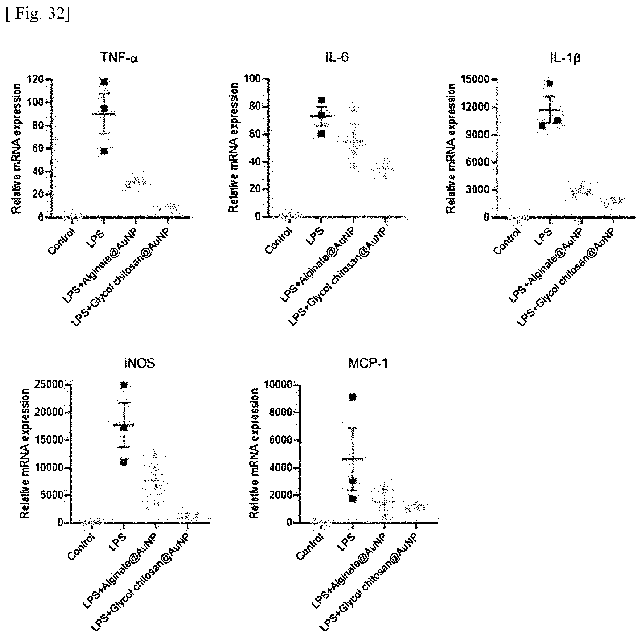

As a result, the above factors (TNF-α, IL-6, IL-1 beta, MCP-1, and iNOS) were all inflammatory factors and secreted in large amounts in the cells stimulated with LPS, but in the groups treated with Glycol chitosan@AuNP before stimulation with LPS, it was confirmed that the secretion of all was reduced (FIG. 32). In addition, the secretion of inflammatory factors was reduced more by Glycol chitosan@AuNP than by Alginate@AuNP, indicating a greater anti-inflammatory effect. Therefore, it was confirmed that the inflammatory response was alleviated mainly by scavenging ROS/RNS induced by LPS stimulation.

### 18. Confirmation of pro-inflammatory cytokine secretion in Raw 264.7 cells by ELISA

To confirm how much pro-inflammatory cytokine secretion in Raw 264.7 cells stimulated by LPS is inhibited by AuNP, ELISA was performed. The Raw 264.7 cells were seeded in a 12-well plate (1.0 x 10⁵ cells per well) and cultured in a complete medium (DMEM, 10% FBS, 1% PS) for 24 hours. Afterward, the cells were washed with PBS and incubated with AuNPs (125 µg/mL) for 5 hours. Before washing the cells in a medium with LPS (1 µg/mL) or without LPS for 20 hours, the cells were washed with PBS, and the cell supernatant was obtained and then stored at -20 °C before analysis. ELISA verification was performed in accordance with the protocol of an ELISA kit that had been purchased. The analyzed factors are TNF-alpha (EliKine^{™} Mouse TNF-α ELISA Kit, Abbkine), IL-6 (IL-6 Mouse ELISA Kit, Invitrogen), IL-1 beta (IL-1 beta Mouse ELISA Kit, Invitrogen), MCP-1 (MCP-1 Mouse ELISA Kit, Invitrogen), and iNOS (Mouse NOS2/iNOS(Nitric Oxide Synthase 2, Inducible).

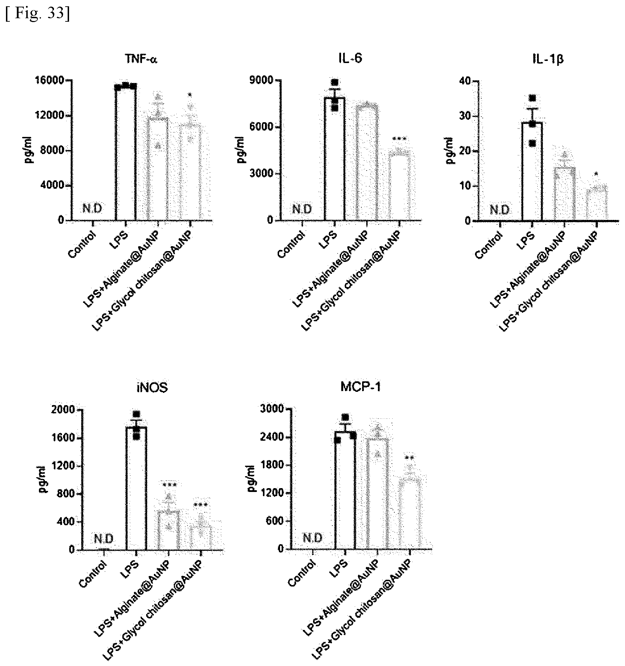

As a result, all the factors (TNF-α, IL-6, IL-1 beta, MCP-1, and iNOS) were inflammatory factors, and a large amount of them were secreted from the LPS-stimulated cells, but it was confirmed that, in the group treated with Glycol chitosan@AuNP before stimulation with LPS, the secretion was reduced (FIG. 33). In addition, it was thought that the anti-inflammatory effect was greater for Glycol chitosan@AuNP than for Alginate@AuNP, as the secretion of the inflammatory factors was reduced more. According to this, it was confirmed that an inflammatory response was alleviated mainly by eliminating ROS/RNS induced by LPS stimulation.

### 19. In vivo inductively coupled plasma-mass spectrometry (ICP-MS)

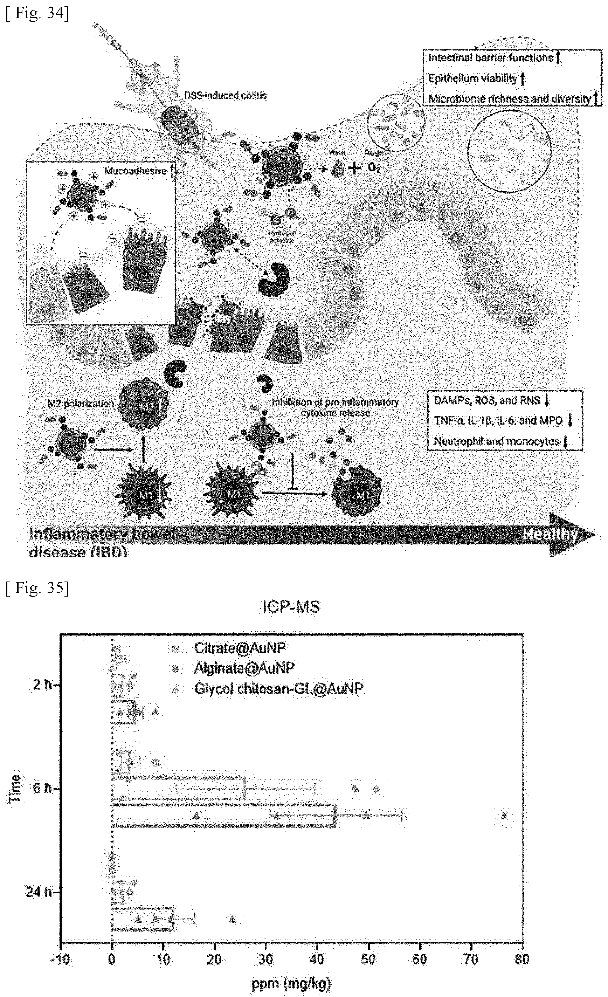

It was confirmed how much Glycol chitosan@AuNP and Alginate@AuNP were absorbed into colon tissue when they were orally administered to IBD mouse models induced by dextran sodium sulfate (DSS). The mechanism of oral administration of the drug to the mice with colitis induced by DSS is shown in FIG. 34. The types of groups are as follows: Citrate@AuNP (N=5), Glycol chitosan@AuNP (N=5), and Alginate@AuNP (N=5). Specifically, 7-week-old male C57BL/6 mice were housed (five per cage), and fed drinking water supplemented with DSS for one week. The mice were fed 60 mg/kg each of Citrate@AuNP, Glycol chitosan@AuNP and Alginate@AuNP on the last 7 days, and sacrificed after 2, 6, and 24 hours. After isolation, the colon tissue was fixed with 4% paraformaldehyde before ICP-MS.

As a result of confirming the residual level over time by ICP-MS when AuNPs were administered to the colon tissue, it was confirmed that the bioavailability of the drug was reduced in IBD patients due to drug destabilization caused by digestive fluid or drug clearance such as diarrhea (FIG. 35). Therefore, the degree of absorbance of Citrate@AuNP, Alginate@AuNP, and Glycol chitosan@AuNP was determined through ICP-MS, and it was confirmed that the absorbance of Glycol chitosan@AuNP was higher than those of the other particles. This is because, due to its positive charge, the particle interacts with a mucus layer having a negative charge. Due to this interaction, it was confirmed that the drug was retained for approximately 24 hours, and the retention time was approximately 3.4 times higher and the clearance was reduced by 2.7 times compared to alginate.

### 20. In vivo bio-transmission electron microscopy (BIO-TEM)

It was qualitatively confirmed how much Glycol chitosan@AuNP and Alginate@AuNP were adsorbed to colon tissue when being orally administered to IBD mouse models induced by DSS. The experimental materials for the experiment were Karnovsky's fixation (primary fixation), 0.05 M sodium cacodylate buffer (for washing), 2% osmium tetroxide (1 mL) for post fixation, 0.1 M cacodylate buffer (1 mL) and 0.5% uranyl acetate for staining, ethanol (30, 50, 70, 80, and 90%) for water removal, propylene oxide for transition, propylene oxide (1 mL) for infiltration, and Spurr's resin (1 mL). Soren's phosphate buffer consists of solution A and solution B (A: 0.2.M Na₂HPO₄2H₂O, B: 0.2.M NaH₂PO₄H₂O) and was treated for 10 minutes for a fixative rinse. Additional treatment with 1% osmium tetroxide (OsO₄) was performed to stain a tissue membrane. The resulting tissue membrane was washed for 10 minutes to remove OsO₄ remaining in Soren's phosphate buffer. The dehydration process was carried out using different concentrations of ethanol (reacted at 30%, 50%, 70%, and 90% for 10 minutes and then reacted at 100% for 20 minutes three times). The Spurr's kit method, which enables the formation of epoxy resin blocks with embedded media having low viscosity, was applied to all samples. Epoxy resin specimens were cut into 80 nm-thick sections using an ultramicrotome (EM UC7). The sections were air-dried for at least 1 hour. Uranyl acetate (2%) was mounted on copper grids for 20 minutes. After washing with DW, lead citrate (0.4%) was mounted and stained for 10 minutes. The sections were placed on the grids, and observed using a 80 kV transmission electron microscope.

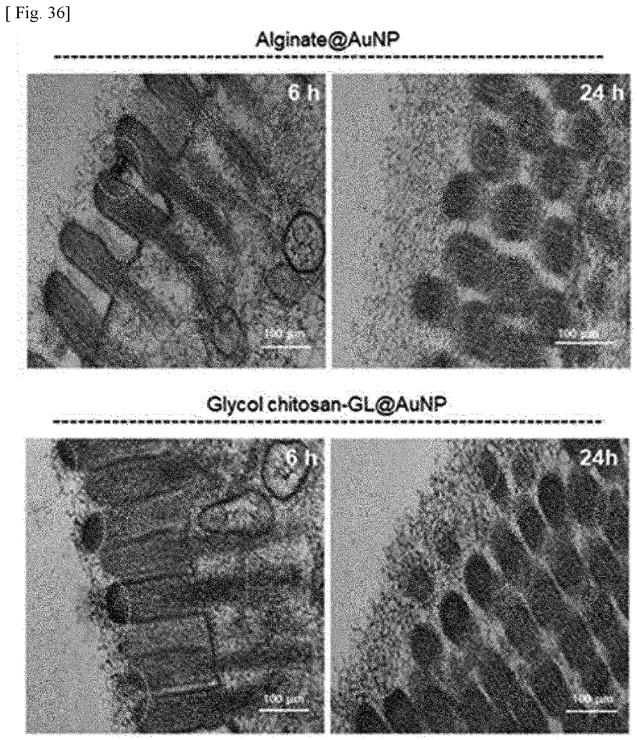

As a result, it was confirmed that, from 6 hours to 24 hours, Alginate@AuNP and Glycol chitosan@AuNP remained in the mucus layer (FIG. 36).

### 21. Synthesis of Glycol chitosan and Glycyrrhizin

This experiment was conducted to additionally introduce glycyrrhizin (GL), which can regulate HMGB1, to glycol chitosan in AuNPs. GL (2 mM and 10 mL) was dissolved in distilled water, and the pH was adjusted using 1 N NaOH until GL was completely dissolved in the solution. Afterward, NaIO₄ (2 mM and 10 mL) was dissolved in distilled water, and darkness was maintained by foil wrapping. A SP solution was slowly dropped to form oxidized GL (oGL). The GL solution was stirred at room temperature for 30 minutes in the dark and for 30 minutes in the light. Glycol chitosan (64 mg) was dissolved in 20 mL of DW, and the pH was adjusted using 1.0 M HCl until glycol chitosan was completely dissolved in the solution. Afterward, the same volume of oGL was slowly added dropwise to the GC solution. The solution was reacted at 4 °C for 2 hours, and sodium cyanoborohydride was added in an amount equivalent to 1/1,000 of the total solution and reacted overnight at 4 °C. The resulting product was dialyzed using a MWCO 3.5 kDa dialysis bag to discard unreacted substances. The GC-GL conjugate was obtained by freeze-drying for 2 days, and the yield was confirmed by measuring a weight. Afterward, to measure H-NMR, the resulting product was dissolved in D₂O. To perform H-NMR, nuclear magnetic resonance (NMR; VNMRS 600MHz, Agilent Technologies) was used, and to perform FT-IR, Fourier transform infrared (FT-IR, NICOLET IS50, Thermo Fisher Scientific) was used.

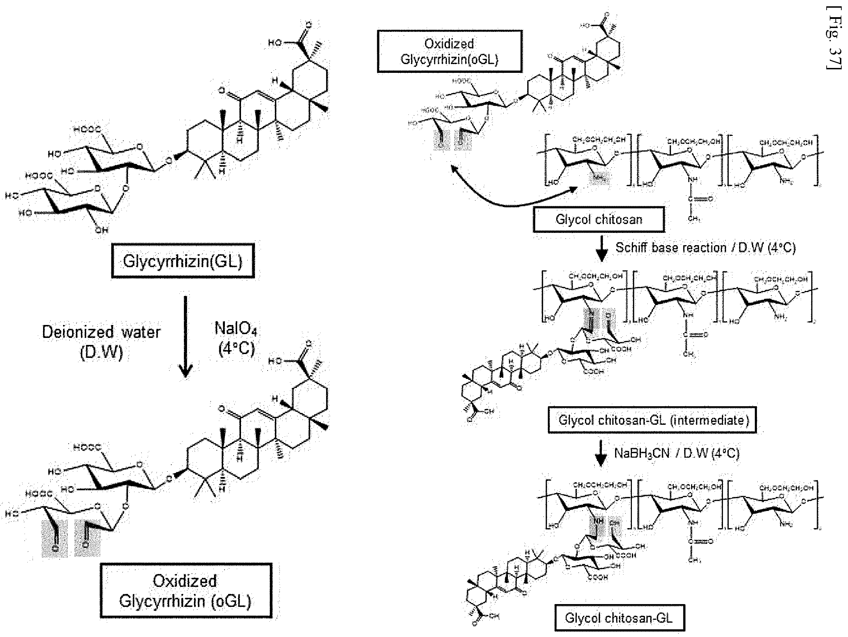

The scheme of conjugation between glycyrrhizin and glycol chitosan is shown in FIG. 37. Specifically, glycyrrhizin dissolved in DW was reacted with NAIO₄ to produce an aldehyde group at the glucuronic acid moiety of glycyrrhizin. When the oxidized glycyrrhizin was slowly added dropwise to glycol chitosan dissolved in DW, an imine group was formed by a Shiff base reaction between the aldehyde group and an amine group, resulting in a Glycol chitosan-GL intermediate. Accordingly, to form a stable secondary amine group, sodium cyanoborohydride was added.

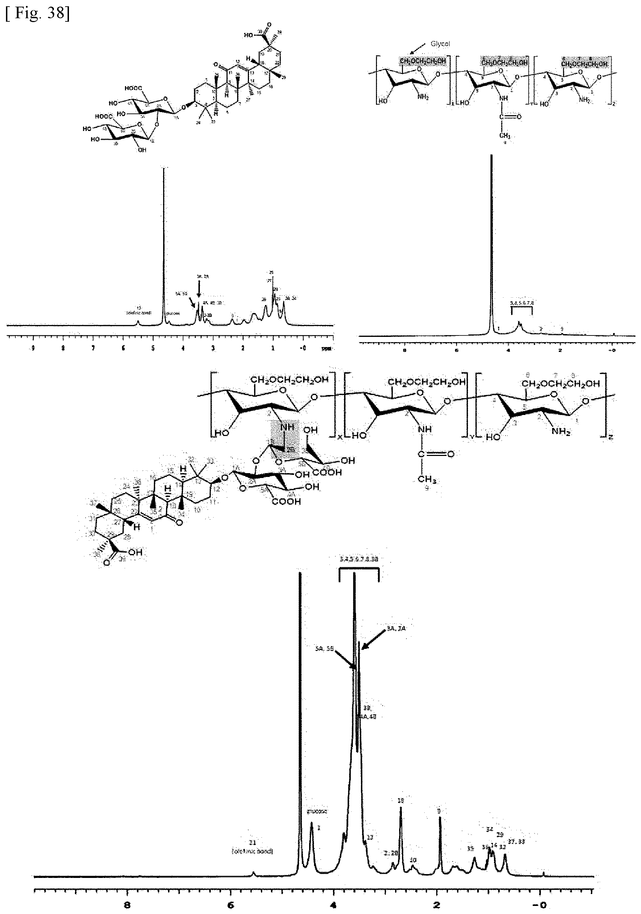

Afterward, as a result of confirming the synthesis result of glycol chitosan-glycyrrhizin through H-NMR, when a secondary amine group was finally formed by the reaction between the oxidized glycyrrhizin and an amine group of glycol chitosan, it was confirmed that a peak appeared at approximately 2.8 ppm (FIG. 38).

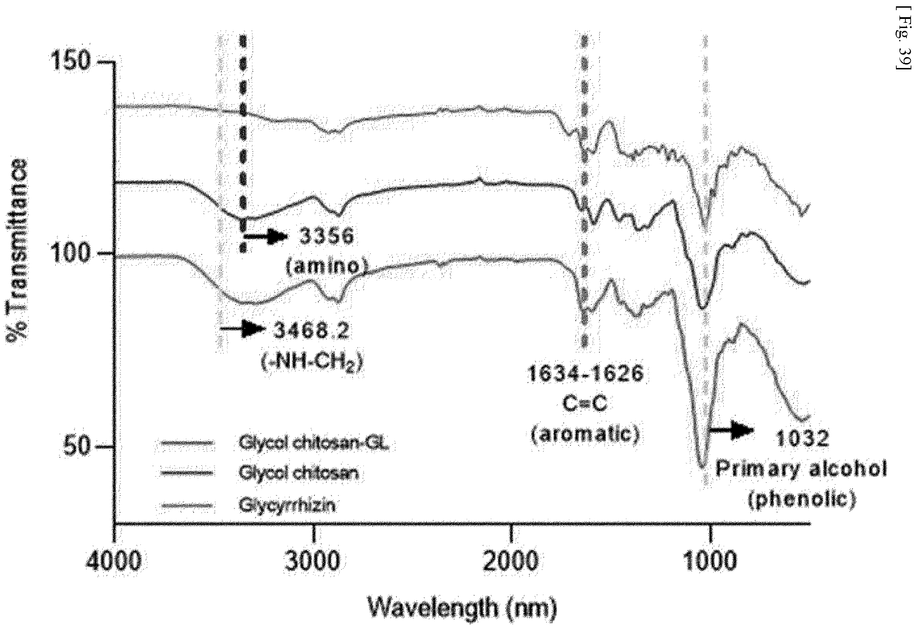

In addition, the synthesis result of glycol chitosan-glycyrrhizin was confirmed by FT-IR. The peak of the aromatic C=C bond of glycyrrhizin and the phenolic primary alcohol peak of glycol chitosan are characteristic peaks of these substances. The peak of the amine group of glycol chitosan appears at 3356 cm⁻¹, and the peak of the secondary amine group of the conjugation form appears at 3468.2 cm⁻¹. By detecting broad peaks near these areas, the conjugation between glycol chitosan and glycyrrhizin was confirmed (FIG. 39).

### 22. Synthesis of AuNPs coated with glycol chitosan-glycyrrhizin

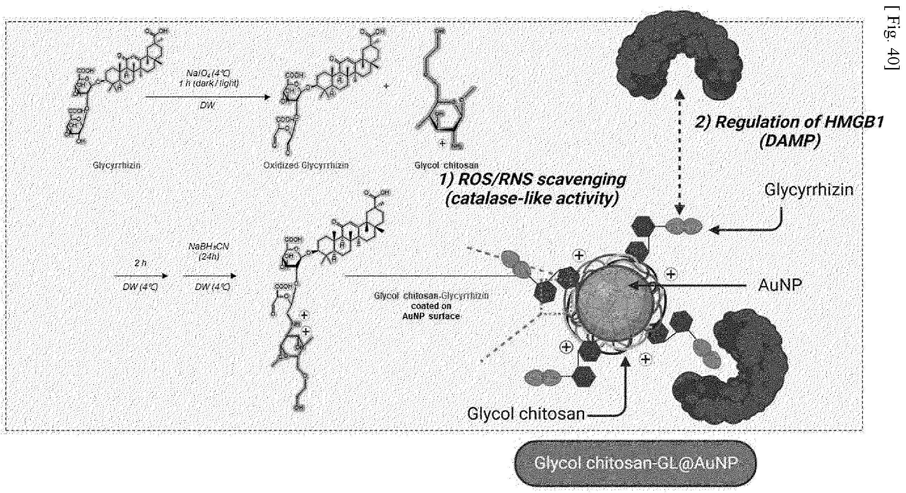

This experiment was conducted to confirm the synthesis of AuNPs coated with glycol chitosan-glycyrrhizin (Glycol chitosan-GL@AuNP). Here, the synthesis scheme of glycol chitosan and glycyrrhizin and the scheme for coating AuNPs with them are shown in FIG. 40. After adding the solution of Example 17 to distilled water, the pH was adjusted to 8 using 1 N NaOH, and heated at 100 °C on a hot plate until GC-GL was completely dissolved in the solution. A gold solution (2 mM) was slowly added dropwise to the GC-GL solution. The yellow solution turned colorless and then wine-colored within 30 minutes, indicating the formation of GC-GL@AuNP. After centrifugation of the AuNP solution at 16,000 rpm for 30 minutes, the supernatant containing excess reactants was discarded, and the AuNP pellet was redispersed in DW and assessed using UV/vis (Nanodrop 2000).

### 23. Confirmation of colloidal stability of AuNPs coated with glycol chitosan-glycyrrhizin according to pH

To confirm whether Citrate@AuNP (1:10), Alginate@AuNP (0.2%), Glycol chitosan@AuNP (0.32%), and Glycol chitosan-GL@AuNP are stably maintained without changing their charges in a biomimetic environment (GI tract biomimicking environment), dynamic light scattering (DLS) values were measured. Specifically, the pH of the AuNPs was adjusted to pH 2 and pH 6 with HCl and NaOH, respectively. After 12 hours, it was confirmed how stable the zeta potentials of the AuNPs are maintained.

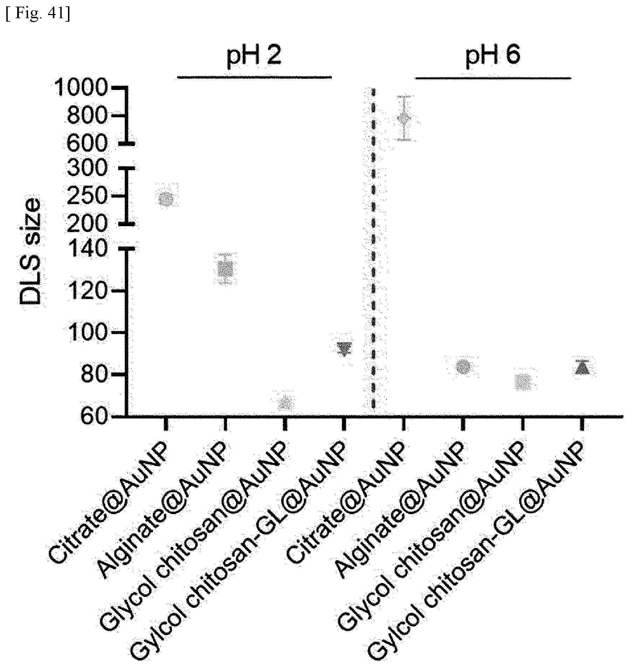

As a result, the value of Citrate@AuNP (1:10) was 240 nm, which is approximately 8 times higher than 30 nm, and the value of Alginate@AuNP 0.2% was 120 nm, which is approximately 2 times higher than 56 nm, whereas the value of Glycol chitosan@AuNP 0.32% was 60 nm, which is not significantly different from the existing 80 nm (FIG. 41).

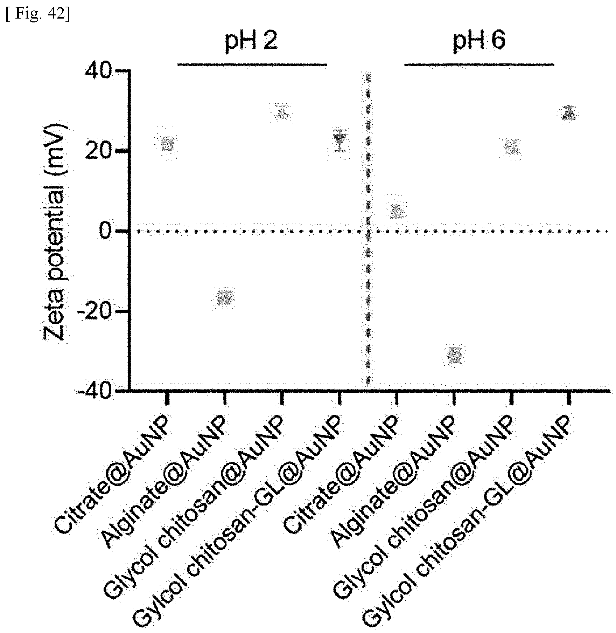

In addition, to confirm how stable the zeta potentials of Citrate@AuNP (1:10), Alginate@AuNP 0.2%, Glycol chitosan@AuNP 0.32%, and Glycol chitosan-GL@AuNP are maintained when Citrate@AuNP (1:10), Alginate@AuNP 0.2%, Glycol chitosan@AuNP 0.32%, and Glycol chitosan-GL@AuNP were placed in the pH 2 and pH 6 environments for 12 hours, the zeta potentials (mV) were measured. The results are shown in FIG. 42. Specifically, Citrate@AuNP (1:10) has a preexisting negative charge, but when placed at pH 2 for 12 hours, it was confirmed that the zeta potential value changed to positive. In addition, it was confirmed that Alginate@AuNP 0.2% previously has a zeta potential value of approximately -34 mV, but when placed at pH 2 for up to 12 hours, it has a zeta potential of approximately -20 mV. This showed that the carboxylic acids of citrate and alginate are protonated, resulting in decreased zeta potential values. However, it was confirmed that the zeta potential of Glycol chitosan@AuNP 0.32% was maintained at both pH 2 and pH 6. Finally, Glycol chitosan-GL@AuNP also showed no significant change in zeta potential, so it was expected that Glycol chitosan@AuNP and Glycol chiotsan-GL@AuNP would stably maintain their properties *in vivo.*

Furthermore, as a result of measuring PDI values to confirm a particle size in an aqueous solution and to confirm whether the particles are stably and uniformly dispersed when AuNPs were placed at pH 2 and pH 6 for 12 hours, it was confirmed that all the PDI values were 0.5 or less, indicating that the particles were dispersed in a stable and uniform manner (FIG. 7).

### 24. Co-culture of Raw 264.7 cells and Caco-2 cells

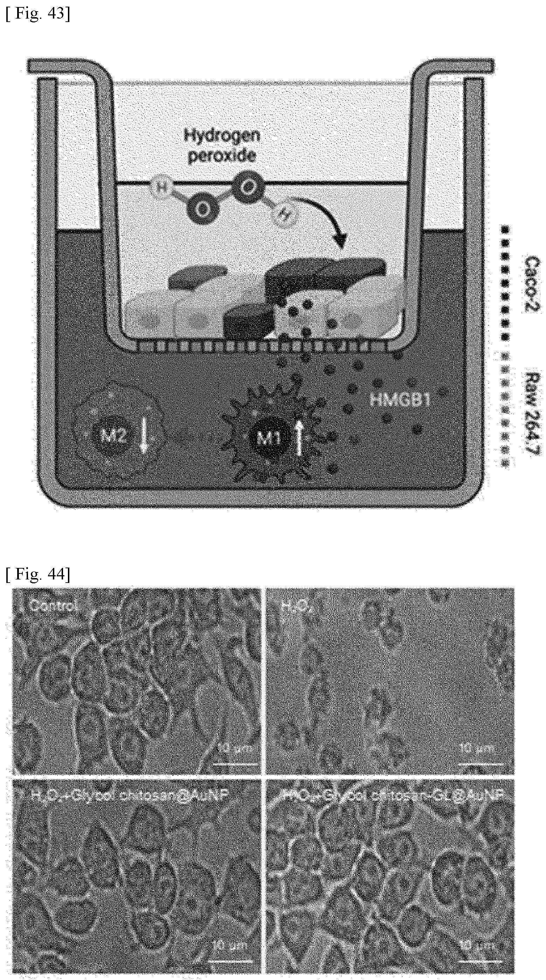

After Raw 264.7 cells and Caco-2 cells were co-cultured and the Caco-2 cells were stimulated with hydrogen peroxide, the effect of the HMGB1 secreted thereby on Raw 264.7 cells was confirmed, and the effect of co-treatment of AuNP and hydrogen peroxide was also confirmed. Here, a co-culture system of the Raw 264.7 cells and the Caco-2 cells is shown in FIG. 43. To confirm the secretion of proinflammatory cytokines, Caco-2 cells were seeded in a Transwell insert chamber (apical side) with a diameter of 6.6 mm and a pore size of 0.4 µm (density: 2.5 x 10⁴ cells/well). Raw 264.7 cells were seeded in a basolateral chamber (density: 5.0 x 10⁴ cells/well). After 24 hours, an experiment was conducted with four groups (Group 1: negative control, Group 2: positive control; H₂O₂ (100 µM), Group 3: co-treatment with H₂O₂ (100 µM) and Glycol chitosan@AuNP (125 µg/mL), and Group 4: co-treatment with H₂O₂ (100 µM) and Glycol chitosan-GL@AuNP (125 µg/mL) in apical chamber, and culture of cells for 20 hours).

In the co-culture system of Raw 264.7 cells and Caco-2 cells, the morphology of the Raw 264.7 cells was confirmed. As a result, in the co-culture system, it was confirmed that the Raw 264.7 cells of the H₂O₂-treated group were killed or activated by the secreted HMGB1. In the Glycol chitosan@AuNP- or Glycol chitosan-GL@AuNP-treated group, it was confirmed that there was almost no activated form of Raw 264.7 cells due to the action of AuNPs (FIG. 44).

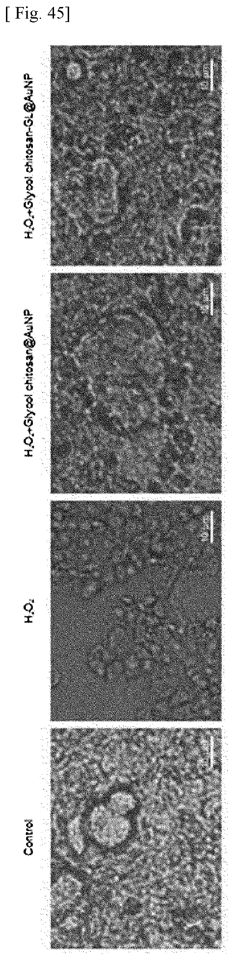

In addition, in the co-culture system of Raw 264.7 cells and Caco-2 cells, the morphology of the Caco-2 cells was confirmed. As a result, it was confirmed that the monolayer was destroyed due to stimulation in the H₂O₂-treated group, but it was maintained as is when treated with AuNPs (FIG. 45).

### 25. ELISA verification of HMGB1 and inflammatory factors (TNF-alpha, IL-6, IL-1 beta, MCP-1, and iNOS) in co-culture system

Analysis was performed on a cell supernatant in a basolateral chamber. ELISA verification was performed in accordance with the protocol of an ELISA kit that had been purchased. The analyzed factors are HMGB1 (EliKine^{™} Mouse HMGB1 ELISA Kit, Abbkine), TNF-alpha (EliKine^{™} Mouse TNF-α ELISA Kit, Abbkine), IL-6 (IL-6 Mouse ELISA Kit, Invitrogen), IL-1 beta (IL-1 beta Mouse ELISA Kit, Invitrogen), MCP-1 (MCP-1 Mouse ELISA Kit, Invitrogen), and iNOS Mouse NOS2/iNOS (Nitric Oxide Synthase 2, Inducible).

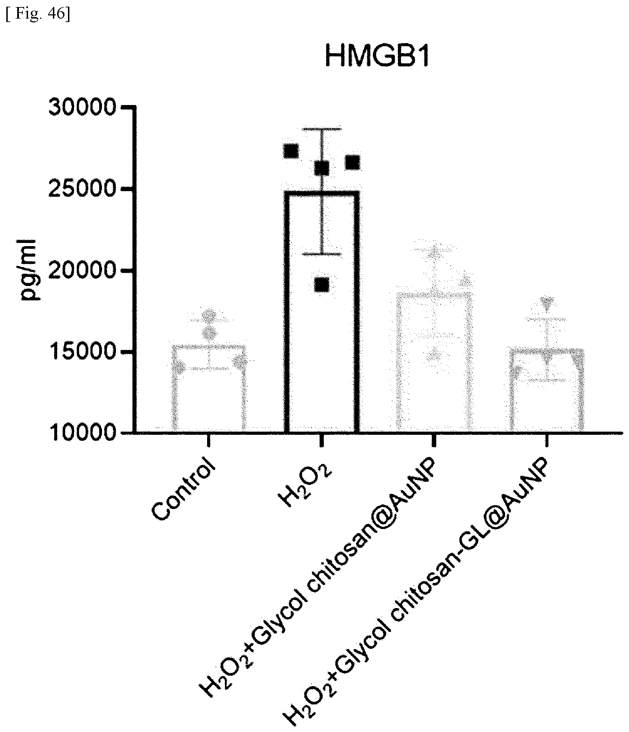

In the co-culture system of Raw 264.7 cells and Caco-2 cells, as a result of ELISA verification of the amount of HMGB1 secretion, it was confirmed that, when Caco-2 cells were stimulated with H₂O₂ (100 µM), HMGB1 was secreted the most, whereas when Glycol chitosan@AuNP and H₂O₂ were co-treated, the concentration of secreted HMGB1 due to Caco-2 disruption decreased (FIG. 46). In addition, when GL was additionally introduced, it was confirmed that a lower concentration of HMGB1 was secreted than Glycol chitosan@AuNP. Taken together, it can be seen that Glycol chitosan-GL@AuNP suppresses HMGB1 secretion more than Glycol chitosan@AuNP.

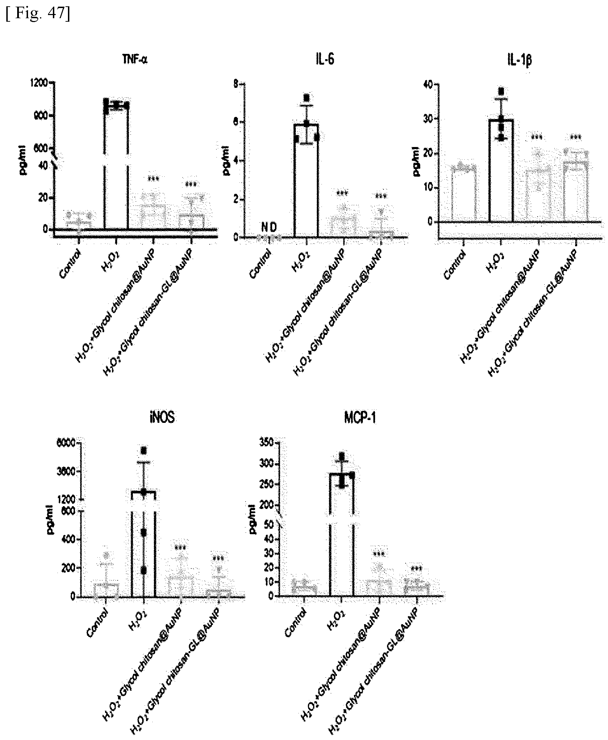

In addition, as a result of ELISA verification of inflammatory factors secreted from Raw 264.7 cells in the co-culture system of Raw 264.7 cells and Caco-2 cells, it was confirmed that GL, which is a direct antagonist of HMGB1, inhibited LPS-induced lethality, thereby attenuating inflammation caused by M1 polarization (FIG. 47). It was confirmed that the secretion of pro-inflammatory cytokines from Raw 264.7 cells activated by HMGB1 was hardly detected in Glycol chitosan-GL.

### 26. FACS verification of M1 and M2 polarizations of Raw 264.7 cells in co-culture system

Flow cytometry was performed to confirm M1 and M2 polarization ratios when Raw 264.7 cells were treated with AuNPs (Glycol chitosan@AuNP and Glycol chitosan-GL@AuNP). For flow cytometry, the cells were separated using a scraper, and centrifuged at 12,000 rpm for 3 minutes. After removing the supernatant, the cells were redispersed with PBS, and incubated with or without antibodies at room temperature for 20 minutes. The antibodies used were CD206 APC-eFluor 780, and CD80-FITC. There were a total of 7 groups: Negative control (staining), positive control (non-staining), Raw cells stained with CD 206 marker (staining), Raw cells stained with CD 80 marker (staining), Raw cells stained with CD 206 marker and CD 80 marker (co-staining), Raw cells, incubated with Glycol chitosan@AuNP, and stained with CD 206 and CD 80 (co-staining), Raw cells, incubated with Glycol chitosan-GL@AuNP, and stained with CD 206 and CD 80 (co-staining). The samples were analyzed by BD FACS(BD FACSCalibur, BD Bioscience, New Jersey, USA), and data was analyzed using FlowJo V10. The unstained positive control was used for gating.

The morphology of Raw 264.7 cells in the co-culture system of Raw 264.7 cells and Caco-2 cells was identified. As a result, it was confirmed that, in the co-culture system, in the H₂O₂ -treated group, Raw 264.7 cells were killed or activated by secreted HMGB1. In the Glycol chitosan@AuNP- or Glycol chitosan-GL@AuNP-treated group, there was almost no activated form of Raw 264.7 cells due to the action of AuNPs (FIG. 44).

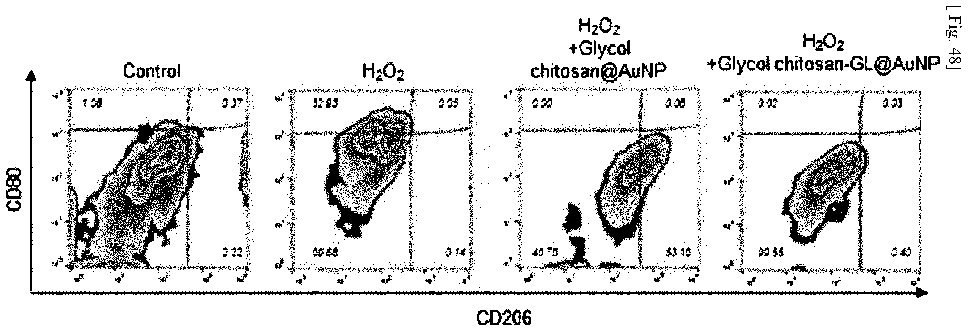

As a result of FACS verification of the M1 and M2 polarizations of Raw 264.7 cells in the co-culture system of Raw 264.7 cells and Caco-2 cells, it was confirmed that both of Glycol chitosan@AuNP and Glycol chitosan-GL@AuNP reduced M1 polarization from 32.93% to approximately 0% after H₂O₂ damage (FIG. 48). In addition, it was confirmed that Glycol chitosan@AuNP showed an M2 polarization at 53.16%, and Glycol chitosan-GL@AuNP showed almost no M2 polarization at only 0.4%.

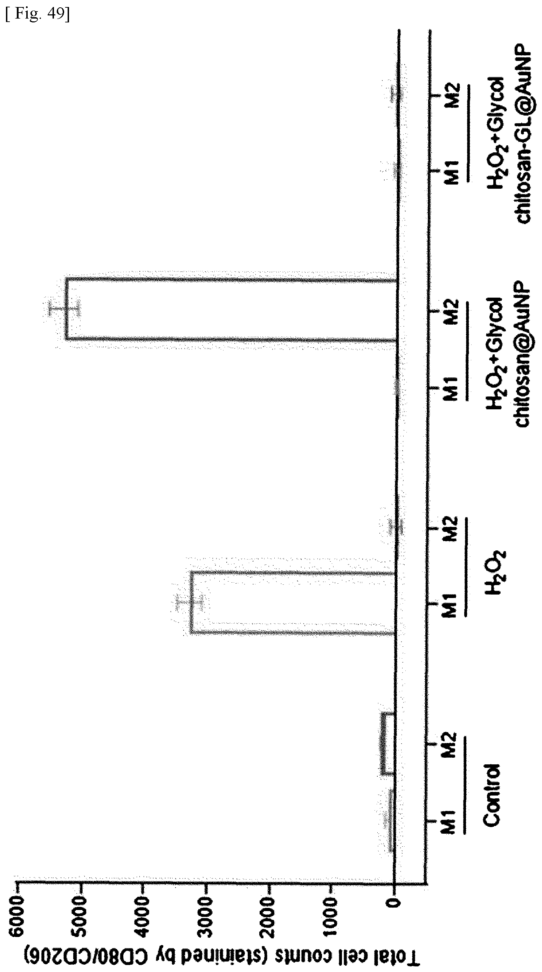

As a result of analyzing populations for the M1 and M2 polarizations of Raw 264.7 cells in the co-culture system of Raw 264.7 cells and Caco-2 cells, Glycol chitosan@AuNP showed a higher M2 polarization ratio, but Glycol chitosan-GL@AuNP showed almost no change to M1 and M2 polarizations, suggesting that the cells are maintained in a monocytic morphology (FIG. 49). That is, it can be assumed that Glycol chitosan-GL@AuNP hardly stimulate Raw 264.7 cells.

### 27. Preparation of animal experiment and measurement parameters

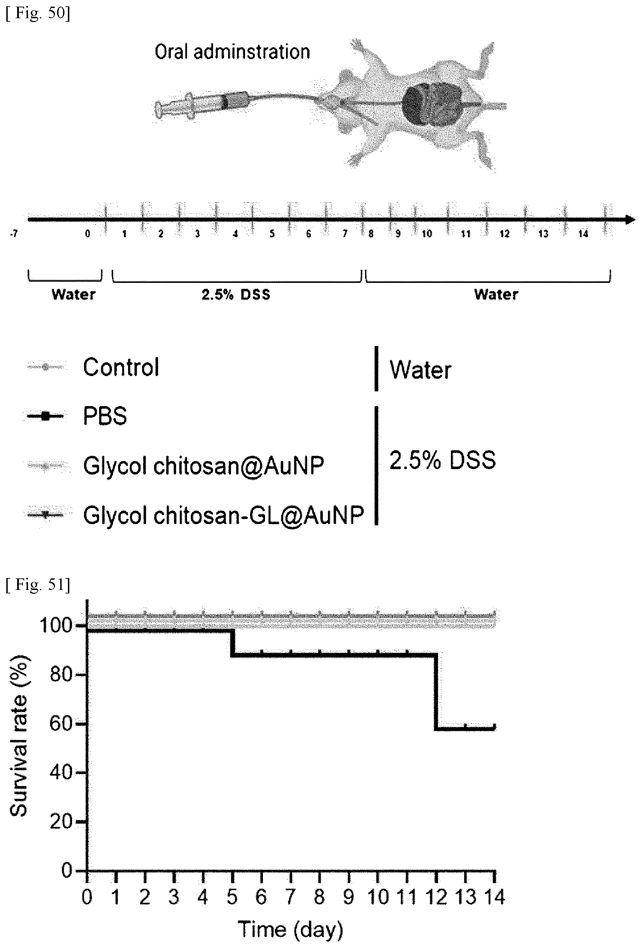

Seven-week-old male C57BL/6 mice were housed (five mice per cage), and acclimated for one week. A specific scheme is shown in FIG. 50. Healthy mice of a negative control (Negative control) were fed only normal water, and mice of a positive control (Positive control) and experimental groups (Glycol chitosan@AuNP and Glycol chitosan-GL@AuNP) were fed drinking water containing 2.5%(w/v) dextran sodium sulfate (DSS) for 7 days and then fed normal drinking water for 7 days. 200 µL of 30 mg/kg Glycol chitosan@AuNP (N=5), 200 µL of Glycol chitosan-GL@AuNP (N=5), and 200 µL of PBS (N=5) were orally administered to the DSS-induced groups for 2 weeks, and on the last day of the experiment (Day 14), the mice were euthanized, and the colon and organs (lungs, heart, kidneys, liver, and spleen) were removed. The extent of damage was determined by measuring the weights and lengths of the spleen and colon, and photographs of the anus.

The results of measuring the survival rate of the mice is shown in FIG. 51, and it was confirmed that, in the DSS-induced group, the survival rates were 80% and 60% on day 5 and day 12, respectively. On the other hand, it was confirmed that, in the Glycol chitosan@AuNP- and GC-GL@AuNP-administered groups, the survival rates were maintained at 100%.

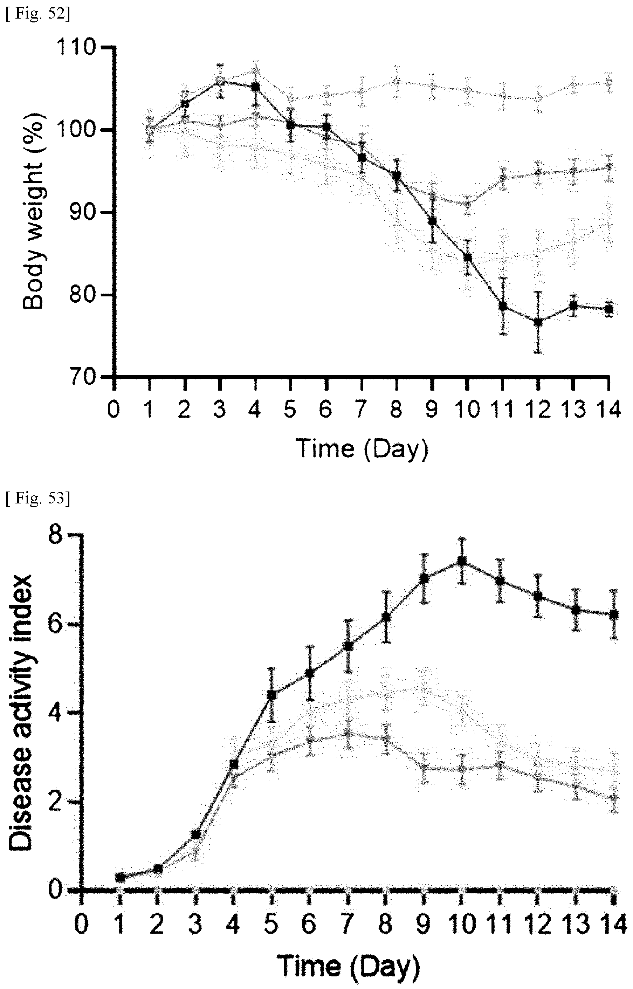

The result of measuring the body weights of the mice are shown in FIG. 52, and it was confirmed that, in the Glycol chitosan@AuNP- and Glycol chitosan-GL@AuNP-administered groups, the body weights of all mice decreased and then recovered from day 10, and the degree of body weight recovery of the mice in the Glycol chitosan-GL@AuNP-administered group was excellent.

The results of measuring the disease activity indices (DAIs) of the mice are shown in FIG. 53, and it was confirmed that the Glycol chitosan@AuNP- and Glycol chitosan-GL@AuNP-administered groups had low DAI values until day 14 when all experiments were completed. The DAI values were as follows: stool consistency (hard : 0 points, soft : 2 points, and diarrhea : 4 points), the extent of bleeding in stool using Hemoccult Sensa (Beckman Coulter) (no bleeding : 0 points, bleeding : 2 points, and macroscopically visible : 4 points), and body weight loss (less than 1% : 0 point, 1 to 5% : 1 point, 5 to 10% : 2 points, 10 to 20% : 3 points, and 20% or more: 4 points).

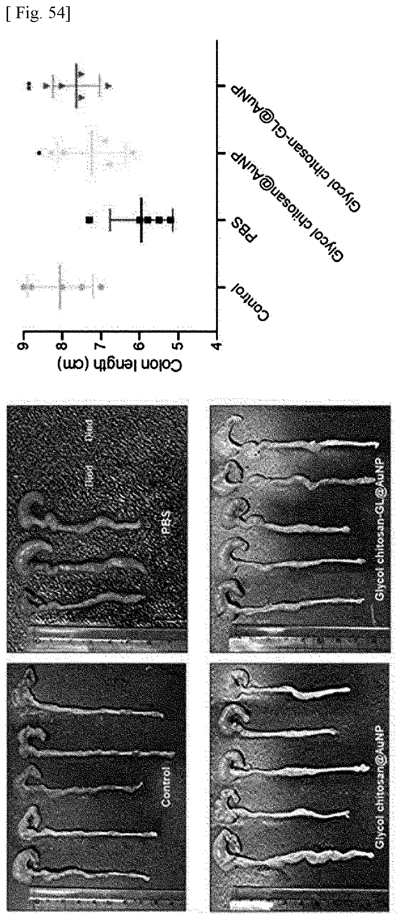

The results of measuring the colon lengths of the mice are shown in FIG. 54, and it was confirmed that, on day 14, the length of the colon tissue of a mouse fed DSS to induce IBD was approximately 5 cm shorter than that of a control, but the length of the colon tissue of a mouse fed AuNPs recovered to a length similar to that of the control.

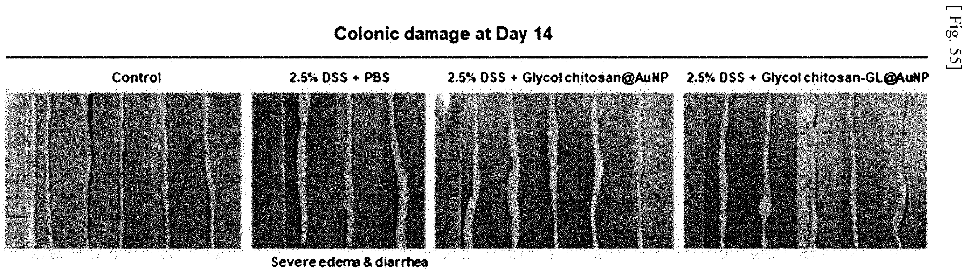

The results of confirming damage to the mouse colons are shown in FIG. 55, and it was confirmed that the group co-treated with 2.5% DSS and PBS showed a very poor colon condition due to edema and diarrhea, whereas the groups treated with AuNPs showed very weak colons or colons recovered to the normal condition.

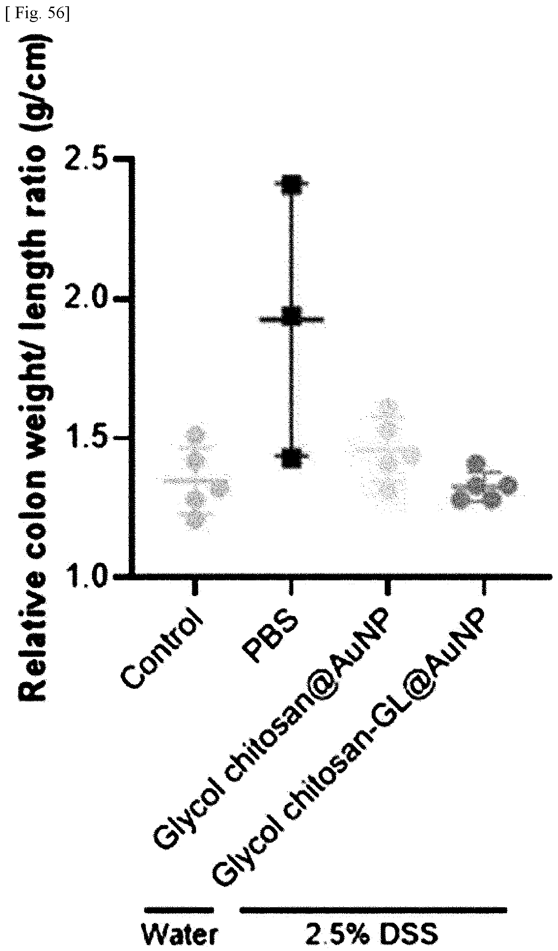

The results of measuring the colon weights and lengths of the mice are shown in FIG. 56, and it was confirmed that there was almost no toxicity to the entire body because both types of AuNPs showed similar values to the normal condition.

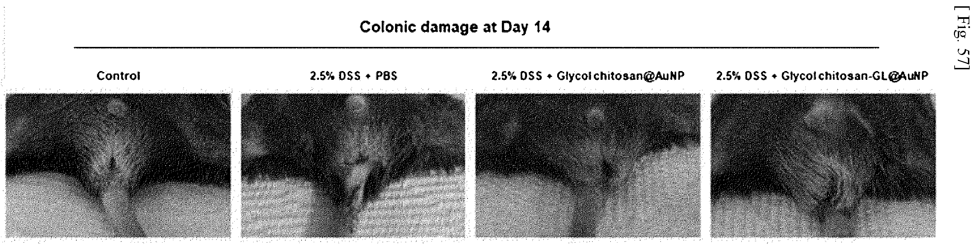

The photographs of the mouse anus are shown in FIG. 57, and it was confirmed that the group co-treated with 2.5% DSS and PBS showed poor anal conditions due to bloody stool, but the groups treated with AuNPs recovered.

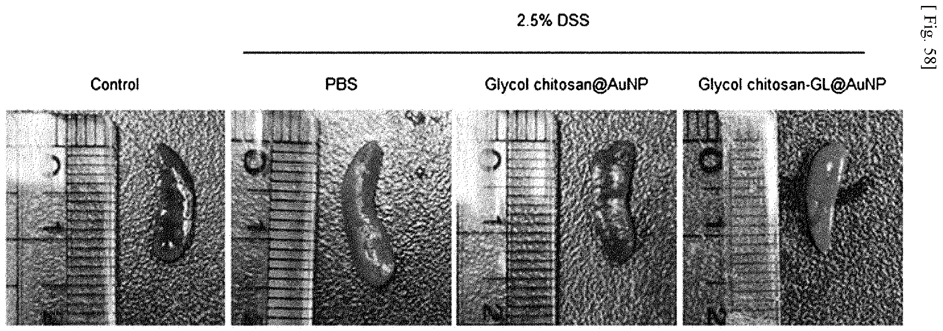

The results of confirming the hypertrophy of the mouse spleens are shown in FIG. 58, and it was confirmed that the group co-treated with 2.5% DSS and PBS showed an enlarged spleen with a length of approximately 1.5 cm, whereas the groups treated with AuNPs showed shorter spleen lengths, which are similar to the normal condition.

### 28. Confirmation of tight junctions by immunohistochemistry (IHC)

This experiment was conducted to determine the extent of tight junction recovery (ZO-1 and occludin) when Glycol chitosan@AuNP and Glycol chitosan-GL@AuNP were co-treated. Tissue processed on a slide was fixed in 4% paraformaldehyde. Caco-2 cells were washed twice with PBS-Tween 20 for approximately 2 minutes, and for blocking, the cells were incubated in 20% goat serum-containing PBS-Tween 20 as a solvent at room temperature for 30 minutes. The goat serum (20% in PBST-20) and primary antibodies (anti-mouse occludin, anti-rabbit ZO-1) that were diluted 100: 1 were incubated at room temperature for 1 hour, and washed with PBS. In addition, secondary antibodies (goat anti mouse-488, goat anti rabbit-594) were diluted in PBS in a ratio of 1:200 and incubated with the cells. The cells were blocked from light and washed twice with PBS for 3 minutes. After adding one or two drops of DAPI mounting solution (blue, nuclear staining), the slide was covered with a cover slide and observed under a fluorescence microscope.

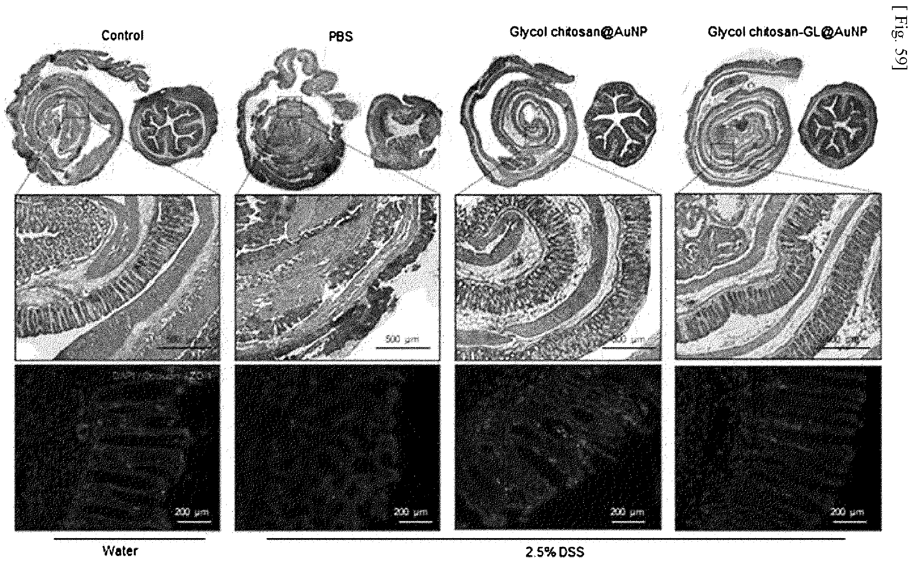

The results are shown in FIG. 59. Specifically, the sample treated with only PBS after DSS induction showed the destruction of a tight junction protein such as occludin (green) or Zo-1 (red), resulting in the destruction of the monolayer of intestinal epithelial cells. When AuNPs were co-treated, yellow (red, green merged) fluorescence was shown, indicating tight junction recovery.

### 29. ELISA verification of HMGB1 and anti-inflammatory factors in mouse serum

After cardiac blood collection from mice under general anesthesia, the blood was centrifuged at 3,000 rpm for 30 minutes at 4 °C in a tube coated with an anticoagulant. ELISA verification was performed in accordance with the protocol of an ELISA kit that had been purchased. Specifically, an experiment for the identification of anti-inflammatory factors in serum was performed on day 14 to confirm how Glycol chitosan-GL@AuNP scavenging ROS/RNS and HMGB1 exhibits an anti-inflammatory effect in DSS-induced mouse models.

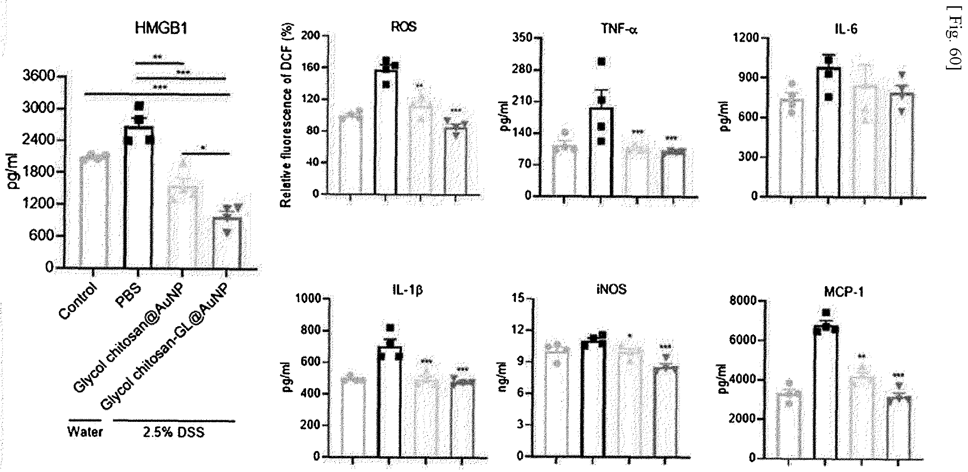

As a result, it was confirmed that Glycol chitosan-GL@AuNP treatment showed a significant decrease in HMGB1, as compared with other treatment groups (PBS and Glycol chitosan@AuNP treatment). It was confirmed that HMGB1 was actively or passively released from necrotic tissue or stressed cells, and all M1 polarization markers such as TNF-α, IL-6, IL-1β, iNOS, and MCP-1 were increased by DSS treatment, but decreased by AuNP treatment (FIG. 60).

### 30. ELISA verification of HMGB1 and anti-inflammatory factors in mouse colons

The middle portion of colon tissue was cut, the cut tissue was put into an EP tube and frozen in liquid nitrogen. 300 µL of RIPA buffer (Pierce^{™}) was added per 5 mg of the tissue, homogenized using a homogenizer (FastPrep-24 5G, MP Biomedicals, LLC, USA), and centrifuged at 4 °C and at 14,800 rpm for 20 minutes. Afterward, the supernatant was collected and stored at -20 °C until analysis. HMGB1 (EliKine^{™} Mouse HMGB1 ELISA Kit, Abbkine), TNF-alpha (EliKine^{™} Mouse TNF-α ELISA Kit, Abbkine), IL-6 (IL-6 Mouse ELISA Kit, Invitrogen), IL-1 beta (IL-1 beta Mouse ELISA Kit, Invitrogen), MCP-1 (MCP-1 Mouse ELISA Kit, Invitrogen), and iNOS Mouse NOS2/iNOS (Nitric Oxide Synthase 2, Inducible) were then analyzed using an ELISA kit. ELISA verification was performed in accordance with the protocol of an ELISA kit that had been purchased. Specifically, an experiment for identifying anti-inflammatory factors in the colons was performed on day 14 to confirm how Glycol chitosan-GL@AuNP scavenging ROS/RNS and HMGB1 exhibits an anti-inflammatory effect in DSS-induced mouse models.

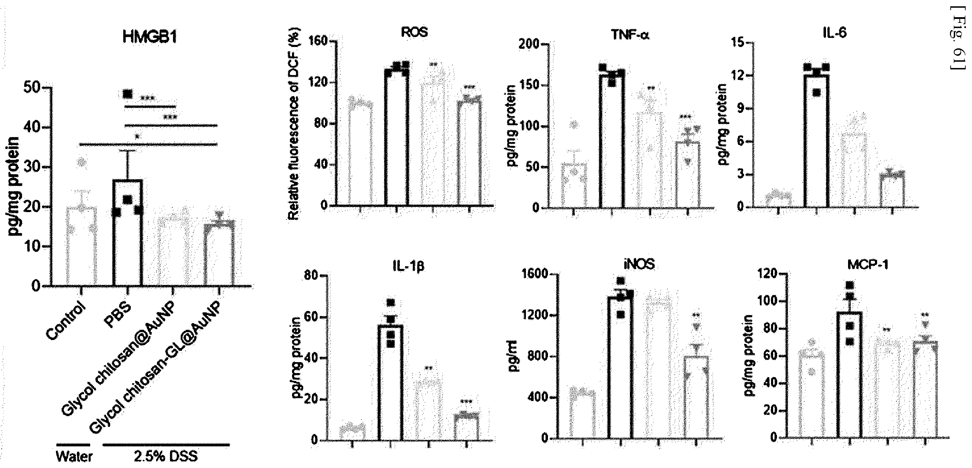

As a result, it was confirmed that Glycol chitosan-GL@AuNP treatment showed a significant decrease in HMGB1, as compared with other treatment groups (PBS and Glycol chitosan@AuNP treatment). It was confirmed that HMGB1 was actively or passively released from necrotic tissue or stressed cells, and all inflammatory factors such as TNF-α, IL-6, IL-1β, iNOS, and MCP-1 were increased by DSS treatment, but decreased by AuNP treatment (FIG. 61).

### 31. PCR analysis of HMGB1 and anti-inflammatory factors in mouse colons

The end of colon tissue was cut, and 600 µL of RLT buffer (RNeasy Mini Kit, QIAGEN, Germany) was added per 30 mg of the cut tissue, and 10 µL of 2-mercaptoethanol (Sigma) was added per 1 mL of the RLT buffer. After homogenization was performed using a homogenizer (FastPrep-24 5G, MP Biomedicals, LLC, USA), the supernatant was collected by centrifugation at 4 °C and 14,800 rpm for 3 minutes. After adding the same volume of 70% ethanol, the resulting solution was pipetted. Then, mRNA was extracted from the tissue using a RNeasy Mini Kit (QIAGEN, Germany). 1 µg of RNA was reverse-transcribed to cDNA using an iScript cNDA synthesis kit (BIO RAD Laboratories, USA), and qRT-PCR was performed in an Applied Biosystems instrument (USA) using SYBR Green PCR Master Mix (Applied Biosystems^{™}, USA). The relative mRNA expression of the genes shown in Table 1 was normalized using GAPDH as an endogenous control.

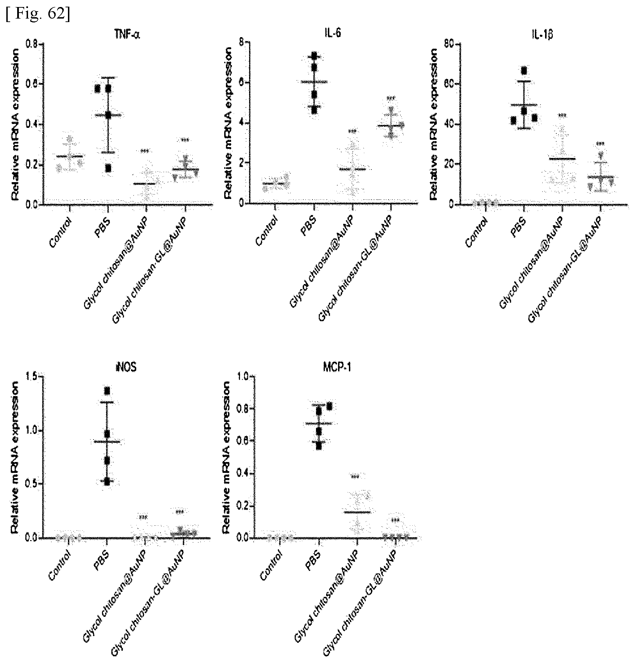

As a result of qRT-PCR, it was confirmed that the mRNA levels of related pro-inflammatory cytokines were significantly enhanced in DSS-challenged mice, whereas expression as significantly reduced after treatment with AuNPs coated with glycol chitosan (FIG. 62).

### 32. Numbers of M1- and M2-polarized cells in mouse colons

This experiment was conducted to confirm M1 and M2 polarization ratios of immune cells in the lamina propria of the colon. After cutting the upper part of the colon tissue, the aggregated lymph nodes (Payer's patches) and fat were removed. The tissue was cut longitudinally and washed three times with cold PBS. The colon tissue dissected at 1-cm intervals was immersed in RPM I (10% FBS, 1% PS) containing EDTA (2 mM) and put into an incubator (37.5 °C, 250 rpm, 40 minutes), cells of the epithelial cell layer were isolated, the solution was filtered using a 70-micron cell strainer and then centrifuged at 1,500 rpm and 4 °C for 10 minutes to obtain a pellet. Afterward, after gating using CD45-APC-Cy7 (BD Pharmingen, San Diego, USA) always present in M1 and M2 immune cells, the cells were stained with CD206 APC-eFluor 780 and CD80-FITC. The staining method was performed in the same manner as described in Example 15.

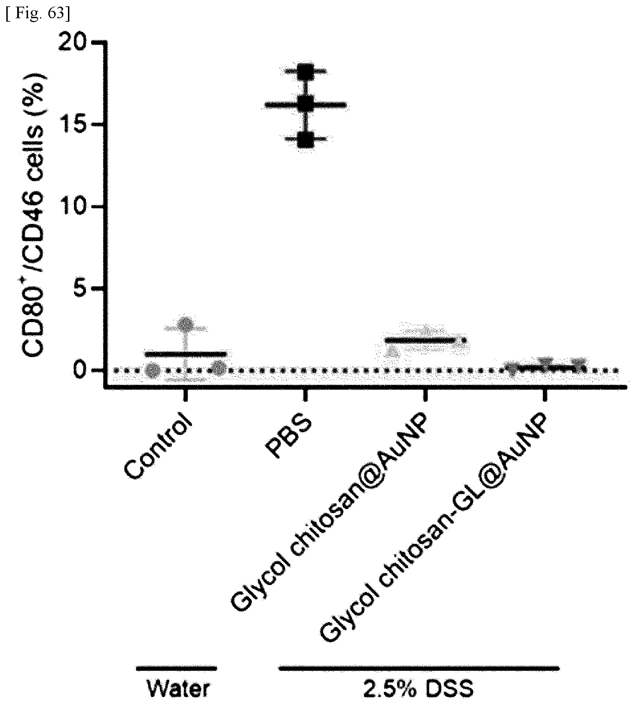

While the group which was treated with PBS after DSS induction showed a high M1 polarization ratio, the Glycol chitosan@AuNP-treated group showed a low M1 polarization ratio and the Glycol chitosan-GL@AuNP-treated group was decreased to the control level. That is, when IBD models were treated with gold particles, it was confirmed that all M1 ratios of immune cells decreased in the colon tissue (FIG. 63). In addition, it was confirmed that the ratio of pro-inflammatory CD80⁺/CD46⁺ leukocytes indicating the M1 polarization macrophages in colonic leukocytes previously collected from the PBS-treated group was higher than those of the groups treated with glycol chitosan-coated AuNPs.

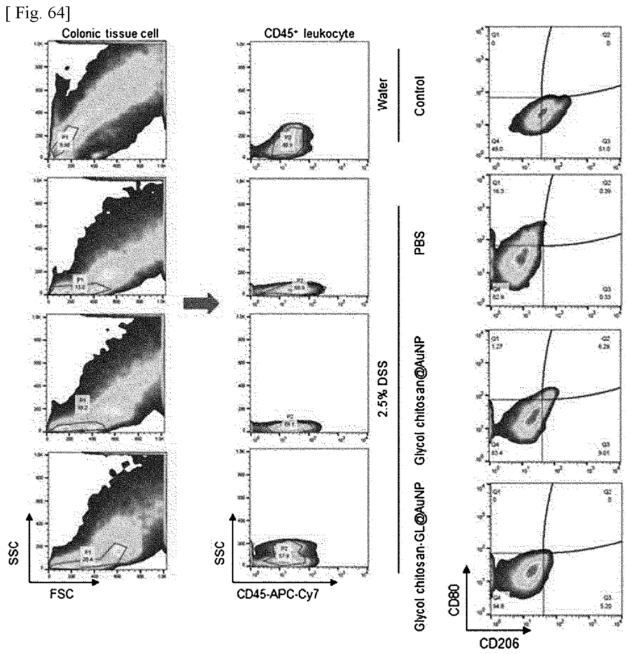

Moreover, flow cytometry was performed to determine the numbers of M1 and M2 cells in the mouse colons. After reading 10⁴ immune cells, the cells were gated based on CD45 expressed in all immune cells, M1 and M2 polarization ratios were determined within the gated range. As a result, it was confirmed that Glycol chitosan-GL@AuNP significantly reduced colonic inflammatory cytokines at both protein and mRNA levels, and reduced the ratio of inflammatory CD80⁺/CD45⁺ monocytes in colonic leukocytes (FIG. 64).

### 33. Tissue processing

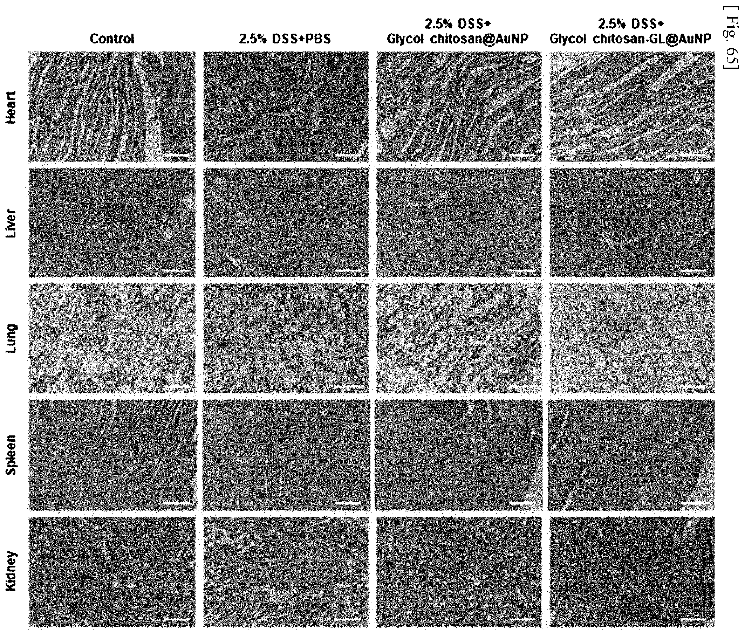

For histological analysis, tissue was fixed with 4% paraformaldehyde, and tissue processing was automatically performed using a Leica TP1020 semi-enclosed benchtop tissue processor (Leica Biosystems, Wetzlar, Hesse, Germany). The tissue was embedded in paraffin, and the paraffin block was cut to a thickness of 7 µm using a Leica RM2145 microtome (Leica Biosystems). As a result of confirming organ toxicity, it was confirmed that the sample was not toxic to the major organs including the heart, liver, lungs, kidneys and spleen (FIG. 65).

### 34. ELISA verification of C-reactive protein (CRP)

This experiment was conducted to determine the level of CRP, which is a blood inflammatory factor. After cardiac blood collection from mice under general anesthesia, the blood was centrifuged in a tube coated with an anticoagulant at 3000 rpm for 30 minutes at 4 °C. An ELISA method was performed in accordance with an experimental method in a kit (Mouse C-Reactive Protein ELISA - ALPCO).

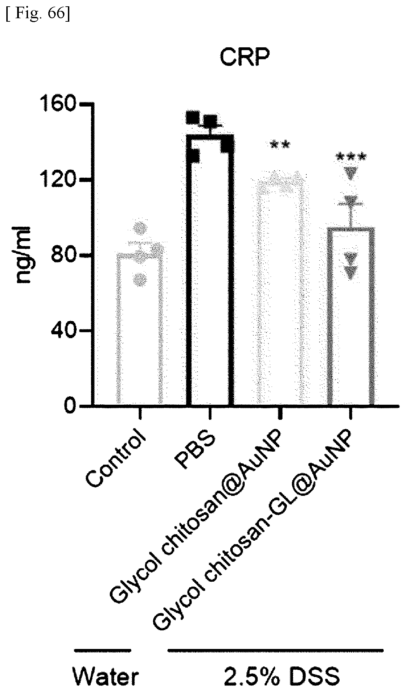

As a result, it was confirmed that the mice with a disease induced by DSS showed an increased level of CRP, which is a blood inflammatory factor, but Glycol chitosan-GL@AuNP recovered the CRP level to the normal condition (FIG. 66).

### 35. ELISA verification of calprotectin

This experiment was conducted to confirm the level of calprotectin, which is an inflammatory factor in feces. The middle part of colon tissue containing feces was cut, and the tissue was put into an EP tube and frozen in liquid nitrogen. 300 µL of RIPA buffer (Pierce^{™}) was added per 5 mg of the tissue, the tissue was homogenized using a homogenizer (FastPrep-24 5G, MP Biomedicals, LLC, USA) and centrifuged at 4 °C and 14,800 rpm for 20 minutes. After centrifugation, the supernatant was collected and stored at -20 °C until analysis, and an ELISA method was performed in accordance with an experimental method in a kit (Mouse Calprotectin ELISA - Cusabio).

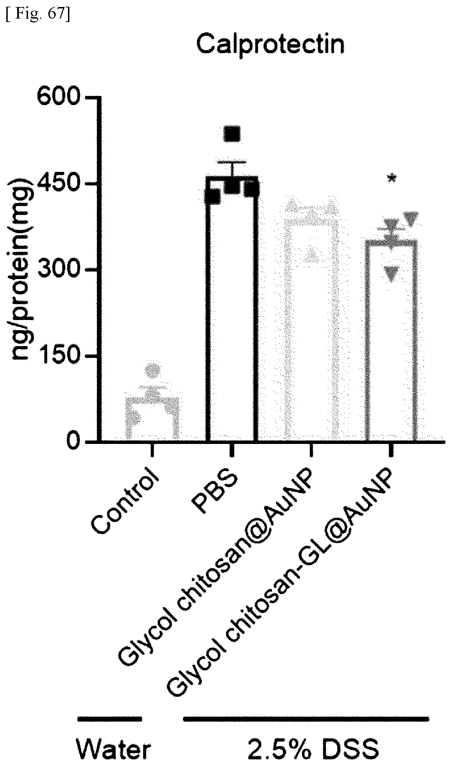

As a result, it was confirmed that calprotectin, which is a type of DAMP (S100A8/9) distinct from HMGB1, was decreased by Glycol chitosan-GL@AuNP treatment. Accordingly, it was confirmed that Glycol chitosan-GL@AuNP can control various types of DAMP molecules (FIG. 67).

### 36. Examination of mucus by immunohistochemistry (IHC)

This experiment was conducted to confirm the extent of the recovery of a mucus layer when Glycol chitosan@AuNP and Glycol chitosan-GL@AuNP were treated. Tissue processed on a slide was fixed in 4% praformaldehyde. Afterward, cells were washed twice with PBS-Tween 20 for approximately 2 minutes, and for blocking, the cells were incubated in 20% goat serum-containing PBS-Tween 20 as a solvent at room temperature for 30 minutes. The goat serum (20% in PBST-20) and primary antibodies (anti-mouse occludin, anti-rabbit ZO-1) that were diluted 100:1 were incubated at room temperature for 1 hour, and washed with PBS. In addition, secondary antibodies (goat anti mouse-488, goat anti rabbit-594) were diluted in PBS in a ratio of 1:200 and incubated with the cells. After adding one or two drops of DAPI mounting solution (blue), the slide was covered with a cover slide and observed under a fluorescence microscope.

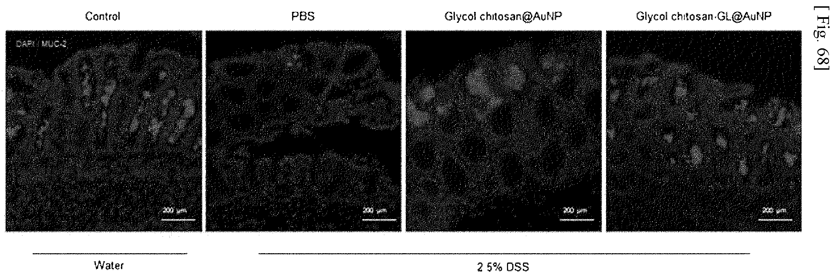

As a result, it was confirmed that mucus was stained in a subepithelial goblet cell region, which has a role in mucus secretion, and Glycol chitosan@AuNP and Glycol chitosan-GL@AuNP prevent DSS-induced mucosal damage (FIG. 68).

### 37. Confirmation of blood influx

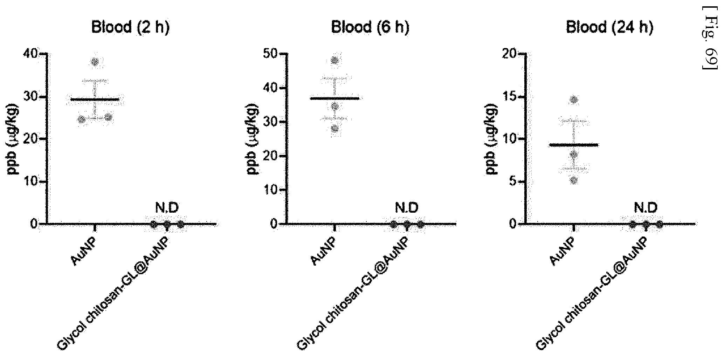

This experiment was conducted to determine a blood influx amount of Glycol chitosan-GL@AuNP. Two, six, and 24 hours after oral administration of 10 mg/kg of Glycol chitosan-GL@AuNP to 7-week-old male C57BL/6 mice, blood was collected from the heart (n=3). As a result, when blood was collected 2, 6, and 24 hours after oral administration, Glycol chitosan-GL@AuNP was not detected, confirming that the blood influx of Glycol chitosan-GL@AuNP exhibiting an intestinal mucosa-adhesive property was delayed (FIG. 69).

### 38. Biochemical and blood tests

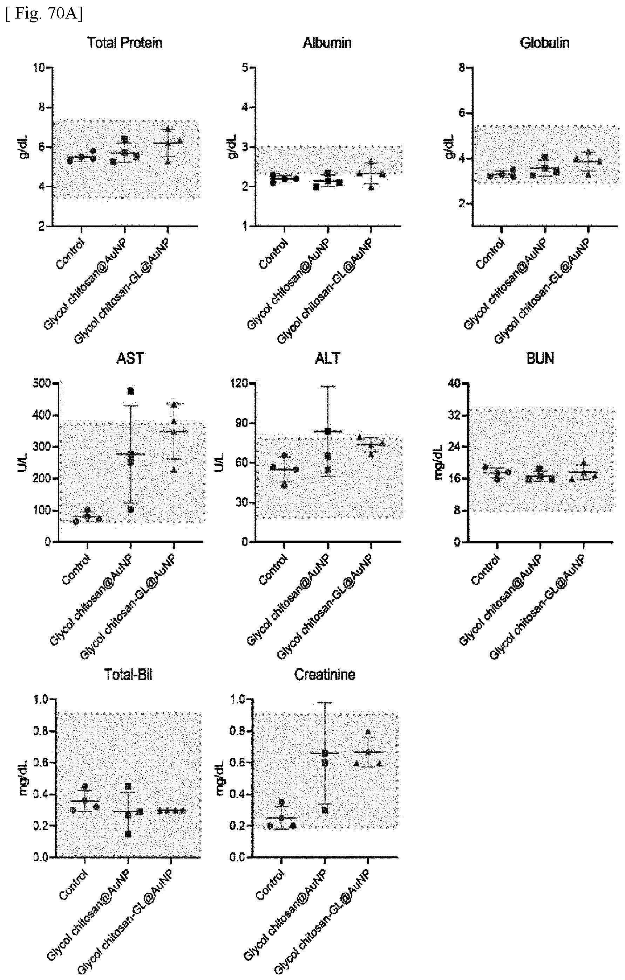

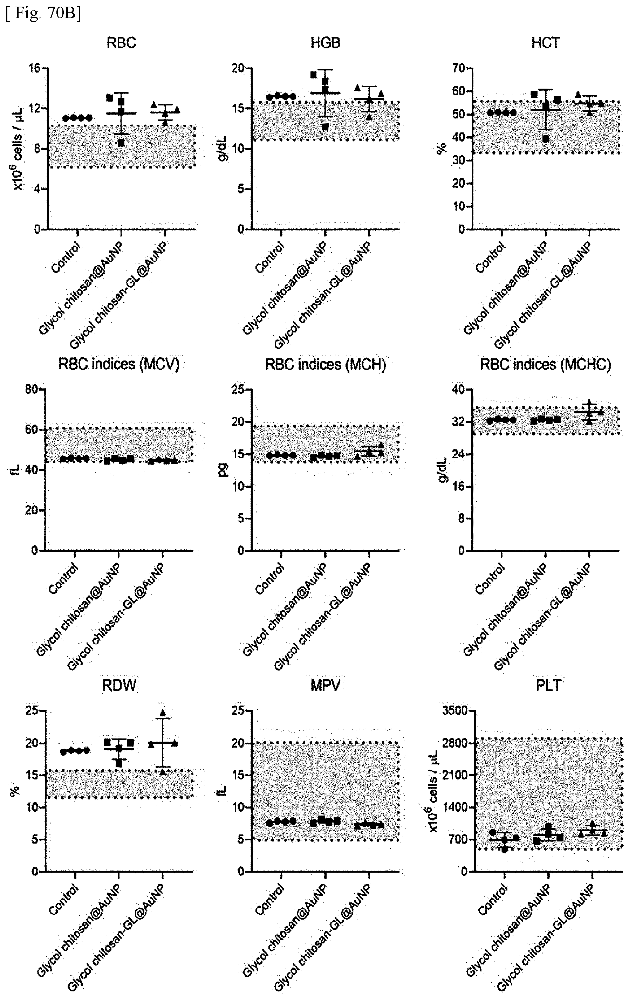

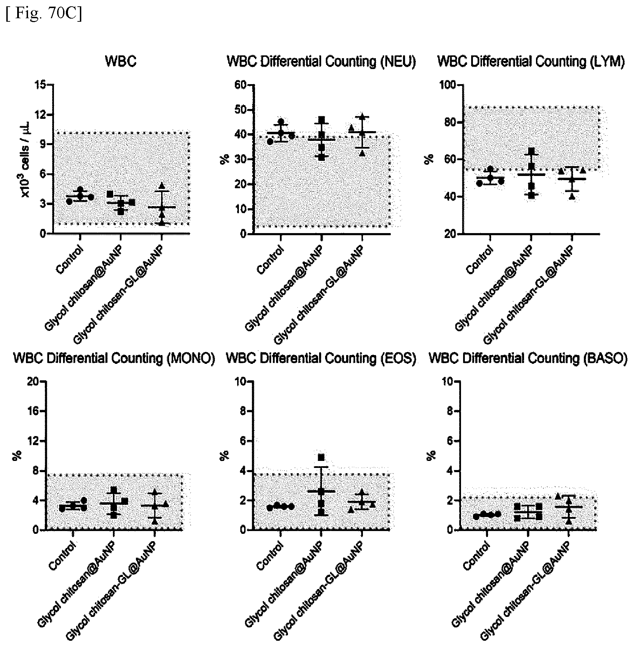

This experiment was conducted to confirm the toxicities and blood residual volume of Glycol chitosan@AuNP and Glycol chitosan-GL@AuNP. PBS and Glycol chitosan-GL@AuNP (10 mg/kg) were orally administered to 7-week-old male Balb/c mice once a day for 14 days (n=4). On day 14, serum (100 µL, for biochemical test) and plasma (200 µL, for CBC test) were collected from mice via cardiac puncture. The samples were analyzed by Daegun Health Care (DK Korea, Seoul, Korea). Specifically, FIG. 70A shows the results of analyzing total protein, albumin, globulin, AST, ALT, BUN, total bilirubin, and creatinine, FIG. 70B shows the results of analyzing RBC, HGB, HCT, RBS indices (MCV, MCH, MCHC), RDW, MPV, and PLT, and FIG. 70C shows the results of analyzing WBC, and WBC differential counts (NEU, LYM, MONO, EOS, and BASO).

As shown in FIGS. 70A to 70C, the colored area indicates the normal range of each index, and it was confirmed that most factors were within the normal range.

### 39. Microbiome analysis through feces

This experiment was conducted to perform 16s ribosomal RNA gene sequencing and taxonomic classification, and to analyze diversity through feces analysis. After orally administering PBS and Glycol chitosan-GL@AuNP (10 mg/kg) to Balb/c mice once a day for 4 days, on day 14, feces samples were collected, stored at -80 °C, and then sent to Macrogen (Seoul, Korea) for analysis. Sequencing libraries of V3 and V4 regions were constructed using the Illumina MiSeq sequencing platform, and 16s RNA amplicons were amplified using V3-V4 region primers. The sequences were assigned to operational taxonomic units (OTUs) using a 0.03 cutoff value, and classification was performed using the Ribosomal Database Project 16s rRNA gene training set (v.9) with an 80% confidence threshold for taxonomic classification. Alpha diversity was measured by Chao1 and Shannon indices, and the generalized UniFrac (GUniFrac) method was applied to calculate a phylogenetic distance. Unweighted UniFrac values for beta diversity were plotted along two principal coordinates based on 10,000 reading values per sample. The similarity between different groups was assessed by ANOSIM analysis with the vegan package (v2.5-7) in R (v4.0.5), and data was processed with GraphPad Prism (version 8.3.0; GraphPad Software Inc., San Diego, CA, USA).

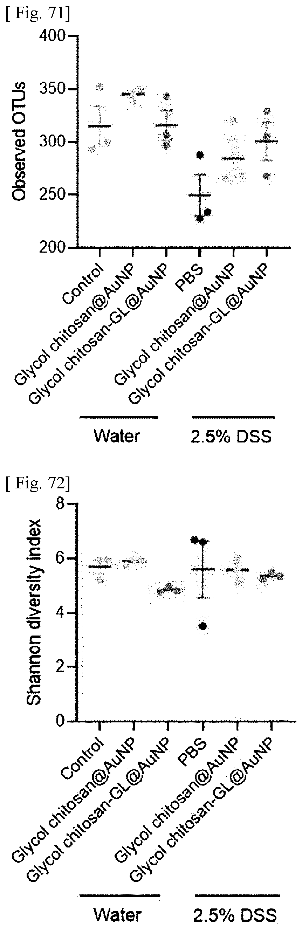

As a result of measuring OTU richness, it was confirmed that DSS-treated groups are changed in the composition of microbiota, as compared to a control (FIG. 71). In addition, it was confirmed that an imbalance in the gut microbial communities, which is associated with various inflammatory and immune disorders, plays an important role in IBD development. Here, OUT is a unit used to analyze microbial diversity and group similar sequences into species.

As a result of measuring the Shannon diversity value, it was confirmed that the species diversity was similar in DSS-treated groups including the control (FIG. 72). Shannon's diversity is an indicator that shows how diverse the classes of data are within one group, and can represent the diversity of biological species in one group.

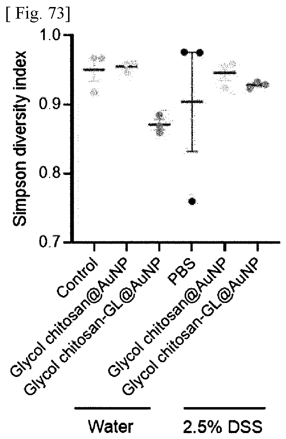

As a result of measuring the Simpson index for alpha diversity of the gut microbiota, the Glycol chitosan@AuNP- and Glycol chitosan-GL@AuNP-treated groups of the controls and the DDS groups showed less change in microbial diversity (FIG. 73). The Simpson index can measure the diversity of species using the number of animal species and the proportion of species distribution, and the closer the value is to 0, the higher the diversity, and the closer the value is to 1, the lower the diversity.

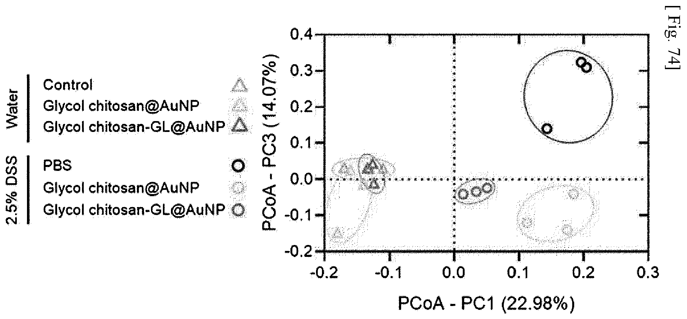

As a result of analyzing the beta diversity of the gut microbiota, beta diversities between the Glycol chitosan-GL@AuNP-treated groups of the healthy mice and DSS groups are mostly related, confirming that Glycol chitosan-GL@AuNP can recover intestinal immune homeostasis without microbial disturbance in the intestines (FIG. 74).

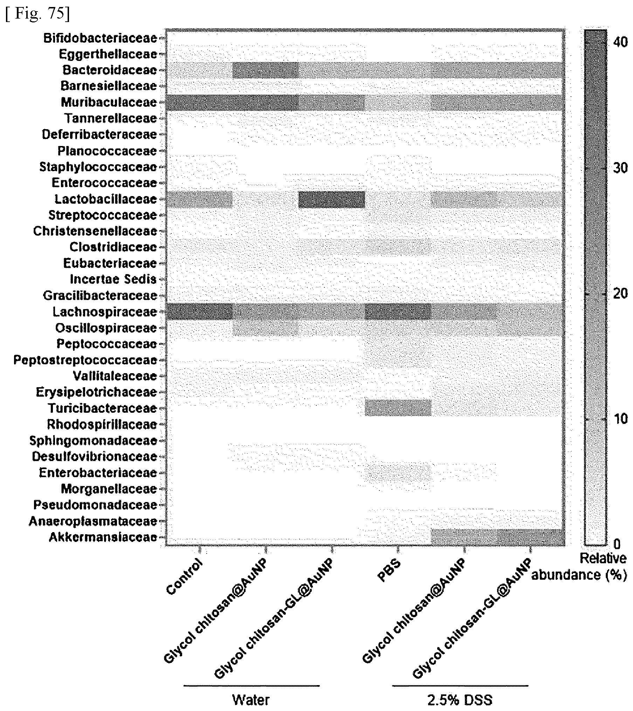

As a result of analyzing the heatmap of the taxonomic composition of microorganisms at the family level (FIG. 75), it was confirmed that, in both Glycol chitosan-GL@AuNP-treated groups of the healthy mouse and DDS groups, Glycol chitosan-GL@AuNP can recover intestinal immune homeostasis without microbial disturbance. In addition, it was confirmed that Glycol chitosan-GL@AuNP treatment significantly increased *Akkermansia muciniphila,* which is a gut microbe associated with a protective intestinal barrier function. However, it was confirmed that the amount of *Turicibacter sanguinis,* which is highly correlated with acute colitis, was reduced.

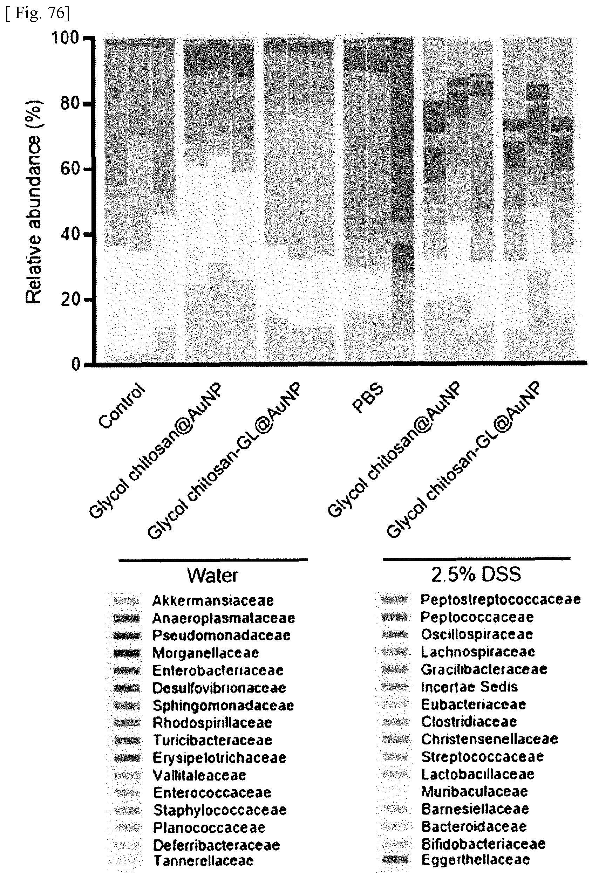

As a result of confirming the relative diversity of the intestinal microbiome (FIG. 76), in the Glycol chitosan-GL@AuNP-treated groups of the healthy mouse and DDS groups, Glycol chitosan-GL@AuNP can recover intestinal immune homeostasis without intestinal microbiota disturbance. In addition, it was confirmed that Glycol chitosan-GL@AuNP treatment significantly increased *Akkermansia muciniphila,* which is a gut microbe associated with a protective intestinal barrier function, and through additional analysis at the family level, it was confirmed that the Glycol chitosan-GL@AuNP treatment significantly increased *Lactobacillus intestinalis* having a protective intestinal barrier function and a beneficial role in IBD and *Muribaculum intestinale,* which is a gut microbe associated with essential anaerobes capable of alleviating DDS-induced IBD. However, it was confirmed that the amount of *Turicibacter sanguinis* highly correlated with acute colitis was reduced.

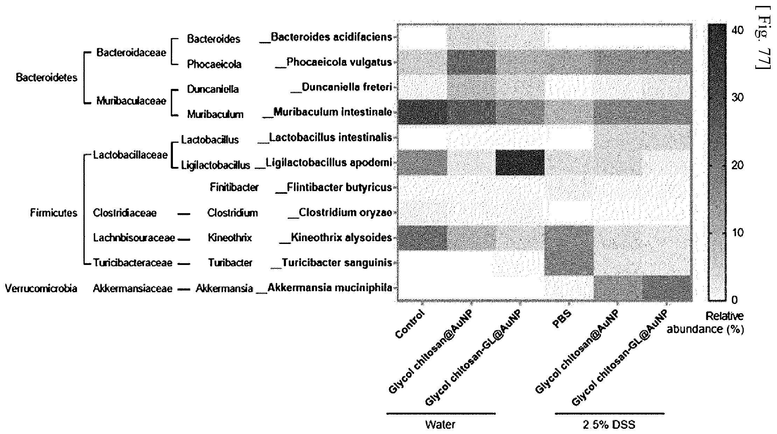

As a result of analyzing the heatmap of the taxonomic composition of microorganisms at the phylum-species levels (FIG. 77), it was confirmed that, in both Glycol chitosan-GL@AuNP-treated groups of the healthy mouse and DDS groups, Glycol chitosan-GL@AuNP can recover intestinal immune homeostasis without microbial disturbance. In addition, it was confirmed that Glycol chitosan-GL@AuNP treatment significantly increased *Akkermansia muciniphila,* which is a gut microbe associated with a protective intestinal barrier function. Through additional analysis at the family level, it was confirmed that the Glycol chitosan-GL@AuNP treatment significantly increased *Lactobacillus intestinalis* having a protective intestinal barrier function and a beneficial role in IBD and *Muribaculum intestinale,* which is a gut microbeassociated with the essential anaerobes capable of alleviating DSS-induced IBD. However, it was confirmed that the amount of *Turicibacter sanguinis* highly correlated with acute colitis was reduced.

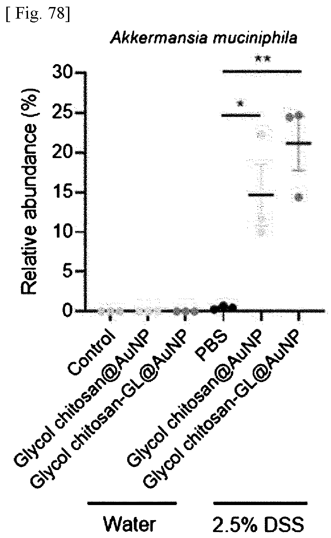

As a result of confirming the relative richness of *Akkermansia muciniphila* in the microbiota, and through analysis at the family level, it was confirmed that Glycol chitosan-GL@AuNP treatment significantly increased *Akkermansia muciniphila,* which is a gut microbe associated with a protective intestinal barrier function (FIG. 78).

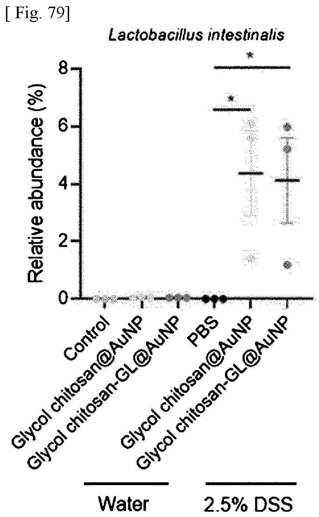

As a result of confirming the relative richness of *Lactobacillus intestinalis* in the microbiota, and through analysis at the family level, it was confirmed that Glycol chitosan-GL@AuNP treatment significantly increased *Lactobacillus intestinalis,* which has a beneficial role in IBD (FIG. 79).

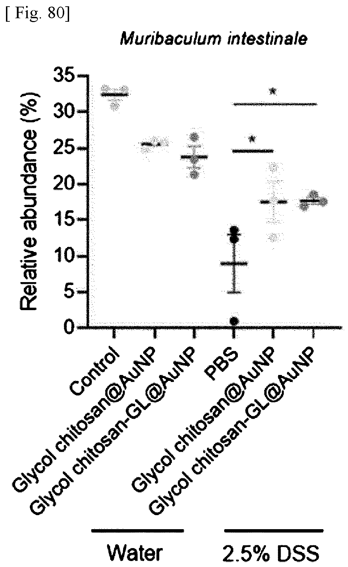

As a result of confirming the relative richness of *Muribaculum intestinale* in the microbiota, and through analysis at the family level, it was confirmed that Glycol chitosan-GL@AuNP treatment significantly increased *Muribaculum intestinale,* which is a gut microbe associated with the essential anaerobes capable of alleviating DSS-induced IBD (FIG. 80).

## Claims

1. A gold nanozyme comprising glycol chitosan and a gold particle.

2. The gold nanozyme of claim 1, wherein the glycol chitosan is embedded in the gold particle.

3. The gold nanozyme of claim 1, wherein the gold nanozyme acts as one or more enzymes selected from the group consisting of peroxidase, superoxide dismutase (SOD), and catalase.

4. The gold nanozyme of claim 1, wherein the gold nanozyme removes hydroxyl radicals .

5. The gold nanozyme of claim 1, wherein the gold nanozyme further includes glycyrrhizin.

6. The gold nanozyme of claim 5, wherein the glycyrrhizin suppresses the secretion of high mobility group box 1 (HMGB 1).

7. The gold nanozyme of any one of claims 1 to 6, wherein the gold nanozyme is stably maintained at pH 0 to 7.

8. A pharmaceutical composition for use in a method for preventing or treating inflammatory bowel disease, comprising the gold nanozyme any one of claims 1 to 7.

9. The pharmaceutical composition for use according to claim 8, which comprises the gold nanozyme at 100 µg/mL to 150 µg/mL.

10. The pharmaceutical composition for use according to claim 8, wherein the pharmaceutical composition suppresses the expression of one or more selected from the group consisting of tumor necrosis factor-α (TNF-α), interleukin 6 (IL-6), interleukin-1 β (IL-1β), inducible nitric oxide synthase (iNOS), monocyte chemoattractant protein-1 (MCP-1), C-reactive protein (CRP), and calprotectin.

11. The pharmaceutical composition for use according to claim 8, wherein the pharmaceutical composition inhibits the production of one or more selected from the group consisting of reactive oxygen species (ROS), reactive nitrogen species (RNS), and nitric oxide (NO) in cells.

12. The pharmaceutical composition for use according to claim 8, wherein the pharmaceutical composition inhibits cell polarization or spleen hypertrophy.

13. The pharmaceutical composition for use according to claim 8, wherein the pharmaceutical composition restores a damaged colon or restores tight junctions between cells.

14. The pharmaceutical composition for use according to claim 8, wherein the pharmaceutical composition is used to prevent damage to mucosa or a mucus layer or regenerate the damaged mucosa or mucus layer.

15. The pharmaceutical composition for use according to claim 8, wherein the pharmaceutical composition increases microbiome associated with a barrier protective function.

16. The pharmaceutical composition for use according to claim 8, wherein the inflammatory bowel disease is one or more selected from the group consisting of Crohn's disease, ulcerative colitis, intestinal Bechet's disease, and enteritis.

17. A method of preparing a gold nanozyme, comprising:
mixing a glycol chitosan solution and a gold particle solution;
oxidizing/reducing glycol chitosan and gold particles in the mixed solution; and
forming a nanozyme with the oxidized/reduced glycol chitosan and the gold particles.

18. The method of claim 17, wherein, in the mixing, the glycol chitosan solution further comprises glycyrrhizin.

19. The method of claim 17, wherein, in the mixing, a ratio of the glycol chitosan solution and the gold particle solution is 1:2 to 1:10.

20. The method of claim 17, wherein, in the forming of a nanozyme, the oxidized/reduced glycol chitosan is embedded in the gold particles.
